(19) Europäisches Patentamt
European Patent Office
Office européen des brevets

(11) Veröffentlichungsnummer: **0 012 969**
**B1**

(12) # EUROPÄISCHE PATENTSCHRIFT

(45) Veröffentlichungstag der Patentschrift: **08.06.83**

(21) Anmeldenummer: **79105219.4**

(22) Anmeldetag: **17.12.79**

(51) Int. Cl.³: **C 09 B 62/12,** C 07 D 237/14, D 06 P 1/382, D 06 P 3/02, D 06 P 3/32, D 06 P 3/66

(54) **Neue, wasserlösliche, faserreaktive Verbindungen und deren Verwendung zur Faserveredlung, insbesondere als Farbstoffe, und neue Dihalogenopyridazonyl-carbonsäure- und -sulfonsäure-halogenide und deren Verwendung als faserreaktive Anker sowie Verfahren zur Herstellung dieser neuen Verbindungen.**

(30) Priorität: **21.12.78 DE 2855262**

(43) Veröffentlichungstag der Anmeldung: **09.07.80 Patentblatt 80/14**

(45) Bekanntmachung des Hinweises auf die Patenterteilung: **08.06.83 Patentblatt 83/23**

(84) Benannte Vertragsstaaten: **CH DE FR GB**

(56) Entgegenhaltungen:
**DE-B-1 193 623**
**DE-B-1 239 037**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Schläfer, Ludwig, Dr., Zum Gimbacher Hof 9A, D-6233 Kelkheim (Taunus) (DE)**

**EP 0 012 969 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue, wasserlösliche, faserreaktive Verbindungen und deren Verwendung zur Faserveredlung, insbesondere als Farbstoffe, und neue Dihalogeno-pyridazonyl-carbonsäure- und -sulfonsäure-halogenide und deren Verwendung als faserreaktive Anker sowie Verfahren zur Herstellung dieser neuen Verbindungen

Die vorliegende Erfindung betrifft das technische Gebiet der Veredlung von Fasermaterialien unter Anwendung von faserreaktiven Verbindungen, insbesondere von Farbstoffen, da mit der vorliegenden Erfindung neue faserreaktive Verbindungen, insbesondere Farbstoffe, gefunden wurden, die einen neuen faserreaktiven Anker besitzen.

Faserreaktive Verbindungen, vorwiegend Farbstoffe, insbesondere auch solche, die einen halogensubstituierten stickstoffhaltigen Heterocyclus als faserreaktiven Anker besitzen, sind zahlreich in der Literatur beschrieben. Diese bekannten Verbindungen genügen nicht in allen Fällen den Anforderungen der heutigen Technik; so ist in vielen Fällen ihr Fixiergrad auf dem Fasermaterial nicht zufriedenstellend oder die mit ihnen veredelten Cellulosefasermaterialien sind nicht ausreichend nassecht, so dass die Farbstoffe selbst nicht in allen Fällen eine befriedigende Farbstärke und eine gleichbleibende Farbausbeute oder Nuance bei Anwendung auf unterschiedlichen Fasermaterialien, wie Baumwolle und Zellwolle, und ihre Färbungen eine zu starke Farbtonänderung oder ein zu starkes Ausbluten bei Nassbehandlungen zeigen.

So sind aus den deutschen Auslegeschriften 1 193 623 und 1 239 037 faserreaktive Farbstoffe bekannt, die als faserreaktiven Rest eine 4,5-Dichlor-6-pyridazonyl-Gruppe enthalten. Dieser faserreaktive Rest ist jedoch technisch nur schwer über die Mucochlorsäure zugänglich, die in 20%iger Ausbeute über das 2-Butin-1,4-diol durch chlorierende Oxidation mittels Chlorgas erhältlich ist.

Mit der vorliegenden Erfindung werden neue Verbindungen, insbesondere Farbstoffe, zur Verfügung gestellt, die eine neue faserreaktive Gruppe besitzen und die sich nach üblichen Methoden der Anwendung von faserreaktiven Verbindungen auf solchen Fasermaterialien mit hoher Farbstärke und konstanter Farbausbeute und Nuance applizieren und ausreichend nassecht fixieren lassen.

Die vorliegende Erfindung betrifft demnach neue wasserlösliche Verbindungen der allgemeinen Formel (1)

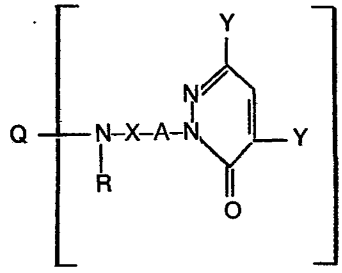

(1)

n

in welcher

Q der Rest einer wasserlöslichen organischen Verbindung ist, die faserveredelnde Eigenschaften besitzt, wie beispielsweise der Rest eines Farbstoffmoleküls oder der Rest einer Verbindung, die Fasermaterial optisch aufzuhellen oder mottensicher zu machen oder ihm wasserabweisende Eigenschaften oder einen weichen Griff oder eine erhöhte Anfärbbarkeit oder eine gute Knitterfestigkeit oder gute Flammfestigkeit zu verleihen vermag, wobei Q wasserlöslichmachende Gruppen, vorzugsweise 1 bis 8 wasserlöslichmachende Gruppen, wie Solfonsäuregruppen, enthält,

A einen Alkylenrest von 1 bis 6 C-Atomen, vorzugsweise von 1 bis 4 C-Atomen, oder einen Alkylenrest von 2 bis 8 C-Atomen, der durch einen Phenylenrest unterbrochen ist, insbesondere einen solchen Alkylen-phenylen-alkylen-Rest mit niederen Alkylengruppen, oder einen Phenylenrest oder einen Phenylenalkylen-Rest mit 1 bis 4 C-Atomen im Alkylenrest oder einen Alkylen-phenylen-Rest mit 1 bis 4 C-Atomen im Alkylenrest bedeutet,

R für ein Wasserstoffatom oder eine Alkylgruppe mit 1 bis 4 C-Atomen, die durch einen Arylrest, wie beispielsweise einen Phenylrest, oder durch eine niedere Alkoxy, eine Hydroxy-, Phenoxy- oder niedere Alkanoyloxy-Gruppe substituiert sein kann, oder für einen Cycloalkylrest, wie beispielsweise einen Cyclohexylrest, oder für einen Arylrest, wie beispielsweise einen Phenylrest, der eine Sulfogruppe enthalten kann, steht,

X die Carbonyl- oder Sulfonylgruppe ist,

Y für ein Halogenatom, wie insbesondere ein Fluor- oder Chloratom, steht und

n eine ganze Zahl von 1 bis 3, vorzugsweise die Zahl 1 oder 2, insbesondere die Zahl 1, darstellt.

Die obengenannten neuen Verbindungen der allgemeinen Formel (1) liegen in Form ihrer freien Säure, bevorzugt jedoch in Form ihrer Salze, insbesondere neutralen Salze, wie beispielsweise ihrer Alkalimetall- oder Erdalkalimetallsalze, so beispielsweise der Natrium-, Kalium- oder Calciumsalze, vor und finden bevorzugt in Form dieser Salze ihre Verwendung zur Applikation auf und zur Veredlung von Fasermaterialien.

Die Angaben «niedere» bedeutet hier wie im folgenden, dass der in der Gruppe enthaltene Alkyl- oder Alkylenrest oder Alkenylrest bevorzugt aus 1 bis 4 C-Atomen besteht.

Die vorliegende Erfindung betrifft vorzugsweise Verbindungen der allgemeinen Formel (1), in welcher Q ein wasserlöslicher organischer Farbstoffrest ist, der vorzugsweise 1 bis 8 wasserlöslichmachende Gruppen enthält. Die Farbstoffe können den verschiedensten organischen Verbindungsklassen angehören; so kann Q für den Rest eines organischen Farbstoffes aus der Anthrachinon-,

Formazan-, Cyanin-, Phthalocyanin-, Benzoxanthen-, Benzothioxanthen-, Xanthen-, Triphenylmethan-, Azin-, Phenazin-, Oxazin-, Phenoxazin-, Thiazin-, Chinolin-, Indolphenazin-, Azomethin-, Styryl-, Nitro- und Naphthalimid-Reihe angehören, wobei die hierfür geeigneten Farbstoffe noch Schwermetalle, wie beispielsweise Kupfer-, Nickel-, Kobalt-, Chrom-Ionen, komplex gebunden, enthalten können; bevorzugt steht der Rest Q jedoch für den Rest eines Mono-, Dis- oder Polyazofarbstoffes, wie eines Trisazo- oder Tetrakisazofarbstoffes, und deren Schwermetallkomplexe, wobei dieser Farbstoffrest Q jeweils vorzugsweise 1 bis 8 wasserlöslichmachende Gruppen enthält.

Die neuen Verbindungen der allgemeinen Formel (1) werden in erfindungsgemässer Weise hergestellt, indem man eine organische Verbindung der allgemeinen Formel (2)

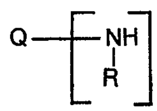 (2)

in welcher Q, R und n die obengenannten Bedeutungen haben, mit einer Verbindung der allgemeinen Formel (3)

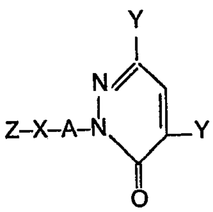 (3)

in welcher A, X und Y die obengenannten Bedeutungen haben und Z für ein Halogenatom, vorzugsweise ein Chloratom, steht, wobei Y und Z gleich oder verschieden voneinander sein können, umsetzt, oder indem man organische Vorprodukte für die Synthese des Molekülrestes der Formel Q in üblicher und hierfür geeigneter Weise miteinander umsetzt, wobei diese Vorprodukte so ausgewählt sind, dass mindestens eines davon eine oder mehrere wasserlöslich-machende Gruppen und mindestens eine Gruppe der Formel (4)

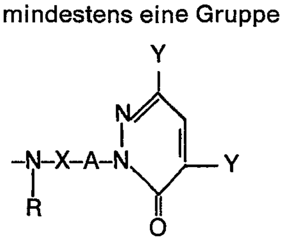 (4)

mit A, R, X und Y der obengenannten Bedeutung enthält und dass man dabei wiederum diese Vorprodukte so auswählt, dass eine Verbindung der Formel (1) erhalten wird, in welcher n für eine Zahl von 1 bis 3 steht und der Rest Q 1 bis 8 wasserlöslich-machende Gruppen enthält.

Vorprodukte, die erfindungsgemäss zur Herstellung der Verbindungen der allgemeinen Formel (1) eingesetzt werden können, sind beispielsweise für die Herstellung von Azofarbstoffen entsprechende Diazokomponenten und Kupplungskomponenten, von denen eine mindestens eine wasserlöslich-machende Gruppe bzw. eine faserreaktive Gruppe der Formel (4) enthält.

Die vorliegende Erfindung betrifft weiterhin die neuen Verbindungen der Formel (3) mit der obengenannten Bedeutung sowie deren Verwendung als faserreaktive Anker in Verbindungen mit faserveredelnden Eigenschaften, insbesondere für die Herstellung von faserreaktiven organischen Farbstoffen.

Die neuen Verbindungen der Formel (3) können in erfindungsgemässer Weise hergestellt werden, indem man eine Hydrazinverbindung der allgemeinen Formel (5)

$$HO–X–A–NH–NH_2 \qquad (5)$$

mit A und X der obengenannten Bedeutung mit Maleinsäureanhydrid zu einer Verbindung der Formel (6)

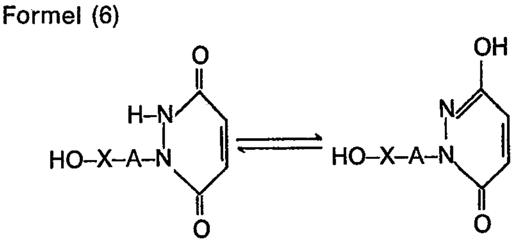 (6)

umsetzt und diese mit geeigneten Chlorierungsmitteln, wie z.B. Phosphortrichlorid, Phosphoroxychlorid, Phosphorpentachlorid, Thionylchlorid, Sulfurylchlorid oder elementarem Chlor, in die Verbindungen der Formel (3), in welcher Y und Z jedes ein Chloratom bedeuten, überführt.

Die Chlorierungsreaktion der Verbindungen der Formel (6) zu Verbindungen der Formel (3) kann analog der in Helvetica Chimica Acta 37, 510 (1954) beschriebenen Verfahrensweise hergestellt werden. Aus den Verbindungen der Formel (3), in denen Z und Y jedes für ein Chloratom steht, können die entsprechenden Fluorderivate durch Umsatz mit geeigneten Fluorierungsmitteln, mit deren Hilfe die Chloratome durch Fluoratome ausgetauscht werden, hergestellt werden.

Man kann aber auch von solchen Verbindungen der Formel (5) ausgehen, in welcher der Formelrest –X–OH statt der Carboxygruppe die Cyangruppe bedeutet, die Cyangruppe nach Ringschluss zum Heterocyclus zur Carboxygruppe hydrolysieren und anschliessend die Chlorierung durchführen.

Die Verbindungen der Formel (5) erhält man beispielsweise analog zu bekannten Verfahrensreaktionen durch Diazotierung von entsprechenden aromatischen Aminen der Formel (7)

$$HO–X–A–NH_2 \qquad (7)$$

in welcher A und X die obengenannte Bedeutung haben und die primäre Aminogruppe direkt am aromatischen Rest von A steht, mit anschliessender Reduzierung der erhaltenen Diazoniumverbindung mittels schwefliger Säure oder deren Salz, wie Natriumsulfit. Verbindungen der allgemeinen Formel (5), in welcher die Hydrazingruppe an einen Alkylenrest gebunden ist, lassen sich beispielsweise durch Umsetzung der entsprechenden Alkenyl-carbonsäure oder des Alkenylnitrils mit Hydrazin (mit späterer Verseifung der Cyangruppe zur Carboxygruppe) herstellen; eine analoge Verfahrensweise ist beispielsweise aus der deutschen Patentschrift 598 185 bekannt. Verbindungen der allgemeinen Formel (5), in welcher die Hydrazingruppe an einen Benzylrest gebunden ist, lassen sich beispielsweise durch Hydrierung der entsprechenden Carbon- oder Sulfonsäureverbindungen von Acylbenzalhydrazinen gemäss DE-PS 1 003 215 herstellen.

Erfindungsgemässe Verbindungen der Formel (3) sind beispielsweise die Verbindungen der Formeln

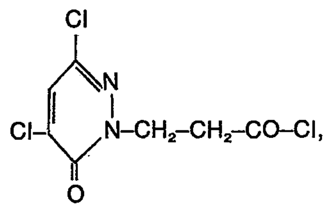

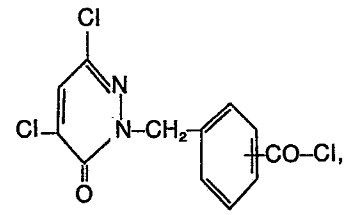

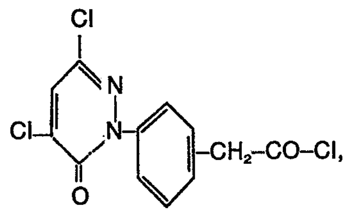

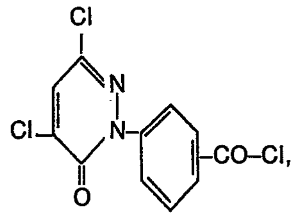

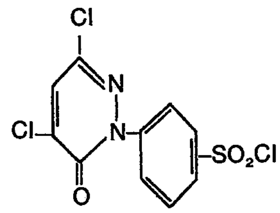

sowie deren entsprechenden Fluorderivate.

Die Vorprodukte für den Molekülrest der Formel Q, die eine oder mehrere faserreaktive Gruppen der Formel (4) enthalten, lassen sich in ähnlicher Weise, wie die Verbindungen der allgemeinen Formel (1) herstellen, indem man von Verbindungen der allgemeinen Formel (8)

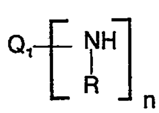 (8)

ausgeht, in welcher R und n die obengenannten Bedeutungen haben und $Q_1$ für den Rest des entsprechenden Vorproduktes mit gegebenenfalls einer oder mehreren wasserlöslich-machenden Gruppen steht (so beispielsweise, falls Q der Rest eines Monoazofarbstoffes ist, $Q_1$ für den Rest einer Diazo- oder Kupplungskomponente steht), und diese Aminoverbindung der Formel (8) mit einer Verbindung der Formel (3) umsetzt. Geht man von Verbindungen der Formel (8) aus, in welcher $Q_1$ eine primäre Aminogruppe enthält, beispielsweise von einer Diaminobenzol-sulfonsäure, die als Diazokomponente oder auch als Kupplungskomponente geeignet ist, so erfolgt die Umsetzung mit dem Sulfonsäurechlorid oder Carbonsäurechlorid der Formel (3) nur in einer Stufe zur Monoamino-acylamino-Verbindung.

Erfindungsgemässe Vorprodukte der allgemeinen Formel (9)

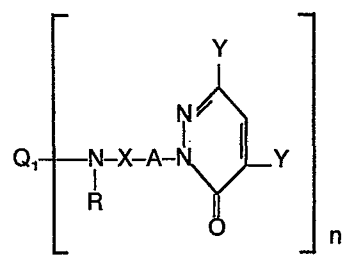 (9)

mit $Q_1$, A, R, X, Y und n der obengenannten Bedeutung, die sich insbesondere als Diazokomponenten oder Kupplungskomponenten für die Herstellung von Azofarbstoffen eignen, sind insbesondere solche Verbindungen, in welchen $Q_1$ für einen Benzol- oder Naphthalinrest steht, der durch eine primäre Aminogruppe substituiert ist und gegebenenfalls durch 1, 2 oder 3 Sulfonsäuregruppen und/oder 1, 2 oder 3 weitere Substituenten substituiert ist, die der Gruppe niederes Alkyl, niederes Alkoxy, Carboxy, Hydroxy, Acetylamino, Benzoylamino, Phenylamino, durch Substituenten aus der Gruppe niederes Alkyl, niederes Alkoxy, Sulfo, Carboxy, Acetylamino, Benzoylamino und Halogen, wie Chlor, substituiertes Phenylamino,

Carbonsäureamid, durch niederes Alkyl und/oder Phenyl mono- oder disubstituiertes Carbonsäureamid (wobei der Phenylrest noch durch Substituenten aus der Gruppe niederes Alkyl, niederes Alkoxy, Sulfo, Carboxy, Chlor und Amino substituiert sein kann), Sulfonsäureamid, durch niederes Alkyl und/oder Phenyl mono- oder disubstituiertes Sulfonsäureamid (wobei der Phenylrest noch durch Substituenten aus der Gruppe niederes Alkyl niederes Alkoxy, Sulfo, Carboxy, Chlor und Amino substituiert sein kann), Cyan, Nitro, Halogen, wie Chlor, Fluor und Brom, und Trifluormethyl angehören.

Bevorzugt sind Verbindungen der allgemeinen Formel (9) als Diazokomponenten, in welcher $Q_1$ eine primäre Aminogruppe besitzt und einen Benzolkern darstellt, der durch 1 oder 2 Substituenten substituiert ist, die der Gruppe niederes Alkyl, niederes Alkoxy, Chlor, Brom, Sulfo, Carboxy, Acetylamino, Benzoylamino, durch Substituenten aus der Gruppe Methoxy, Methyl, Chlor und Sulfo substituiertes Benzoylamino, Phenylamino, durch Substituenten aus der Gruppe Methoxy, Methyl und Sulfo substituiertes Phenylamino, Hydroxy, Nitro, Carbonsäureamid, Sulfonsäureamid, Monoalkylcarbonsäureamid, Dialkylcarbonsäureamid, Monoalkylsulfonsäureamid, Dialkylsulfonsäureamid, N-Phenylcarbonsäureamid, N-Phenylsulfonsäureamid, N-Alkyl-N-phenyl-carbonsäureamid, N-Alkyl-N-phenyl-sulfonsäureamid (wobei die Alkylreste in den Amiden jeweils niedere Alkylreste sind und die Phenylreste noch durch Substituenten aus der Gruppe Sulfo, niederes Alkyl, niederes Alkoxy, Chlor und Amino substituiert sein können) und niederes Dialkylcarbonsäureamid, in welcher der eine Alkylrest durch einen Phenylrest substituiert ist, der wiederum Substituenten aus der Gruppe Sulfo, niederes Alkyl, niederes Alkoxy, Chlor und Amino substituiert sein kann, angehören, und A, R, X und Y die obengenannten Bedeutungen haben und n für die Zahl 1 steht.

Verbindungen der allgemeinen Formel (9), die als Diazokomponenten geeignet sind, sind weiterhin solche, in welchen $Q_1$ für den Naphthalinkern steht, der durch eine Aminogruppe substituiert ist und durch 1 oder 2 Sulfonsäuregruppen und durch 1 oder 2 weitere Substituenten substituiert sein kann, die der Gruppe niederes Alkyl, niederes Alkoxy, Nitro, Chlor und Carboxy angehören, und A, R, X und Y die obengenannten Bedeutungen haben und n für die Zahl 1 oder 2, bevorzugt 1 steht.

Verbindungen der allgemeinen Formel (9) die als Kupplungskomponenten für die Herstellung von Azofarbstoffen geeignet sind, sind beispielsweise solche, in denen $Q_1$ für den Rest des Naphthols steht, der durch 1, 2 oder 3 Sulfogruppen und durch eine weitere Aminogruppe, Phenylamino oder niedere Alkylaminogruppe substituiert sein kann, des weiteren solche, in denen $Q_1$ für den Benzolkern steht, der durch eine Hydroxygruppe und/oder eine Aminogruppe oder eine Phenylamino- oder niedere Alkylaminogruppe substituiert ist und durch eine niedere Alkyl- oder niedere Alkoxygruppe oder ein Chloratom substituiert sein kann. Verbindungen der allgemeinen Formel (9), die als Kupplungskomponenten für die Herstellung von Azofarbstoffen geeignet sind, sind weiterhin beispielsweise solche, in welchen $Q_1$ für den Naphthalinkern steht, der durch eine Aminogruppe substituiert ist und durch 1, 2 oder 3 Sulfogruppen substituiert sein kann, des weiteren der Rest des Pyrazol-5-ons oder Pyrazol-3-ons oder des 5-Amino-pyrazols oder 3-Aminopyrazols, wobei diese Pyrazolreste, so die des Pyrazol-5-ons oder des 5-Aminopyrazols jeweils in 3-Stellung, durch niederes Alkyl, Phenyl, Chlorphenyl, Methylphenyl, Carboxy oder niederes Carbalkoxy und jeweils in 1-Stellung durch einen Phenyl- oder Naphthylrest substituiert sind, wobei diese Phenyl- und Naphthylreste die faserreaktive(n) Gruppe(n) gebunden enthalten und weiterhin durch 1, 2 oder 3 Substituenten aus der Gruppe niederes Alkyl, niederes Alkoxy, Chlor, Brom, Trifluormethyl, Sulfo, Carboxy, Acetylamino, Benzoylamino, Amino und Cyan substituiert sein können. Weiterhin ist beispielsweise $Q_1$ der Rest des Acetoacetyl-anilid- oder -naphthylamids, in welchen die aromatischen Kerne die faserreaktive(n) Gruppe(n) der Formel (4) gebunden enthalten und diese aromatischen Kerne noch durch 1, 2 oder 3 Substituenten substituiert sein können, die der Gruppe niederes Alkyl, niederes Alkoxy, Chlor, Brom, Sulfo, Carboxy, Acetylamino, Benzoylamino, Nitro und Cyan angehören, ferner alle weiteren organischen Verbindungen, die mit aromatischen Diazoniumverbindungen zu Azoverbindungen kuppeln, wie z.B. Malonsäure und ihre Derivate, wie Malonsäurealkylester und Malodinitril, und Barbitursäure und ihre Derivate.

Verbindungen der allgemeinen Formel (9), die bevorzugt als Kupplungskomponenten eingesetzt werden können, sind beispielsweise Verbindungen der allgemeinen Formeln (9a), (9b), (9c), (9d), (9e), (9f), (9g), (9h), (9i) oder (9j)

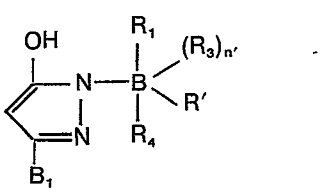
(9a)

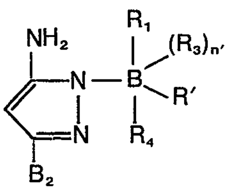
(9b)

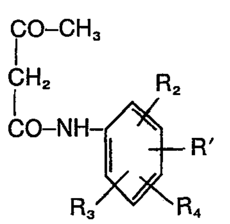
(9c)

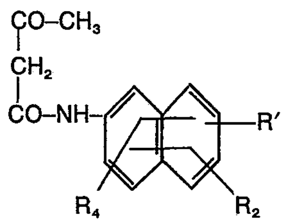

(9d)

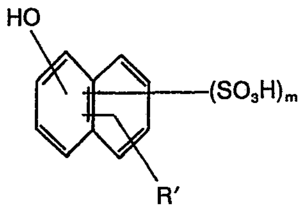

(9e)

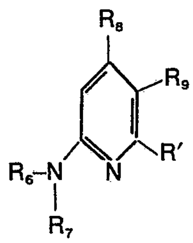

(9f)

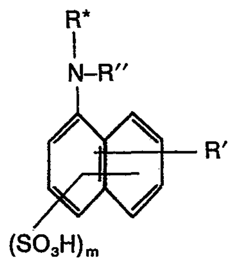

(9g)

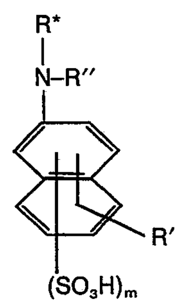

(9h)

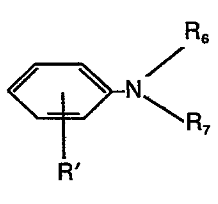

(9i)

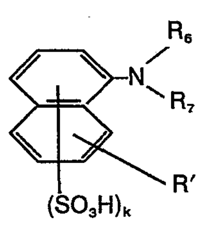

(9j)

In diesen Formeln bedeuten:

R′ ist der faserreaktive Acylamidrest der allgemeinen Formel (4);

$R_1$ ist ein Wasserstoffatom, eine niedere Alkylgruppe, wie insbesondere eine Methyl- oder Äthylgruppe, eine niedere Alkoxygruppe, wie insbesondere eine Methoxy- oder Äthoxygruppe, ein Chlor- oder Bromatom, eine niedere Alkanoylaminogruppe, wie eine Acetylaminogruppe, oder eine Sulfonsäuregruppe;

$R_2$ ist ein Wasserstoffatom, eine niedere Alkylgruppe, wie insbesondere eine Methyl- oder Äthylgruppe, eine niedere Alkoxygruppe, wie insbesondere eine Methoxy- oder Äthoxygruppe, ein Chlor- oder Bromatom oder eine Sulfonsäuregruppe;

$R_3$ ist ein Wasserstoffatom, eine niedere Alkylgruppe, wie insbesondere die Methyl- oder Äthylgruppe, eine niedere Alkoxygruppe, wie insbesondere die Methoxy- oder Äthoxygruppe, ein Chlor- oder Bromatom;

$R_4$ ist ein Wasserstoffatom, eine niedere Alkylgruppe, wie insbesondere die Methyl- oder Äthylgruppe, eine niedere Alkoxygruppe, wie insbesondere die Methoxy- oder Äthoxygruppe, oder die Sulfonsäuregruppe, wobei $R_1$, $R_2$, $R_3$ und $R_4$ gleich oder verschieden voneinander sein können;

n′ ist die Zahl 1 oder 2;

B ist ein Benzol- oder Naphthalinkern;

$B_1$ ist eine niedere Alkylgruppe, bevorzugt Methylgruppe, eine Carboxygruppe, eine Carbomethoxy- oder Carbäthoxygruppe oder der Phenylrest;

$B_2$ ist eine niedere Alkylgruppe, bevorzugt die Methylgruppe, eine Carbomethoxy- oder Carbäthoxygruppe, eine Carbonamidgruppe oder ein Phenylrest, der durch 1 oder 2 Substituenten aus der Gruppe niederes Alkyl, niederes Alkoxy, Chlor, Brom und Sulfo substituiert sein kann, wobei $R_1$, $R_3$, $R_4$ und $B_1$ bzw. $B_2$ gleich oder verschieden voneinander sein können;

R* ist ein Wasserstoffatom oder ein niederer Alkylrest;

R″ ist ein Wasserstoffatom, ein niederer Alkylrest oder ein Phenylrest, der durch 1 oder 2 Substituenten aus der Gruppe niederes Alkyl, niederes Alkoxy, Chlor, Brom und Sulfo substituiert sein kann, wobei R* und R″ gleich oder verschieden voneinander sein können;

k ist die Zahl Null oder 1;

m steht für die Zahl Null, 1 oder 2;

$R_6$ ist ein Wasserstoffatom oder eine niedere Alkylgruppe, die durch eine Hydroxy-, Cyan-, Carboxy-, Sulfo-, Sulfato-, Carbomethoxy-, Carbäthoxy- oder Acetoxygruppe substituiert sein kann;

$R_7$ ist ein Wasserstoffatom oder eine niedere Alkylgruppe, die durch eine Hydroxy-, Cyan-, Carboxy-, Sulfo-, Sulfato-, Carbomethoxy-, Carbäthoxy- oder Acetoxygruppe substituiert sein kann, oder ein Benzylrest oder ein Phenylrest, der durch niederes Alkyl, niederes Alkoxy, Chlor und/oder Sulfo substituiert sein kann;

$R_8$ ist ein Wasserstoffatom oder eine niedere

Alkylgruppe, die Methyl- oder Äthylgruppe, oder eine durch niederes Alkoxy oder Cyan substituierte niedere Alkylgruppe;

$R_9$ ist ein Wasserstoffatom, eine niedere Alkylgruppe, wie Methylgruppe, eine niedere Sulfoalkylgruppe, wie Sulfomethylengruppe, die Cyan- oder die Carbonamidgruppe, wobei $R_1$, $R_6$, $R_7$, $R_9$ und $R_9$ gleich oder verschieden voneinander sein können.

Bevorzugte Verbindungen der allgemeinen Formel (9), die als Diazokomponenten zur Herstellung von faserreaktiven Azofarbstoffen Verwendung finden können, sind beispielsweise die Verbindungen der Formel (9k), (9m), (9n) und (9p)

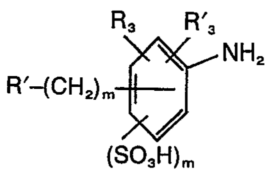

(9k)

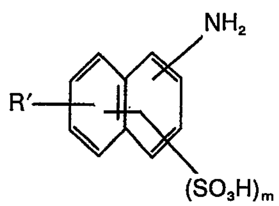

(9m)

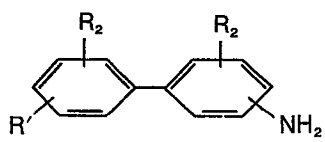

(9n)

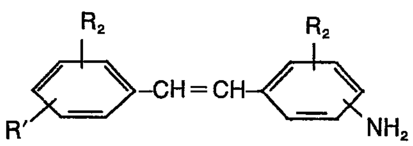

(9p)

In diesen Formeln besitzen die Formelreste R′, $R_2$, $R_3$ und m die obengenannten Bedeutungen, wobei die Indices m gleich oder verschieden voneinander sein können, und $R'_3$ ist ein Wasserstoffatom, eine niedere Alkylgruppe, wie insbesondere die Methyl- oder Äthylgruppe, eine niedere Alkoxygruppe, wie insbesondere die Methoxy- oder Äthoxygruppe, ein Chlor- oder Bromatom oder eine Hydroxygruppe, wobei $R_3$ und $R'_3$ gleich oder verschieden voneinander sein können.

Verbindungen der allgemeinen Formeln (9) sind beispielsweise: 1,3-Diaminobenzol, 1,3-Diaminobenzol-4-sulfonsäure, 1,4-Diaminobenzol, 1,4-Diaminobenzol-2-sulfonsäure, 1,4-Diaminobenzol-2,5-disulfonsäure, 1,4-Diamino-2-methyl-benzol, 1,4-Diamino-2-methoxy-benzol, 1,3-Diamino-4-methyl-benzol, 1,4-Diaminobenzol-2,6-disulfonsäure, 1-Amino-8-naphthol-3,6-disulfonsäure, 1-Amino-8-naphthol-4,6-disulfonsäure, 2-Amino-8-naphthol-6-sulfonsäure, 2-Amino-8-naphthol-4,6-disulfonsäure, 2-Amino-5-naphthol-7-sulfonsäure, 1-Amino-5-naphthol-7-sulfonsäure, 1-Amino-8-naphthaol-4-sulfonsäure oder -5-sulfonsäure oder -6-sulfonsäure, 2-Aminophenol-4- oder -5-sulfonsäure, 3-Aminophenol-6-sulfonsäure, 1,6-Diamino-2-naphthol-4-sulfonsäure, 1,6-Diaminonaphthalin-4-sulfonsäure, 2,6-Diamino-4,8-disulfonaphthalin, 2,6-Diamino-1-naphthol-4,8-disulfonsäure, 1,3-Diaminobenzol-5-sulfonsäure, 1,3-Diamino-5-methylbenzol, 2,6-Diaminophenol-4-sulfonsäure, 1-(3′- oder -4′-Aminophenyl)-3-methyl-5-pyrazolon, 1-(3′- oder -4′-Aminophenyl)-pyrazolon(5)-3-carbonsäure oder deren niedere Alkylester, wie Methyl- oder Äthylester, 1-(4′-Amino-2′-sulfo-phenyl)-pyrazolon(5)-3-carbonsäure oder deren Methyl- oder Äthylester, 1-(5′-Amino-2′-sulfophenyl)-pyrazolon(5)-3-carbonsäure oder deren Methyl- oder Äthylester, 1-(2′-Methyl-5′-amino-3′-sulfo-phenyl)-pyrazolon(5)-3-carbonsäure oder deren Methyl- oder Äthylester, 4-Acetoacethylamino-anilin-2-sulfonsäure, 4-(ω-N-Methylamino-methyl)-anilin-2-sulfonsäure oder 3-(ω-N-Methylamino-methyl)-anilin-6-sulfonsäure, wobei in allen diesen Aminoverbindungen die eine primäre oder sekundäre Aminogruppe oder eine dieser beiden Aminogruppen entsprechend Formel (9) durch den faserreaktiven Acylrest der Verbindung der allgemeinen Formel (3) substituiert ist.

Diese Diazokomponenten und Kupplungskomponenten der Formel (9) können zur Herstellung der erfindungsgemässen Farbstoffe (1) in üblicher Weise mit aromatischen Aminen, beispielsweise solchen, die zur Herstellung von Azofarbstoffen in der Literatur allseits bekannt sind, und Kupplungskomponenten, beispielsweise solchen, die in der Literatur allgemein zur Herstellung von Azofarbstoffen bekannt sind, umgesetzt werden. Hierbei kann man nach den üblichen Verfahrensweisen zu Monoazofarbstoffen, Disazofarbstoffen oder Polyazofarbstoffen, beispielsweise Trisazofarbstoffen oder Tetrakisazofarbstoffen, gelangen. Die Azofarbstoffe können nachträglich durch übliche Methoden der Metallisierung (s. Houben-Weyl, «Methoden der organischen Chemie», 4. Ausgabe [1965], Band 10/3, S. 452 ff; Angewandte Chemie 70, 232–238 [1958]; Angewandte Chemie 64, 397 [1952] in die entsprechenden Schwermetallkomplexfarbstoffe, wie die Kupfer-, Kobalt- oder Chromkomplexfarbstoffe, übergeführt werden.

Aromatische Amine ohne faserreaktiven Rest der Formel (4), die als Diazokomponenten eingesetzt werden können, sind beispielsweise die Amine der Formeln (10a), (10b), (10c) und (10d) und die Diamine der Formel (10e):

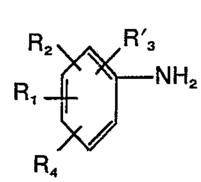

(10a)

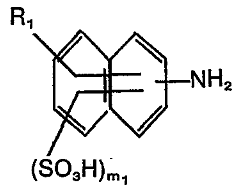

(10b)

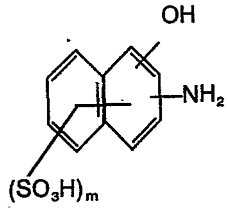

(10c)

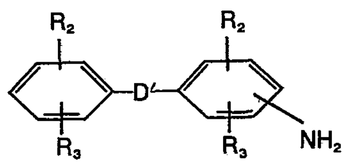

(10d)

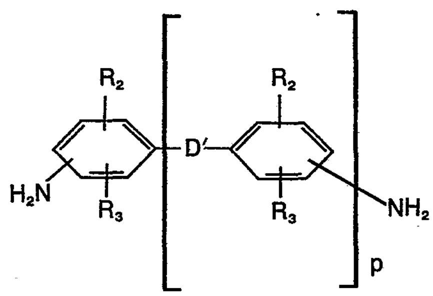

(10e)

worin $R_1$, $R_2$, $R_3$, $R_4$ und m die obengenannten Bedeutungen haben und D′ eine direkte Bindung ist oder für die Gruppe –NHCO–, –CO–NH–, –CO–, $-SO_2-$, –CH = CH–, –N = N–, –O– oder $-CH_2-$ steht und $m_1$ die Zahl Null, 1, 2 oder 3 und p die Zahl Null oder 1 bedeuten.

Kupplungskomponenten ohne faserreaktiven Rest der Formel (4), die zur Herstellung der erfindungsgemässen Farbstoffe eingesetzt werden können, sind beispielsweise die Verbindungen der Formeln (10f), (10g), (10h), (10i), (10j), ($10k_1$), ($10k_2$), (10m), (10n), (10p), (10q), ($10r_1$), ($20r_2$), ($10r_3$) und (10s)

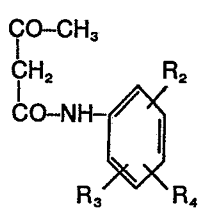

(10f)

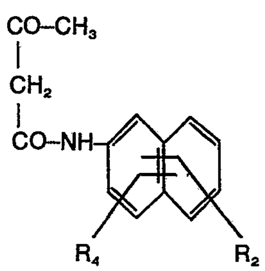

(10g)

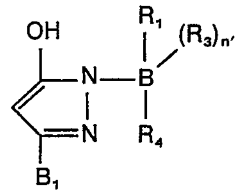

(10h)

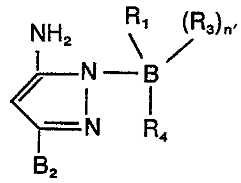

(10i)

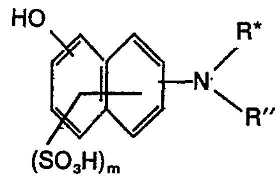

(10j)

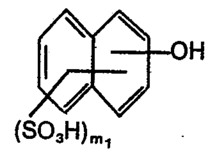

($10k_1$)

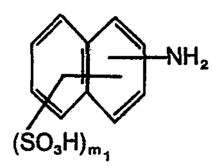

($10k_2$)

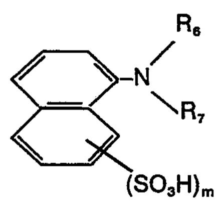

(10m)

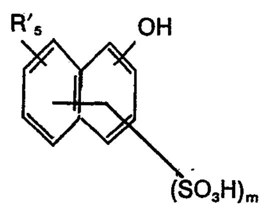

(10n)

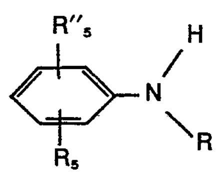

(10p)

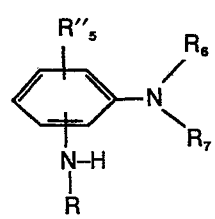

(10q)

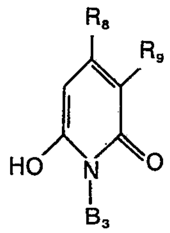

(10r₁)

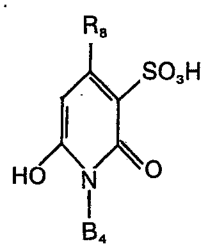

(10r₂)

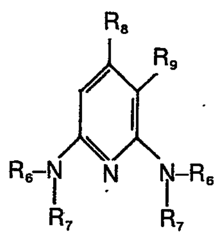

(10r₃)

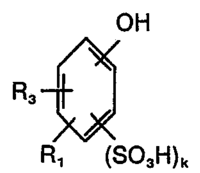

(10s)

in welchen R, $R_1$, $R_2$, $R_3$, $R_4$, $R_6$, $R_7$, $R_8$, $R_9$, R″, R*, k, m, $m_1$, n′, B, $B_1$ und $B_2$ die obengenannten Bedeutungen haben,

$R_5$ ein Wasserstoffatom, eine niedere Alkylgruppe, wie eine Methyl- oder Äthylgruppe, eine niedere Alkoxygruppe, wie eine Methoxy- oder Äthoxygruppe, ein Chlor- oder Bromatom ist,

$R''_5$ ein Wasserstoffatom, eine niedere Alkylgruppe, wie Methyl- oder Äthylgruppe, eine niedere Alkoxygruppe, wie Methoxy- oder Äthoxygruppe, ein Chlor- oder Bromatom, eine niedere Alkanoylaminogruppe, wie Acetylaminogruppe, oder eine Amino-, Ureido-, niedere Alkylsulfonylamino-, niedere Alkylamino- oder niedere Dialkylaminogruppe darstellt,

$B_3$ ein Wasserstoffatom oder eine niedere Alkylgruppe, wie eine Methyl- oder Äthylgruppe, die durch Phenyl oder Sulfophenyl substituiert sein kann, bedeutet,

$B_4$ ein Wasserstoffatom, eine niedere Alkylgruppe, eine durch niederes Alkoxy, wie Methoxy, oder durch eine Acetylamino-, Benzoylamino- oder eine Cyangruppe substituierte niedere Alkylgruppe, eine niedere Alkenylgruppe, eine Cyclohexylgruppe, die Phenylgruppe oder ein durch Substituenten aus der Gruppe Carboxy, Sulfo, Benzoylamino, Acetylamino oder Chlor substituiertes Phenylrest oder eine Aminogruppe ist, die durch Phenyl, niederes Alkyl, Acetyl, Benzoyl, Hydroxy oder Methoxy substituiert ist, und

$R'_5$ für die Phenylureido-, eine niedere Alkanoylamino- oder niedere Alkenoylamino-, wie Acetylamino-, Propionylamino-, Acryloylaminogruppe, oder eine Benzoylaminogruppe, die durch Chlor, Methyl, Methoxy, Nitro, Sulfo und/oder Carboxy substituiert sein kann, steht.

Verbindungen ohne faserreaktiven Rest der allgemeinen Formel (4), die zur Herstellung von erfindungsgemässen Farbstoffen der allgemeinen Formel (1) sowohl als Diazo- als auch als Kupplungskomponenten eingesetzt werden können, so beispielsweise für die Herstellung von Dis- und Polyazofarbstoffen, sind beispielsweise Verbindungen der allgemeinen Formeln ($10t_1$), ($10t_2$), (10u), (10v), (10w), (10x), (10y) und (10z)

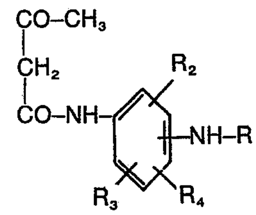

($10t_1$)

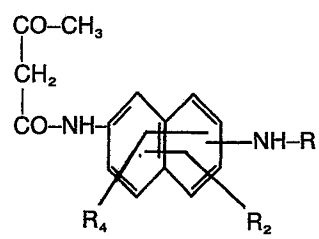

($10t_2$)

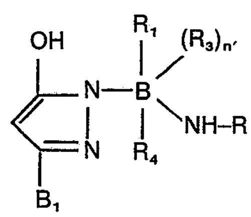

(10u)

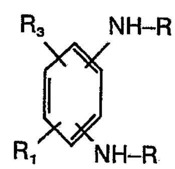

(10v)

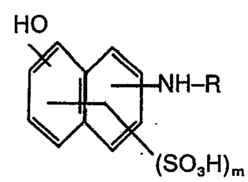

(10w)

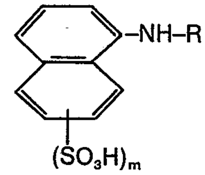

(10x)

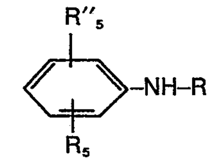 (10y)

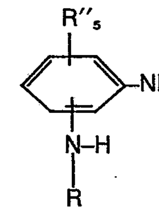 (10z)

in welchen B, $B_1$, $B_2$, R, $R_1$, $R_2$, $R_3$, $R_4$, $R_5$, $R''_5$ m und n' die oben angegebene Bedeutung haben und ein R zwingend für ein Wasserstoffatom steht, falls die Verbindung als Diazokomponente verwendet wird. Ihre Verwendung als Kupplungskomponenten ermöglicht darüberhinaus, die erhaltene Azoverbindung an der freien Aminogruppe der Kupplungskomponente mit dem faserreaktiven Säurechlorid der allgemeinen Formel (3) umzusetzen.

Aromatische Amine ohne faserreaktive Gruppe der allgemeinen Formel (4), die zur Herstellung der erfindungsgemässen Farbstoffe als Diazokomponenten eingesetzt werden können und beispielsweise den allgemeinen Formeln (10a), (10b), (10c) und (10d) entsprechen, sind beispielsweise:
Anilin, 2-Methoxy-anilin, 2-Äthoxy-anilin,
2-Methyl-anilin, 4-Nitro-2-amino-anisol,
4-Chlor-2-amino-anisol, 4-Methyl-anilin,
4-Methoxy-anilin, 2-Nitro-4-aminoanisol,
2-Methoxy-5-methyl-anilin, 4-Nitro-anilin,
2-Chlor-4-nitroanilin, 2,6-Dichlor-4-nitroanilin,
2-Nitro-4-methyl-anilin, 2,5-Dimethoxyanilin,
2,5-Dimethyl-anilin, 2,4-Dimethyl-anilin,
4-Butyl-anilin, 2-Cyano-4-nitroanilin,
2,5-Diäthoxy-anilin, 4-Cyano-anilin, 2-Chloranilin,
3-Chloranilin, 4-Chloranilin, 2,5-Dichloranilin,
4-Chlor-2-nitroanilin, 4-Chlor-2-methyl-anilin,
3-Chlor-2-methyl-anilin, 4-Chlor-2-aminotoluol,
4-(p'-Tolylsulfonyl)-anilin,
2-Äthoxy-1-naphtylamin, 1-Naphthylamin,
2-Naphthylamin,
4-Benzylamino-2,5-diäthoxyanilin,
3-Amino-4-methyl-benzonitril,
4-Methylsulfonylanilin, 2-Trifluormethyl-anilin,
1-Trifluormethyl-3-chloranilin,
2-Aminobenthiazol,
2-(2'-Aminophenyl)-benzotriazol,
1-Aminoanthrachinon-3-sulfonsäure,
2,4-Dichlor-anilin-5-carbonsäure,
2-Aminobenzoesäure, 4-Amino-benzoesäure,
3-Chlor-anilin-6-carbonsäure,
Anilin-2- oder -3- oder -4-sulfonsäure,
2,5-Disulfo-anilin, 2,4-Disulfo-anilin,
2-Amino-toluol-4-sulfonsäure,
2-Amino-anisol-4-sulfonsäure,
2-Amino-anisol-5-sulfonsäure,
2-Äthoxy-anilin-5-sulfonsäure,
2-Äthoxy-anilin-4-sulfonsäure,
4-Sulfon-2-amino-benzoesäure,
2,5-Dimethoxy-anilin-4-sulfonsäure,
2,4-Dimethoxyanilin-5-sulfonsäure,
2-Methoxy-5-methyl-anilin-4-sulfonsäure,
4-Amino-anisol-3-sulfonsäure,
4-Amino-toluol-4-sulfonsäure,
2-Amino-toluol-5-sulfonsäure,
2-Chlor-anilin-4-sulfonsäure,
2-Chlor-anilin-5-sulfonsäure,
2-Brom-anilin-4-sulfonsäure,
2,6-Dichlor-anilin-4-sulfonsäure,
2,6-Dimethyl-anilin-3-sulfonsäure oder -4-sulfonsäure,
2,4,6-Trimethyl-anilin-3-sulfonsäure,
4-Acetylamino-2-sulfo-anilin,
1-Aminonaphthalin-4-sulfonsäure,
2-Naphthylamin-5-sulfonsäure oder -6- oder -8-sulfonsäure,
2-Aminonaphthalin-3,6,8-trisulfonsäure,
2-Aminonaphthalin-6,8-disulfonsäure,
2-Aminonaphthalin-1,6-disulfonsäure,
2-Aminophenol-4-sulfonsäure,
2-Aminophenol-5-sulfonsäure,
3-Aminophenol-6-sulfonsäure,
4- oder 5-Nitro-2-aminophenol,
4-Nitro-6-sulfo-2-aminophenol,
1-Hydroxy-2-aminonaphthalin-6,8- oder -4,6-disulfonsäure,
2-Aminonaphthalin-1-sulfonsäure,
2-Aminonaphthalin-1,6- oder -4,8-disulfonsäure,
6-Nitro-2-hydroxy-4-sulfo-aminonaphthalin,
6-Nitro-2-aminonaphthalin-4,8-disulfonsäure,
4-Amino-diphenylamin,
4-Amino-4'-methoxy-diphenylamin,
4-Amino-4'-methoxy-diphenylamin-3-sulfonsäure,
4-(2'-Methylphenyl-azo)-2-methylanilin,
4-Aminoazobenzol,
4'-Nitrophenylazo-1-aminonaphthalin,
4'-Amino-3'-methyl-3-nitro-benzophenon, 4-Amino-2-methyl-benzophenon,
3-Amino-2,4-dimethyl-benzophenon,
2-Amino-4'-methoxy-benzophenon,
4-Amino-benzophenon,
4-(4'-Aminophenylazo)-benzolsulfonsäure,
4-(4'-Amino-3'-methoxyphenylazo)-benzolsulfonsäure,
2-Äthoxy-1-naphthylamin-6-sulfonsäure.

Aromatische Diamine ohne faserreaktive Gruppe der allgemeinen Formel (4), die als Tetrazokomponenten zur Herstellung der erfindungsgemässen Farbstoffe verwendet werden können und die beispielsweise der allgemeinen Formel (10e) entsprechen, sind beispielsweise:
1,3-Diaminobenzol,
1,3-Diaminobenzol-4-sulfonsäure,
1,4-Diaminobenzol,
1,4-Diaminobenzol-2-sulfonsäure,
1,4-Diamino-2-methyl-benzol,
1,4-Diamino-2-methoxy-benzol,
1,3,Diamino-4-methyl-benzol,
1,3-Diaminobenzol-5-sulfonsäure,
1,3-Diamino-5-methyl-benzol,
1,6-Diamino-naphthalin-4-sulfonsäure,
2,6-Diamino-4,8-disulfo-naphthalin,

3,3′-Diamino-diphenylsulfon,
4,4′-Diamino-diphenylsulfon,
3,3′-Diamino-diphenylsulfon-disulfonsäure,
4,4′-Diamino-stilben-2,2′-disulfonsäure,
2,7′-Diamino-diphenylsulfon,
2,7′-Diamino-diphenylsulfon-4,5-disulfonsäure,
4,4′-Diamino-benzophenon,
4,4′-Diamino-3,3′-dinitro-benzophenon,
3,3′-Diamino-4,4′-dichlor-benzophenon,
4,4′- oder 3,3′-Diamino-diphenyl,
4,4′-Diamino-3,3′-dichlor-diphenyl,
4,4′-Diamino-3,3′-dimethoxy- oder -3,3′-dimethyl- oder -2,2′-dimethyl-oder -2,2′-dichlor- oder -3,3′-diäthoxy-diphenyl,
4,4′-Diamino-3,3′-dimethyl-6,6′-dinitro-diphenyl,
4,4′-Diamino-2,2′- oder 3,3′-disulfo-diphenyl,
4,4′-Diamino-3,3′-dimethyl- oder -3,3′-dimethoxy- oder -2,2′-dimethoxy-6,6′-disulfo-diphenyl,
4,4′-Diamino-2,2′,5,5′-tetrachlor-diphenyl,
4,4′-Diamino-3,3′-dinitro-diphenyl,
4,4′-Diamino-2,2′-dichlor-5,5′-dimethoxy-diphenyl,
4,4′-Diamino-2,2′- oder -3,3′-dicarbonsäure,
4,4′-Diamino-3,3′-dimethyl-diphenyl-5,5′-disulfonsäure,
4,4′-Diamino-2-nitro-diphenyl,
4,4′-Diamino-3-äthoxy- oder -3-sulfo-diphenyl,
4,4′-Diamino-3,3′-dimethyl-5-sulfo-diphenyl,
4,4′-Diamino-diphenylmethan,
4,4′-Diamino-3,3′-dimethyldiphenylmethan,
4,4′-Diamino-2,2′,3,3′-tetramethyl-diphenylmethan,
4,4′-Diamino-diphenyläthan,
4,4′-Diaminostilben,
4,4′-Diamino-diphenylmethan-3,3′-dicarbonsäure,
1,2-Di-(4′-amino-phenoxy)-äthan.

Aromatische Monoamine und Diamine ohne faserreaktive Gruppe der allgemeinen Formel (4), die sowohl als Diazokomponenten als auch als Kupplungskomponenten bei der Herstellung von erfindungsgemässen Farbstoffen, so beispielsweise von Dis- und Polyazofarbstoffen, verwendet werden können und beispielsweise den allgemeinen Formeln ($10t_1$), ($10t_2$), (10u), (10v), (10w), (10x), (10y) und (10z) entsprechen, sind beispielsweise:

1,3-Diaminobenzol, 1,4-Diaminobenzol,
1,4-Diamino-2-methyl-benzol,
1,4-Diamino-2-methoxy-benzol,
1,3-Diamino-4-methyl- oder -methoxy-benzol,
1-Aminonaphthalin,
1-Aminonaphthalin-6- oder -7-sulfonsäure,
3-Acetylamino-anilin, 3-Methylanilin,
3-Chlor-anilin, 2-Amino-8-naphthol-6-sulfonsäure,
2-Amino-8-naphthol-4,6-disulfonsäure,
2-Amino-5-naphthol-7-sulfonsäure,
3-Amino-5-naphthol-7-sulfonsäure,
1-Amino-5-naphthol-7-sulfonsäure,
1-N-Acetoacetylamino-4-aminobenzol,
1-N-Acetoacetylamino-4-N-methyl-aminobenzol,
1-N-Acetoacetylamino-3-methyl-4-aminobenzol,
1-N-Acetoacetylamino-3-methoxy-4-aminobenzol,
1-(3′-Amino-phenyl)-3-methyl-pyrazolon(5),
1-(4′-Aminophenyl)-3-methyl-pyrazolon(5),
1-(3′- oder -4′-Aminophenyl)-3-carbäthoxy-pyrazolon(5),
1-(3′-Sulfo-4′-aminophenyl)-3-carbäthoxy-pyrazolon(5),
1-(3′-Amino-4′-sulfo-phenyl)-3-carbäthoxy-pyrazolon(5),
1-(2′,4′,6′-Trimethyl-3′-amino-5′-sulfo-phenyl)-3-carbäthoxy-pyrazolon(5),
1-(4′-Amino-phenyl)-3-methyl-pyrazolon(5),
1-(3′-Amino-6′-methyl-phenyl)-3-carboxy-pyrazolon(5).

Kupplungskomponenten ohne faserreaktive Gruppe der allgemeinen Formel (4), die zur Herstellung der erfindungsgemässen Farbstoffe verwendet werden können und beispielsweise den allgemeinen Formeln (10f) bis (10k) entsprechen, sind beispielsweise:

1,3-Diaminobenzol, 1,4-Diaminobenzol,
1,4-Diamino-2-methylbenzol,
1,4-Diamino-2-methoxybenzol,
1,3-Diamino-4-methyl- oder -methoxybenzol,
N,N,Dimethylanilin, N,N-Diäthylanilin,
N-Methyl-N-(β-acetoxyäthyl)-anilin,
N,N-Di-(β-cyanoäthyl)-anilin,
Phenol, Kresol, Resorcin, 2-Äthoxy-phenol,
4-Methylphenol, 3-Sulfophenol, 1-Naphthol,
2-Naphthol, 4-Sulfonaphthol, 5-Sulfonaphthol,
3,6-Disulfo-naphthol(8), 4,6-Disulfo-naphthol(8),
1-Aminonaphthalin, 2-Aminonaphthalin,
2-Methylamino-naphthalin,
2-Aminonaphthalin-6- oder -7-sulfonsäure,
2-Aminonaphthalin-3,6-8-trisulfonsäure,
2-Amino-naphthol(8)-6-sulfonsäure,
2-Amino-8-naphthol-4,6-disulfonsäure,
1-Amino-8-naphthol-3,6- oder -4,6-sulfonsäure,
1-Acetylamino-8-naphthol-3,6- oder -4,6-disulfonsäure,
1-Benzoylamino-8-naphthol-3,6- oder -4,6-disulfonsäure,
1-Naphthol-5,7-disulfonsäure,
1-Naphthol-3,6,8-trisulfonsäure,
2-Acetylamino-5-naphthol-7-sulfonsäure,
2-Benzoylamino-8-naphthol-6-sulfonsäure,
2-(p′-Tosylamino)-5-naphthol-7-sulfonsäure,
2-Acetylamino-8-naphthol-3,6-disulfonsäure,
2-Acetylamino-5-naphthol-1,7-disulfonsäure,
2-Benzoylamino-8-naphthol-6-sulfonsäure,
2-Phenylsulfonylamino-5-naphthol-7-sulfonsäure,
2-(N-Methyl-N-acetyl)-amino-8-naphthol-6-sulfonsäure,
N,N-Dimethyl-2-methoxy-5-methyl-anilin,
N-Methyl-N-(β-cyanoäthyl)-anilin,
N,N-Dimethyl-2,5-dimethoxy-anilin,
N,N-Bis-(β-hydroxyäthyl)-2-methoxy-5-chlor-anilin,
N-(β-Cyanoäthyl)-2,5-dimethoxyanilin,
N-(β-Cyanoäthyl)-2-chlor-anilin,
Acetoacetylnaphthylamid,
Acetoacetyl-2-naphthylamid-5-sulfonsäure,
N-Acetoacetylanilin-3- oder -4-sulfonsäure,
N-Acetoacetyl-2-methoxy-5-sulfo-anilin,
N-Acetoacetyl-4-methoxy-3-sulfo-anilin,
N-Acetoacetyl-2-methoxy-5-methyl-4-sulfo-anilin,
N-Acetoacetyl-2,5-dimethoxy-4-sulfo-anilin,
1-(4′-Sulfophenyl)-3-methyl-pyrazolon(5),

1-(3'-Sulfophenyl)-3-carboxy-pyrazolon(5),
1-(2'-Methoxy-4'-sulfophenyl)-3-carboxy-pyrazolon(5),
1-(3'-Sulfophenyl)-3-methyl-5-amino-pyrazol,
1-(4'-Sulfo-phenyl)-3-methyl-5-amino-pyrazol,
1-(2'Methoxy-5'-sulfo-phenyl)-3-methyl-5-aminopyrazol,
1-(2'-Methoxy-5'-methyl-4'-sulfophenyl)-3-methyl-5-aminopyrazol,
Acetoacetylamino-benzol,
Acetoacetylamino-2-methyl-benzol,
Acetoacetylamino-2-methoxy-benzol,
(N-Acetoacetyl-N-methyl)-anilin,
(N-Acetoacetyl-N-äthyl)-anilin,
1-N-Acetoacetylamino-4-aminobenzol,
1-N-Acetoacetylamino-4-N-methyl-aminobenzol,
1-N-Acetoacetylamino-3-methyl-4-aminobenzol,
1-N-Acetoacetylamino-3-methoxy-4-aminobenzol,
1-(3'-Amino-phenyl)-3-methyl-pyrazolon(5),
1-(4'-Aminophenyl)-3-methyl-pyrazolon(5),
1-(3'- oder -4'-Aminophenyl)-3-carbäthoxy-pyrazolon(5),
1-(3'-Sulfo-4'-aminophenyl)-3-carbäthoxy-pyrazolon(5),
1-(3'-Amino-4'-sulfo-phenyl)-3-carbäthoxy-pyrazolon(5),
1-(2',4',6'-Trimethyl-3'-amino-5'-sulfo-phenyl)-3-carbäthoxy-pyrazolon(5),
1-(4'-Amino-phenyl)-3-methyl-pyrazolon-5,
1-(3'-N-methylamino-phenyl)-3-carbomethoxy-pyrazolon-5282 1-(4'-N-Methylamino-phenyl)-3-methyl-5-aminopyrazol,
1-(3'-Amino-6'-methyl-phenyl)-3-carboxy-pyrazolon-5,
1-Amino-3-N-methylamino-benzol,
1-Amino-3-N-äthylamino-benzol,
2-N-Methylamino-8-naphthol-6-sulfonsäure,
3-Methyl-pyrazolon(5),
3-Carboxy- oder 3-Carbamoylpyrazolon(5),
1-Phenyl-3-methyl- oder -3-carboxy-pyrazolon(5),
1-(4'-Nitrophenyl)-3-methyl-pyrazolon(5),
1-(3'-Acetylaminophenyl)-3-carboxy-pyrazolon(5),
1-(3'-Carboxyphenyl)-3-methyl-pyrazolon(5),
2-Methylindol, 2,3-Hydroxynaphtoesäure,
8-Hydroxychinolin, 2-Hydroxychinolin,
2,5-Bis-(N,N-diäthylamino)-pyridin,
2-Hydroxy-4-methyl-5-cyano-pyridon,
Anilin-N-methan-sulfonsäure, o-Toluidin,
m-Toluidin, 2,6-Dimethylanilin,
2,5-Dimethylanilin, o-Äthylanilin,
m-Äthylanilin, o-tert.-Butyl-anilin, m-Chloranilin,
o-Anisidin, m-Anisidin, o-Phenetidin,
m-Phenetidin, N-Methyl-anilin, N-Äthyl-anilin,
N-β-Hydroxyäthyl-anilin, N-β-Acetoxyäthyl-anilin,
N-Butylanilin, 4-β-Cyanoäthyl-anilin,
N-β-Cyano-äthyl-m-toluidin, N-Äthyl-m-toluidin,
2-Amino-hydrochinon-dimethyl- oder -diäthyläther,
Kresidin, 3-Acetylamino-anilin, 3-Ureido-anilin,
3-Methylsulfonylamino-anilin,
2-Methoxy-5-acetylaminoanilin,
1-Amino-naphthalin-6-sulfonsäure,
1-Aminonaphthalin-7-sulfonsäure,
2-Methoxy-1-aminonaphthalin-6-sulfon-säure,
2-Äthoxy-1-aminonaphthalin-6-sulfonsäure,
2-Amino-5-naphthol-7-sulfonsäure,
2-Methylamino-8-naphthol-6-sulfonsäure.

Von den erfindungsgemässen Verbindungen der allgemeinen Formel (1) können als bevorzugt beispielsweise die Verbindungen der folgenden Formeln (11) bis (25) und deren Salze, bevorzugt Alkalimetallsalze, wie Natrium- und Kaliumsalze, genannt werden:

Die Farbstoffe der allgemeinen Formel (11)

$$R'\text{–}D\text{–}N=N\text{–}K \quad (11),$$

in welcher

D als Rest einer Diazokomponente den Benzolkern bedeutet, der durch 1, 2 oder 3 Substituenten substituiert sein kann, die der Gruppe niederes Alkyl, wie Methyl und Äthyl, niederes Alkoxy, wie Methoxy und Äthoxy, Carboxy, Acetylamino, Benzoylamino, Phenylamino, Sulfobenzoylamino, Sulfophenylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, oder

D ein Naphthalinkern ist, der durch 1 oder 2 Sulfonsäuregruppen und/oder eine Carboxy-, Methyl-, Methoxy-, Äthoxy- oder Nitrogruppe oder ein Chloratom substituiert sein kann, oder

D den Benzthiazol-2-yl-Rest bedeutet, der im Benzolkern durch eine Methyl-, Methoxy- oder Sulfonsäuregruppe substituiert sein kann, und

K als Rest einer Kupplungskomponente den 1- oder 2-Naphtholrest darstellt, der durch 1, 2 oder 3 Sulfonsäuregruppen und/oder eine Amino-, Methylamino-, Phenylamino-, Acetylamino-, Benzoylamino- oder N-Methyl-N-acetylamino-Gruppe substituiert sein kann, oder

K als Rest einer Kupplungskomponente den 1- oder 2-Amino-naphthalin-Rest bedeutet, der durch 1, 2 oder 3 Sulfonsäuregruppen und/oder eine Hydroxy- oder Acetylaminogruppe substituiert sein kann, oder

K als Rest einer Kupplungskomponente den 5-Amino-pyrazol-oder 5-Pyrazolon-Rest bedeutet, die in 3-Stellung durch Methyl, Carboxy, Carbamoyl, niederes Carbalkoxy, wie Carbomethoxy und Carbäthoxy, oder Phenyl substituiert sind und in 1-Stellung den Phenylkern oder Naphthylkern gebunden enthalten, wobei der Phenylrest durch 1 oder 2 Sulfonsäuregruppen und/oder 1 oder 2 Substituenten aus der Gruppe niederes Alkyl, niederes Alkoxy, Chlor, Brom, Nitro, Acetylamino und Carboxy und der Naphthylkern durch 1, 2 oder 3 Sulfogruppen und/oder einen Substituenten aus der Gruppe niederes Alkyl, niederes Alkoxy, Nitro, Acetylamino und Carboxy substituiert sein können, oder

K als Rest einer Kupplungskomponente für den Acetoacetylanilid- oder -naphthylamid-Rest steht, wobei der Phenylrest durch 1 oder 2 Substituenten substituiert sein kann, die der Gruppe niederes Alkyl, wie Methyl und Äthyl, niederes Alkoxy, wie Methoxy und Äthoxy, Carboxy, Acetylamino, Benzoylamino, Carbamoyl, durch niederes Alkyl

und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, oder

K als Rest einer Kupplungskomponente für den Anilinrest steht, der im Benzolkern durch 1 oder 2 Substituenten aus der Gruppe niederes Alkyl, niederes Alkoxy und Chlor oder durch eine Amino-, Methylamino-Äthylamino-, Dimethylamino-, Diäthylamino-, Ureido- oder Acetylaminogruppe substituiert sein kann und der am Stickstoffatom des Anilins durch niederes Alkyl mono- oder disubstituiert sein kann, wobei diese niederen Alkylgruppen durch eine niedere Alkoxy-, niedere Alkanoyloxy-, Hydroxy-, Cyan- oder Phenylgruppe substituiert sein können, oder dessen Stickstoffatom ausser durch einen dieser Alkylreste durch Phenyl als zweiten Substituenten substituiert ist, wobei die Azogruppe an K ortho-ständig zu der die Kupplung dirigierenden Hydroxygruppe bzw. ortho- oder para-ständig zu der die Kupplung dirigierenden Aminogruppe steht, und

R′ für den Rest der allgemeinen Formel (4) steht, wobei dieser faserreaktive Rest R′ an einem der aromatischen Kerne von D oder an einem von dessen niederen Alkylsubstituenten gebunden ist;

die Farbstoffe der allgemeinen Formel (12)

$$D{-}N{=}N{-}K{-}R' \qquad (12),$$

in welcher

D als Rest einer Diazokomponente den Benzolkern bedeutet, der durch 1, 2 oder 3 Substituenten substituiert sein kann, die der Gruppe niederes Alkyl, wie Methyl und Äthyl, niederes Alkoxy, wie Methoxy und Äthoxy, Carboxy, Acetylamino, Benzoylamino, Phenylamino, Sulfobenzoylamino, Sulfophenylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom Trifluormethyl und Sulfo angehören, oder

D ein Naphthalinkern ist, der durch 1, 2 oder 3 Sulfonsäuregruppen und/oder eine Carboxy-, Methyl-, Methoxy- Äthoxy-, Nitro- oder Acetylaminogruppe oder ein Chloratom substituiert sein kann, oder

D den Benzthiazol-2-yl-Rest bedeutet, der im Benzolkern durch eine Nitro-, Methyl-, Methoxy- oder Sulfonsäuregruppe substituiert sein kann, und

K den Rest des 1- oder 2-Naphthols darstellt, der durch 1, 2 oder 3 Sulfonsäuregruppen substituiert sein kann, oder

K den Rest des 1- oder 2-Amino-naphthalins oder des Naphthylens bedeutet, der durch 1, 2 oder 3 Sulfonsäuregruppen und/oder eine Hydroxygruppe substituiert sein kann, oder

K den Rest des 5-Aminopyrazol-4-yls oder 5-Pyrazolon-4-yls bedeutet, die in 3-Stellung durch Methyl, Carboxy, Carbamoyl, nideres Carbalkoxy, wie Carbomethoxy und Carbäthoxy, oder Phenyl substituiert sind und in 1-Stellung den Phenylkern oder Naphthylkern gebunden enthalten, wobei der Phenylrest durch 1 oder 2 Sulfonsäuregruppen und/oder 1 oder 2 Substituenten aus der Gruppe niederes Alkyl, niederes Alkoxy, Chlor, Brom, Nitro, Acetylamino und Carboxy und der Naphthylkern durch 1, 2 oder 3 Sulfogruppen und/oder einen Substituenten aus der Gruppe niederes Alkyl, niederes Alkoxy, Nitro, Acetylamino und Carboxy substituiert sein können, oder

K als Rest einer Kupplungskomponente für den Acetoacetylanilid- oder -naphthylamid-Rest steht, wobei der Phenylrest durch 1 oder 2 Substituenten substituiert sein kann, die der Gruppe niederes Alkyl, wie Methyl und Äthyl, niederes Alkoxy, wie Methoxy und Äthoxy, Carboxy, Acetylamino, Benzoylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, oder

K für den p-Phenylenrest steht, der durch 1 oder 2 Substituenten aus der Gruppe niederes Alkyl, niederes Alkoxy und Chlor oder durch eine Dimethylamino-, Diäthylamino-, Ureido- oder Acetylaminogruppe substituiert sein kann, und

R′ für den Rest der allgemeinen Formel (4) steht, wobei dieser faserreaktive Rest R′ an einem der aromatischen Kerne von K oder an einem von dessen niederen Alkylsubstituenten gebunden ist;

die Farbstoffe der allgemeinen Formel (13)

$$R'{-}D{-}N{=}N{-}E{-}N{=}N{-}K \qquad (13),$$

in welcher

D, K und R′ die für die allgemeine Formel (11) angegebenen Bedeutungen haben und

E als Mittelkomponente, die beim Aufbau der Farbstoffe zuerst als Kupplungskomponente, dann als Diazokomponente diente, den Phenylrest bedeutet, der durch einen Substituenten aus der Gruppe niederes Alkyl, niederes Alkoxy, Chlor, Acetylamino, Amino, Methylamino, Äthylamino, Dimethylamino, Diäthylamino und Ureido substituiert sein kann, oder den Naphthalinkern bedeutet, der durch eine Amino- oder Hydroxygruppe, die ortho- oder para-ständig zur ersten Azogruppe stehen, substituiert ist und der durch eine niedere Alkyl-, Nitro- oder Acetylaminogruppe und/oder durch 1 oder 2 Sulfonsäuregruppen substituiert sein kann, oder den Rest der Formel

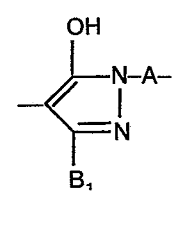

in welcher

$B_1$ für die Methyl- oder Carboxygruppe steht und A den Benzol- oder Naphthalinkern bedeutet, der noch durch 1 oder 2 Substituenten aus der Gruppe Sulfo, Carboxy, niederes Alkyl, niederes Alkoxy und Chlor substituiert sein kann;

die Farbstoffe der allgemeinen Formel (14)

$$D{-}N{=}N{-}E{-}N{=}N{-}K{-}R' \qquad (14),$$

in welcher

D, K und R′ die für die allgemeine Formel (12) angegebenen Bedeutungen haben und

E die für die allgemeine Formel (13) angegebene Bedeutung besitzt;

die Farbstoffe der allgemeinen Formel (15)

$$(R')_{m'}{-}D{-}N{=}N{-}K_1{-}N{=}N{-}D{-}(R')_{m''} \qquad (15)$$

in welcher

R′ für den faserreaktiven Rest der allgemeinen Formel (4) steht,

m′ und m″ gleich oder verschieden voneinander sind und jedes für die Zahl 0 oder 1 steht, wobei die Summe (m′ + m″) die Zahl 1 oder 2 ist,

D die für die allgemeinen Formeln (11) oder (12) angegebenen Bedeutungen haben, je nachdem, ob D einen faserreaktiven Rest R′ gebunden enthält, und

$K_1$ als Rest einer bifunktionellen Kupplungskomponente für den Amino-hydroxy-naphtylen-Rest steht, der durch eine oder zwei Sulfogruppen substituiert sein kann, oder den Diamino-phenylen-Rest darstellt, der noch durch eine Methyl- oder Sulfogruppe substituiert sein kann;

die Farbstoffe der allgemeinen Formel (16)

$$(R')_{m'}{-}K{-}N{=}N{-}D_1{-}N{=}N{-}K{-}(R')_{m''} \qquad (16)$$

in welcher

R′ den faserreaktiven Rest der allgemeinen Formel (4) bedeutet,

m′ und m″ die für die allgemeine Formel (15) angegebenen Bedeutungen haben und

K die für die allgemeine Formel (11) oder (12) angegebene Bedeutung hat, je nach dem, ob K einen faserreaktiven Rest R′ gebunden enthält, und

$D_1$ für den Phenylenrest, der durch eine Sulfo- oder Methylgruppe substituiert sein kann, oder für den Rest der Formel

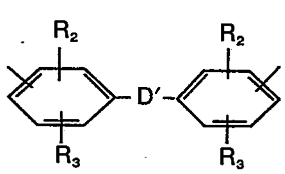

in welcher $R_2$, $R_3$ und D′ die zuvor angegebenen Bedeutungen haben;

die Farbstoffe der allgemeinen Formel (17)

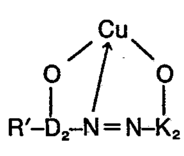

(17)

und deren entsprechenden 1:2-Chromkomplex- und 1:2-Kobaltkomplex-Derivate;

in der Formel (17) bedeuten:

$D_2$ den Benzolkern, der durch 1 oder 2 Substituenten substituiert sein kann, die der Gruppe niederes Alkyl, wie Methyl und Äthyl, niederes Alkoxy, wie Methoxy und Äthoxy, Carboxy, Phenylamino, Sulfobenzoylamino, Sulfophenylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, oder den Naphthalinkern, der durch 1, 2 oder 3 Sulfonsäuregruppen und/oder eine Carboxy-, Methyl-, Methoxy-, Äthoxy-, Nitro- oder Acetylaminogruppe oder ein Chloratom substituiert sein kann, wobei in $D_2$ das komplexbildende Sauerstoffatom ortho-ständig zur Azogruppe gebunden ist, und

$K_2$ den Naphthylenrest, der durch 1, 2 oder 3 Sulfonsäuregruppen und/oder eine Amino-, Methylamino-, Phenylamino-, Acetylamino-, Benzoylamino- oder N-Methyl-N-acetylamino-Gruppe substituiert sein kann, oder

$K_2$ den Rest des Pyrazol-4,5-ylens, der in 3-Stellung durch Methyl, Carboxy, Carbamoyl, niederes Carbalkoxy, wie Carbomethoxy und Carbäthoxy, oder Phenyl substituiert sind und in 1-Stellung den Phenylkern oder Naphthylkern gebunden enthalten, wobei der Phenylrest durch 1 oder 2 Sulfonsäuregruppen und/oder 1 oder 2 Substituenten aus der Gruppe niederes Alkyl, niederes Alkoxy, Chlor, Brom, Nitro, Acetylamino und Carboxy und der Naphthylkern durch 1, 2 oder 3 Sulfogruppen und/oder einen Substituenten aus der Gruppe niederes Alkyl, niederes Alkoxy, Nitro, Acetylamino und Carboxy substituiert sein können, oder

K als Rest einer Kupplungskomponente den des Acetoacetylanilids oder -naphthylamids, wobei der Phenylrest durch 1 oder 2 Substituenten substituiert sein kann, die der Gruppe niederes Alkyl, wie Methyl und Äthyl, niederes Alkoxy, wie Methoxy und Äthoxy, Carboxy, Acetylamino, Benzoylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, wobei in $K_2$ das komplexbildende Sauerstoffatom ortho-ständig zur Azogruppe gebunden ist, und

R′ den Rest der allgemeinen Formel (4), wobei dieser faserreaktive Rest R′ an einem der aromatischen Kerne von $D_2$ oder an einem von dessen niederen Alkylsubstituenten gebunden ist;

die Farbstoffe der allgemeinen Formel (18)

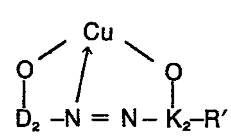

(18)

und deren entsprechenden 1:2-Chromkomplex- und 1:2-Kobaltkomplex-Derivate;

in der Formel (18) bedeuten:

$D_2$ den Benzolkern, der durch 1, 2 oder 3 Substituenten substituiert sein kann, die der Gruppe

niederes Alkyl, wie Methyl und Äthyl, niederes Alkoxy, wie Methoxy und Äthoxy, Carboxy, Acetylamino, Benzoylamino, Phenylamino, Sulfobenzoylamino, Sulfophenylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, oder

$D_2$ den Naphthalinkern, der durch 1, 2 oder 3 Sulfonsäuregruppen und/oder eine Carboxy-, Methyl-, Methoxy-, Äthoxy-, Nitro- oder Acetylaminogruppe oder ein Chloratom substituiert sein kann, wobei jeweils in $D_2$ das komplexbildende Sauerstoffatom ortho-ständig zur Azogruppe gebunden ist, und

$K_2$ den Naphthylenrest, der durch 1 oder 2 Sulfonsäuregruppen substituiert sein kann, oder

$K_2$ den Rest des Pyrazol-4,5-ylens, der in 3-Stellung durch Methyl, Carboxy, Carbamoyl, niederes Carbalkoxy, wie Carbomethoxy und Carbäthoxy, oder Phenyl substituiert sind und in 1-Stellung den Phenylkern oder Naphthylkern gebunden enthalten, wobei der Phenylrest durch 1 oder 2 Sulfonsäuregruppen und/oder 1 oder 2 Substituenten aus der Gruppe niederes Alkyl, niederes Alkoxy, Chlor, Brom, Nitro, Acetylamino und Carboxy und der Naphthylkern durch 1, 2 oder 3 Sulfogruppen und/oder einen Substituenten aus der Gruppe niederes Alkyl, niederes Alkoxy, Nitro, Acetylamino und Carboxy substituiert sein können, oder

$K_2$ als Rest einer Kupplungskomponente für den Acetoacetylanilid- oder -naphthylamid-Rest steht, wobei der Phenylrest durch 1 oder 2 Stubstituenten substituiert sein kann, die der Gruppe niederes Alkyl, wie Methyl und Äthyl, niederes Alkoxy, wie Methoxy und Äthoxy, Carboxy, Acetylamino, Benzoylamino, Carbamoyl, durch niederes Alkyl und/oder Phenyl mono- oder disubstituiertes Carbamoyl, Sulfamoyl, durch niederes Alkyl und/oder Phenyl mono- oder disubstituiertes Sulfamoyl, Cyan, Nitro, Chlor, Brom, Trifluormethyl und Sulfo angehören, und

R′ den Rest der allgemeinen Formel (4), wobei dieser faserreaktive Rest R′ an einem der aromatischen Kerne von $K_2$ oder an einem von dessen niederen Alkylsubstituenten gebunden ist:

die Farbstoffe der allgemeinen Formel (19)

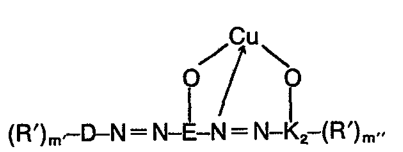
(19)

und deren entsprechenden 1:2-Chromkomplex- und 1:2-Kobaltkomplex-Derivate;

in der Formel (19) haben

R′, m′, m″, D und $K_2$ die für die allgemeinen Formeln (11), (15) und (17) bzw. (18) angegebenen Bedeutungen und

E stellt als Mittelkomponente, die beim Aufbau der Farbstoffe zuerst als Kupplungskomponente, dann als Diazokomponente diente, den Benzolkern dar, der durch einen Substituenten aus der Gruppe niederes Alkyl, niederes Alkoxy, Chlor, Acetylamino, Dimethylamino, Diäthylamino und Ureido substituiert sein kann, oder den Naphthalinkern dar, der durch niederes Alkyl, Nitro, Acetylamino oder Sulfo substituiert sein kann, oder den Rest der Formel

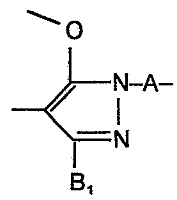

in welcher A und $B_1$ die für Formel (13) angegebenen Bedeutungen haben,

wobei in $K_2$ das komplexbildende Sauerstoffatom ortho-ständig zur Azogruppe gebunden ist;

die Farbstoffe der allgemeinen Formel (20)

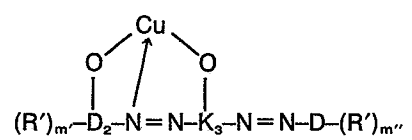
(20)

und deren entsprechenden 1:2-Chromkomplex- und 1:2-Kobaltkomplex-Derivate;

in dieser Formel (20) haben

R′, m′, m″, D und $D_2$ die für die Formeln (15) und (17) bzw. (18) angegebenen Bedeutungen, wobei in $D_2$ das komplexbildende Sauerstoffatom ortho-ständig zur Azogruppe gebunden ist, und

$K_3$ steht als Rest der bifunktionellen Kupplungskomponente Aminonaphthol für den Naphthylenrest, der eine oder zwei Sulfogruppen gebunden enthalten kann;

die Farbstoffe der allgemeinen Formel (21)

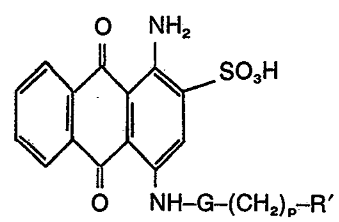
(21),

in welcher

G für den Phenylen- oder Naphthylen- oder den bivalenten Rest des Diphenylsulfons, des Stilbens, des Diphenylamins, des Diphenylmethans oder des Azobenzols steht, wobei jeder der aromatischen Kerne von G noch durch 1 oder 2 Substituenten aus der Gruppe Sulfo, niederes Alkyl, niederes Alkoxy, Chlor und Carboxy substituiert sein können, und

R′ und p die weiter obengenannten Bedeutungen haben;

die Farbstoffe der allgemeinen Formel (22)

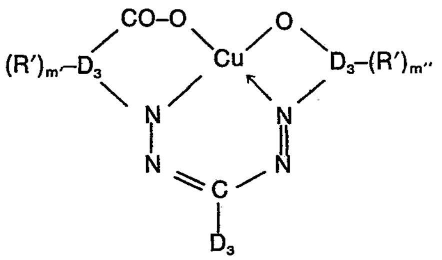

(22)

in welcher

$D_3$ jeweils gleich oder verschieden voneinander sind und jedes den Benzolkern bedeutet, der durch 1 oder 2 Substituenten aus der Gruppe Sulfo, Carboxy, Nitro, niederes Alkyl, niederes Alkoxy und Chlor substituiert sein kann, oder den Naphthalinkern bedeutet, der durch 1, 2 oder 3 Substituenten aus der Gruppe Sulfo, Carboxy, Nitro, Acetylamino und Chlor substituiert sein kann, und

R', m' und m'' die für die Formeln (11) und (12) bzw. (15) angegebenen Bedeutungen haben;

die Farbstoffe der allgemeinen Formel (23)

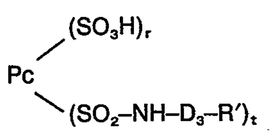

(23)

in welcher

$D_3$ die obengenannte Bedeutung hat,

Pc einen metallfreien oder metallhaltigen, vorzugsweise den Kupfer-, Kobalt- oder Nickelphthalocyaninrest bedeutet,

R' den faserreaktiven Rest der allgemeinen Formel (4) darstellt,

r eine Zahl zwischen 1 und 3 und

t eine Zahl zwischen 1 und 4 bedeuten, wobei die Summe von (r + t) höchstens 4 ist und wobei im Falle, dass t für die Zahl 4 steht, der Rest $D_3$ zwingend mindestens eine Sulfogruppe enthält;

die Farbstoffe der allgemeinen Formel (24)

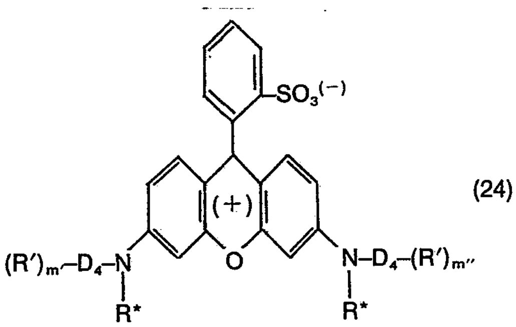

(24)

in welcher

$D_4$ gleich oder verschieden voneinander sein können und jedes einen niederes Alkylenrest bedeutet, der durch eine niedere Alkoxy-, niedere Alkanoyloxy-, Cyan-, Sulfo-, Carboxy- oder Hydroxygruppe substituiert sein kann, oder die obengenannte Bedeutung von $D_3$ besitzt und

R*, R', m' und m'' die obengenannten Bedeutungen haben;

die Farbstoffe der allgemeinen Formel (25)

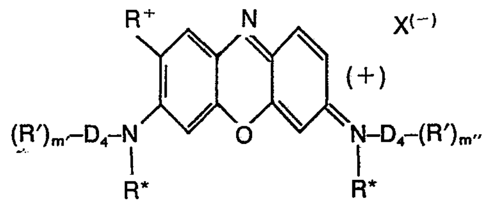

(25)

in welcher

$D_4$, R*, R', m' und m'' die obengenannten Bedeutungen haben und

$R^+$ für ein Wasserstoffatom, für eine niedere Alkylgruppe, wie Methylgruppe, oder für eine niedere Alkoxygruppe, wie Methoxygruppe, steht und

$X^{(-)}$ ein farbloses Anion bedeutet.

Von den erfindungsgemässen Verbindungen der allgemeinen Formel (1) sind weiterhin bevorzugt die Farbstoffe der allgemeinen Formel (11), in welcher der Formelrest R'–D– für einen faserreaktiven Rest der allgemeinen Formel (26a), (26b), (26c), (26d), (26e) oder (26f)

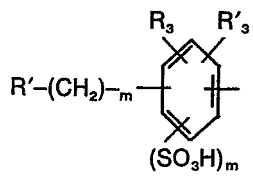

(26a)

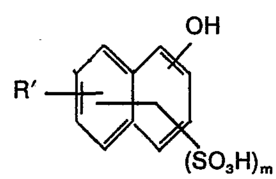

(26b)

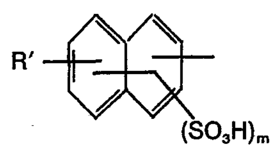

(26c)

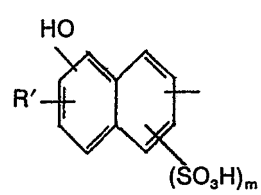

(26d)

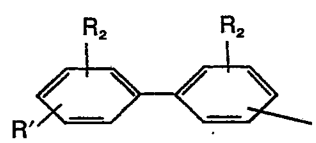

(26e)

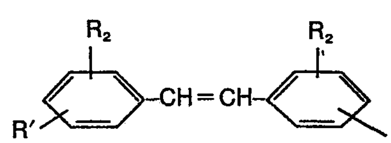

(26f)

mit $R_2$, $R_3$, $R'_3$, m und R' die weiter oben angegebenen steht und der Formelrest –K einen Rest der allgemeinen Formel (27a), (27b), (27c), (27d),

(27e), (27f), (27g), (27h), (27i), (27j), (27k), (27m), (27n), (27p) oder (27q)

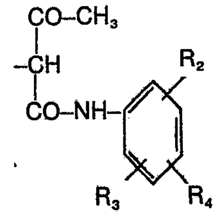
(27a)

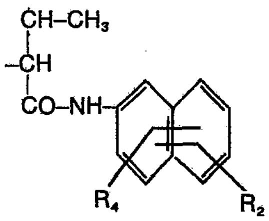
(27b)

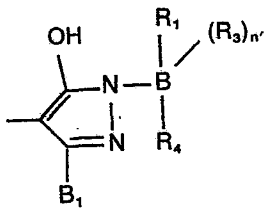
(27c)

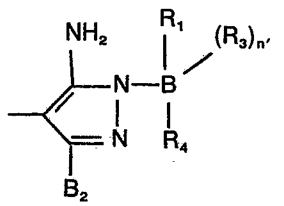
(27d)

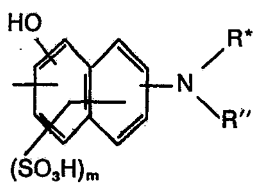
(27e)

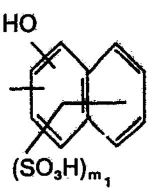
(27f)

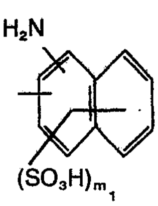
(27g)

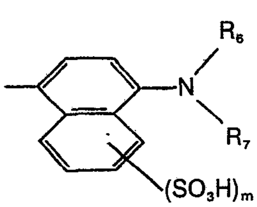
(27h)

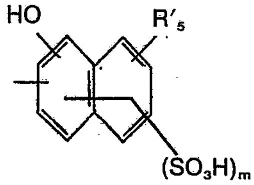
(27i)

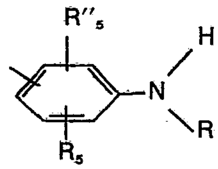
(27j)

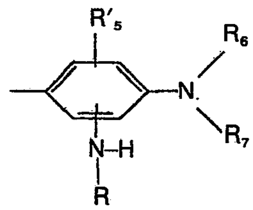
(27k)

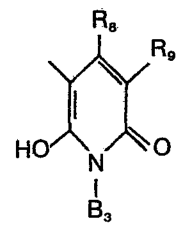
(27m)

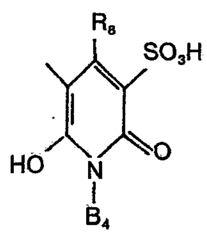
(27n)

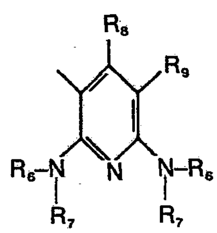
(27p)

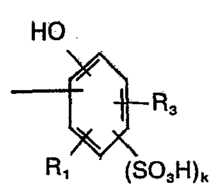
(27q)

mit R, $R_1$, $R_2$, $R_3$, $R_4$, $R_5$, $R_6$, $R_7$, $R_8$, $R_9$, $R'_5$, $R''_5$, R*, R'', k, m, $m_1$, n', B, $B_1$, $B_2$, $B_3$ und $B_4$ der obengenannten Bedeutungen darstellt, des weiteren die Farbstoffe der allgemeinen Formel (12), in welcher D einen Rest der allgemeinen Formel (27r), (27s), (27t) oder (27u)

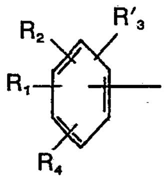

(27r)

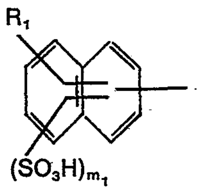

(27s)

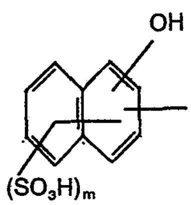

(27t)

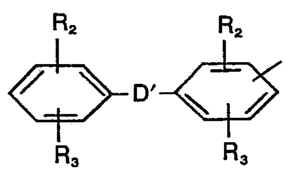

(27u)

mit $R_1$, $R_2$, $R_3$, $R_4$, D′, m und $m_1$ der oben genannten Bedeutung darstellt und der faserreaktive Formelrest –K–R′ einen Rest der allgemeinen Formel (26g), (26h), (26i), (26j), (26k), (26m), (26n), (26p), (26q) oder (26r)

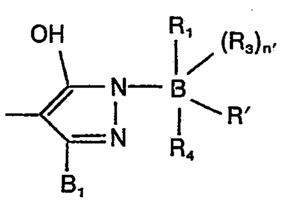

(26g)

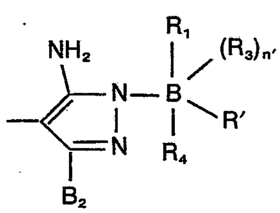

(26h)

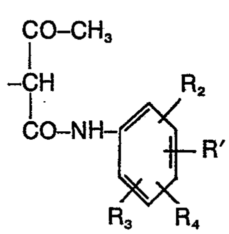

(26i)

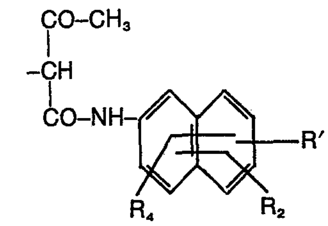

(26j)

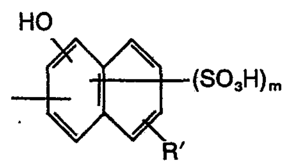

(26k)

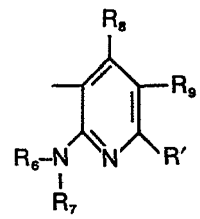

(26m)

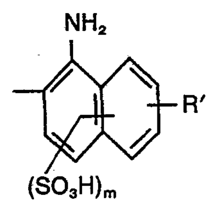

(26n)

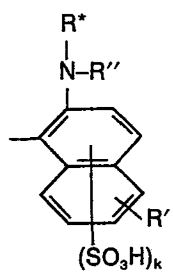

(26p)

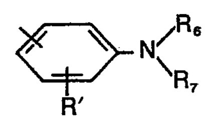

(26q)

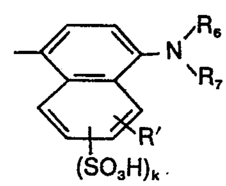

(26r)

bedeutet, in welchen R′, $R_1$, $R_2$, $R_3$, $R_4$, $R_5$, $R_6$, $R_7$, $R_8$, $R_9$, k, m, n′, B, $B_1$, $B_2$, R″ und R* die anfangs angegebenen Bedeutungen haben, des weiteren die Farbstoffe der allgemeinen Formel (13), in welcher der Formelrest R′–D– einen der Reste der obigen allgemeinen Formeln (26a), (26b), (26c), (26d), (26e) oder (26f) und der Formelrest –K einen der Reste der obengenannten Formeln (27a), (27b), (27c), (27d), (27e), (27f), (27g), (27h), (27i),

(27j), (27k), (27m), (27n), (27p) oder (27q) bedeuten und der Formelrest E für einen Rest der allgemeinen Formel (28a), (28b) oder (28c)

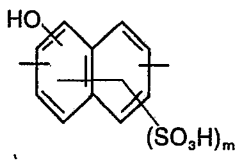

(28a)

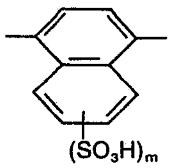

(28b)

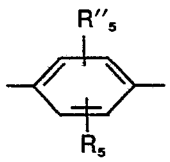

(28c)

steht, in welchen $R_5$, $R''_5$ und m die oben angegebenen Bedeutungen haben, des weiteren die Farbstoffe der allgemeinen Formel (14), in welcher der Formelrest D einen Rest der obigen Formeln (27r), (27s), (27t) oder (27u), der Formelrest –K–R′ einen Rest der obigen Formeln (26g), (26h), (26i), (26j), (26k), (26m), (26n), (26p), (26q) oder (26r) und der Formelrest E für einen Rest der obigen Formeln (28a) (28b) oder (28c) bedeuten, des weiteren die Farbstoffe der allgemeinen Formel (15) und der allgemeinen Formel (16), in welchen m′ und m″ die obengenannten Bedeutungen haben und die Formelreste D, falls m′ oder m″ für die Zahl Null steht, und die Formelreste R′–D–, falls m′ oder m″ für die Zahl 1 steht, die eben genannten Bedeutungen der obigen Formeln (27r), (27s), (27t) oder (27u) bzw. (26a) bis (26f) haben, die Formelreste K, falls m′ oder m″ für die Zahl Null steht, und die Formelreste R′–K– bzw. –K–R′, falls m′ oder m″ für die Zahl 1 steht, die eben genannten Bedeutungen der obigen Formelreste (27a) bis (27q) bzw. der Formelreste (26g) bis (26r) besitzen, $K_1$ den Naphthylenrest bedeutet, der durch eine Aminogruppe in dem einen aromatischen Kern und durch eine Hydroxygruppe in dem zweiten aromatischen Kern substituiert ist und der noch durch eine oder zwei Sulfogruppen substituiert sein kann, und $D_1$ die anfangs für die allgemeine Formel (16) angegebene Bedeutung hat, des weiteren die Farbstoffe der allgemeinen Formel (17), in welcher der Formelrest

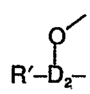

ein Formelrest der allgemeinen Formel (29a) oder (29b)

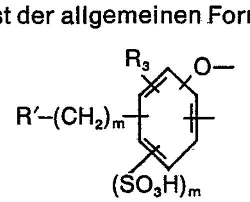

(29a)

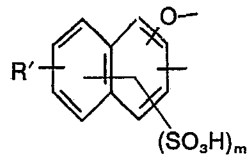

(29b)

mit R′, m und $R_3$ der obengenannten Bedeutungen ist, und in welcher der Formelrest

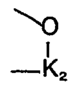

einen Rest der allgemeinen Formel (29c), (29d), (29e), (29f) oder (29g)

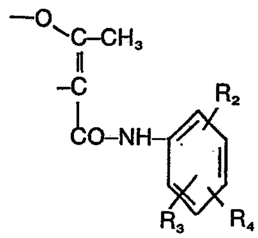

(29c)

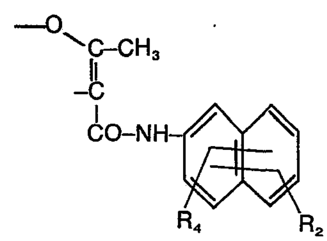

(29d)

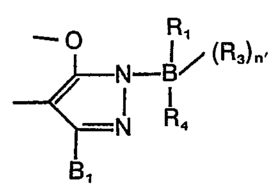

(29e)

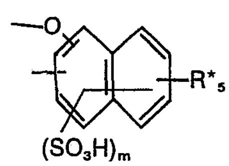

(29f)

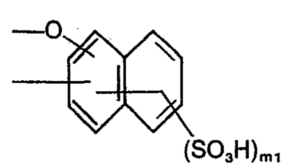

(29g)

darstellt, in welchen $R_1$, $R_2$, $R_3$, $R_4$, B, $B_1$, m und $m_1$ die anfangs angegebenen Bedeutungen haben, wobei jeweils die komplexbildenden Sauerstoffatome ortho-ständig zu den Azogruppen gebunden sind, und $R^*_5$ für ein Wasserstoffatom steht oder die obige Bedeutung von $R'_5$ hat;

des weiteren die Farbstoffe der allgemeinen Formel (18), in welcher der Formelrest

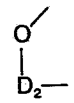

einen Rest der allgemeinen Formel (29h) oder (29i)

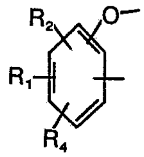

(29h)

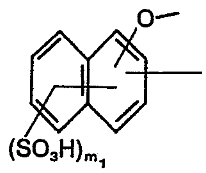

(29i)

in welcher $R_1$, $R_2$, $R_4$ und $m_1$ die obengenannten Bedeutungen haben, und in welcher der Formelrest

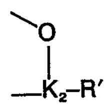

einen Rest der allgemeinen Formel (29j), (29k), (29m) oder (29n)

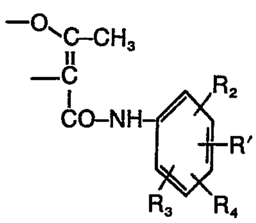

(29j)

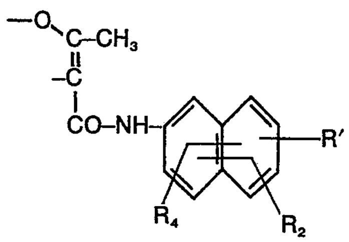

(29k)

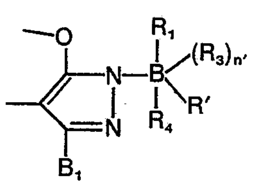

(29m)

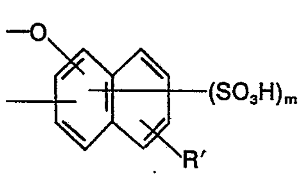

(29n)

darstellt, in denen R', $R_1$, $R_2$, $R_3$, $R_4$, B, $B_1$, m und n' die anfangs angegebenen Bedeutungen haben, wobei jeweils die komplexbildenden Sauerstoffatome ortho-ständig zu den Azogruppen gebunden sind,

des weiteren die Farbstoffe der allgemeinen Formeln (19) und (20), in welchen m' und m'' die obengenannten Bedeutungen haben und die Formelreste D, falls m' oder m'' für die Zahl Null steht, bzw. der Formelrest R'–D– bzw. –D–R', falls m' oder m'' die Zahl 1 bedeutet, die obenangegebenen Reste der Formeln (27q) bis (27s) bzw. (26a) bis (26f) haben, die Formelreste

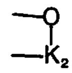 oder 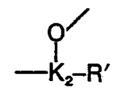

eine der oben angegebenen Reste der allgemeinen Formeln (29c) bis (29g) bzw. (29j) bis (29n) bedeuten und die Formelreste

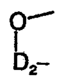 oder 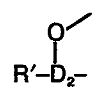

einen der Reste der oben angegebenen Formeln (29h) oder (29i) bzw. (29a) oder (29b) bedeuten und der Formelrest

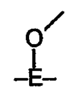

den nachstehenden Formelrest

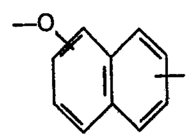

wobei dieser Naphthylenkern noch durch 1 oder 2 Sulfogruppen substituiert sein kann, und der Formelrest

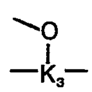

einen Rest der nachstehenden Formel

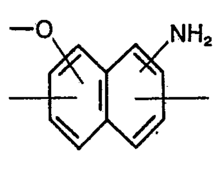

bedeutet, wobei der Naphthylenkern noch durch 1 oder 2 Sulfogruppen substituiert sein kann.

Organische Verbindungen der allgemeinen Formel (2), mit denen man durch Umsetzung eines faserreaktiven Säurehalogenids der allgemeinen Formel (3) zu den erfindungsgemässen Verbindungen der allgemeinen Formel (1) gelangt, sind bevorzugt die Mono- und Diamino-Verbindungen der allgemeinen Formeln (30) bis (44):

R–NH–D–N = N–K (30),

D–N = N–K–NH–R (31),

R–NH–D–N = N–E–N = N–K (32),

D–N = N–E–N = N–K–NH–R (33),

$(R–NH)_{m'}$–D–N = N–$K_1$–N = N–D–$(NH–R)_{m''}$ (34),

$(R–NH)_{m'}$–K–N = N–$D_1$–N = N–K–$(NH–R)_{m''}$ (35),

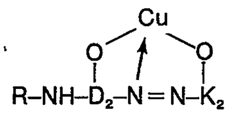
(36),

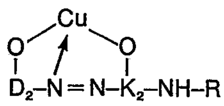
(37),

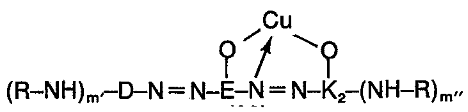
(38)

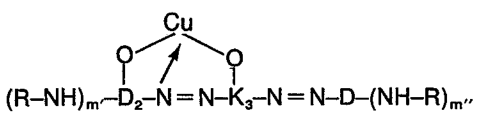
(39)

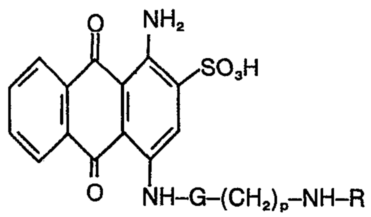
(40),

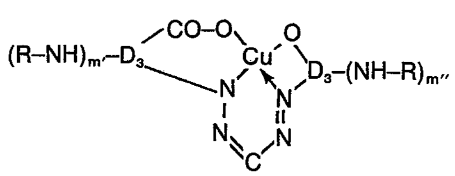
(41)

$D_3$

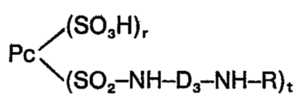
(42)

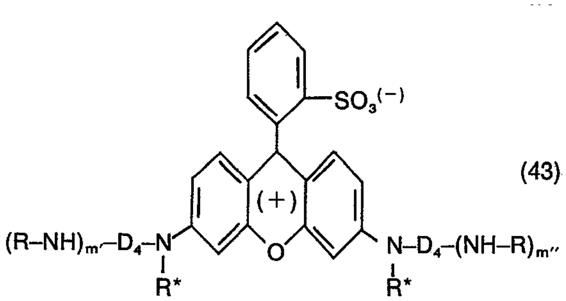
(43)

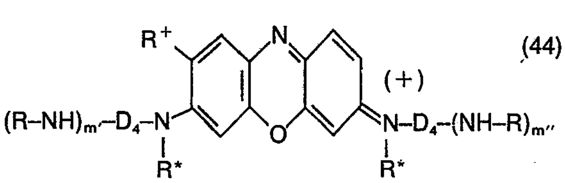
(44)

in welchen R, D, E, K, $D_1$, $D_2$, $K_1$, $K_2$, $K_3$, m′, m″, G, p, $D_3$, Pc, r, t, $D_4$, $R^+$, R* und $X^{(-)}$ die oben angegebenen Bedeutungen für die allgemeinen Formeln (11) bis (25) besitzen.

Die erfindungsgemässen Verbindungen der allgemeinen Formel (1) lassen sich aus ihren Syntheselösungen nach allgemein bekannten Methoden für wasserlösliche Verbindungen isolieren, so beispielsweise durch Ausfällen aus dem Reaktionsmedium mittels eines Elektrolyten, wie beispielsweise Natriumchlorid oder Kaliumchlorid, oder aber durch Eindampfen der Reaktionslösung selbst, beispielsweise durch Sprühtrocknung. Falls die letztgenannte Art der Isolierung der Verbindung der allgemeinen Formel (1) gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

In manchen Fällen kann die wässrige Lösung der Verbindung der allgemeinen Formel (1), gegebenenfalls nach Zusatz einer Puffersubstanz, auch direkt als Flüssigpräparation der Anwendung zur Faserveredlung, wie z.B. zum Färben, falls Q für einen Farbstoffrest steht, zugeführt werden.

Die erfindungsgemässen Verbindungen der allgemeinen Formel (1) sind geeignet, Leder oder Materialien aus natürlichen, regenerierten oder synthetischen stickstoffhaltigen Fasern oder aus natürlichen, regenerierten oder synthetischen hydroxygruppenhaltigen Fasern gemäss den Eigenschaften des Restes Q zu veredeln. Der faserreaktive Anker in den Verbindungen der allgemeinen Formel (1) besitzt die Eigenschaft, mit NH- und OH-Gruppen, – mit den Hydroxygruppen vorzugsweise im alkalischen Bereich, – unter Abspaltung eines Halogens aus dem Pyridazonylrest eine kovalente Bindung einzugehen und somit eine echte Bindung mit der Faser zu erzeugen.

Die vorliegende Erfindung betrifft somit ebenfalls die Verwendung der erfindungsgemässen Verbindungen der allgemeinen Formel (1) zur Faserveredlung von Materialien aus natürlichen, regenerierten oder synthetischen stickstoffhaltigen Fasern oder von natürlichen, regenerierten oder synthetischen hydroxygruppenhaltigen Fasern oder von Leder bzw. ein Verfahren zur Faserveredlung von solchen Materialien (Substraten), bei welchem man eine Verbindung der allgemeinen Formel (1), vorzugsweise aus wässriger Lösung, auf das Substrat appliziert und sie, gegebenenfalls unter der Einwirkung eines Alkalis und von Wärme, auf dem Substrat fixiert.

Stickstoffhaltige Fasermaterialien sind z.B. Fasern aus synthetischen Polyurethanen oder Polyamiden, wie Polyamid-6.6, Polyamid-6, Polyamid-11 und Polyamid-4, natürliche Fasermaterialien aus Polyamiden sind Seide und Wolle und andere Tierhaare.

Hydroxygruppenhaltige Fasermaterialien sind insbesondere Cellulosefasermaterialien, vorzugsweise Baumwolle und andere Pflanzenfasern, wie Leinen, Hanf, Jute, des weiteren die

entsprechenden Fasern aus regenerierter Cellulose, wie beispielsweise Viskose oder Kupferseide.

Die erfindungsgemässen Verbindungen lassen sich auf den genannten Substraten nach den für faserreaktive Verbindungen, beispielsweise nach den für faserreaktive Farbstoffe, allgemein bekannten Anwendungstechniken applizieren. So geht man allgemein in der Weise vor, dass man die Verbindungen der allgemeinen Formel (1) in wässriger Lösung, gegebenenfalls in Anwesenheit eines üblichen Verdickungsmittels und/oder gegebenenfalls anderer Hilfsmittel, die beispielsweise das Aufziehvermögen, das Egalisiervermögen und Migriervermögen verbessern können, auf das Substrat aufbringt, wobei die wässrige Lösung der Verbindung der allgemeinen Formel (1) schwach sauer, neutral oder alkalisch eingestellt sein kann.

Auf natürlichen, regenerierten oder synthetischen Polyamidfasern oder Polyurethanfasern oder auf Leder werden die Verbindungen der allgemeinen Formel (1) in üblicher Weise aus einer wässrig sauren bis wässrig neutralen Lösung (pH-Bereich etwa zwischen 3 und 6,5), vorzugsweise gemäss den Methoden des Ausziehverfahrens, auf die Faser aufgebracht und dort in der Wärme, insbesondere bei einer Temperatur zwischen 60 und 130 °C, fixiert.

So kann man beispielsweise dem die Verbindung der allgemeinen Formel (1) enthaltenden Bad Essigsäure oder Essigsäure und Ammoniumacetat als Puffer zufügen, um den gewünschten pH-Wert zu erhalten. Zwecks Erreichung einer brauchbaren Egalität der Färbungen empfiehlt sich ein Zusatz an üblichen Egalisiermitteln, beispielsweise auf Basis eines Umsetzungsproduktes von Cyanurchlorid mit der dreifach molaren Menge einer Aminobenzolsulfonsäure und/oder einer Aminonaphthalinsulfonsäure und/oder auf Basis eines Umsetzungsproduktes von Stearylamin mit Äthylenoxid. Die Applikation und Fixierung der erfindungsgemässen Verbindung auf dem Substrat kann gemäss dem Ausziehverfahren sowohl bei Siedetemperatur als auch bei einer höheren Temperatur, wie beispielsweise bei 105 bis 120 °C, unter Druck ausgeführt werden, wobei man die Färbung zweckmässig unter langsamer Temperatursteigerung auf 60 °C, nach einiger Zeit unter langsamer Temperatursteigerung auf höhere Temperatur, beginnt.

Bei der Veredelung von hydroxygruppenhaltigen Fasermaterialien wird die Verbindung der allgemeinen Formel (1) in der Regel aus schwach saurer bis alkalischer Lösung auf die Faser aufgebracht und auf ihr in Gegenwart eines alkalischen Mittels, das erforderlichenfalls nachträglich der wässrigen Lösung zugesetzt oder auf die Faser aufgebracht wird, fixiert.

Man erhält auf diese Weise veredelte Fasermaterialien, die für die Verbindungen der allgemeinen Formel (1) gute Nassechtheitseigenschaften besitzen.

Im Einzelnen kann man beispielsweise gemäss üblichen Methoden zur Aufbringung von faserveredelnden faserreaktiven Verbindungen und deren Fixierung auf der Faser, die insbesondere für faserreaktive Farbstoffe zahlreich in der Literatur beschrieben sind, beispielsweise folgendermassen vorgehen:

Zur Faserveredelung von hydroxygruppenhaltigen Fasermaterialien, wie beispielsweise Cellulose, wird das Material mit einer Lösung der Verbindung der allgemeinen Formel (1) geklotzt und eventuell nach Zwischentrocknen des geklotzten Materials mit einer alkalischen Lösung überklotzt oder durch ein alkalisches Bad geführt; diese alkalischen Lösungen können Raumtemperatur oder höhere Temperatur besitzen, beispielsweise eine Temperatur zwischen 15 und 80 °C.

Alkalisch reagierende Stoffe, die in diesen Fixierlösungen verwendet werden können, sind beispielsweise Natriumhydroxid, Natriumcarbonat, Natriumhydrogencarbonat, Kaliumhydroxid, Kaliumcarbonat, Trinatriumphosphat oder Natrium- oder Kaliumsilikat. Die so alkalisch behandelte Ware wird anschliessend entweder bei einer Temperatur zwischen 20 und 50 °C abgelegt (beispielsweise abgetafelt oder auf eine Docke gewickelt) und dort mehrere Stunden lang zur Fixierung der Verbindung der allgemeinen Formel (1) verweilen lassen, oder anschliessend zur Fixierung der erfindungsgemässen Verbindung der Einwirkung von feuchter oder trockener Hitze (so beispielsweise mittels Heissdampf, Heissluft oder Infrarotstrahlung) ausgesetzt. Im Falle der thermischen Fixierung können als Alkalien auch Verbindungen eingesetzt werden, die erst in der Wärme ihre alkalische Wirkung erzielen, wie beispielsweise Natriumacetat, Natriumformiat und Natriumtrichloracetat.

Die alkalischen Mittel können aber auch bereits der wässrigen Farbstofflösung zugegeben werden. In diesem Falle behandelt man das Fasermaterial in der wässrig-alkalischen Lösung der Verbindung der Formel (1), vorteilhaft unter Zusatz eines Elektrolyts, wie Natriumchlorid oder Natriumsulfat, bei höheren Temperaturen, wie beispielsweise bei einer Temperatur zwischen 30 und 110 °C, wobei man vorteilhaft unter langsamer Temperatursteigerung des Ausziehbades auf 60 °C mit der Behandlung der Faser beginnt und die Fixierung anschliessend bei Temperaturen bis zu 110 °C weiterführt und beendet. Ebenfalls ist es möglich, die Faser, wie z.B. die Cellulosefaser, mit einer Lösung einer alkalisch reagierenden Verbindung vorzubehandeln, sodann, gegebenenfalls nach Zwischentrocknung, mit der wässrigen Lösung der Verbindung der Formel (1) zu imprägnieren und anschliessend bei Raumtemperatur, vorzugsweise aber unter Wärmebehandlung, die faserreaktive Verbindung darauf zu fixieren.

Bringt man die Verbindungen der allgemeinen Formel (1) in Form von Druckpasten auf das Fasermaterial, so verwendet man üblicherweise Verdickungsmittel, wie Natriumalginat, Johannisbrotkernmehläther, Celluloseäther, Traganth oder Gummi Arabicum, gegebenenfalls unter Zusatz eines üblichen Druckhilfsmittels und der obengenannten alkalisch reagierenden Verbindungen. Diese Drucke werden dann bei einer Temperatur zwischen 70 und 230 °C, vorzugsweise zwischen 100 und 150 °C, mit Heissluft behandelt (thermofixiert) oder gedämpft. So können die erfindungsgemässen Verbindungen nach den üblichen Druckverfahren einphasig in Anwesenheit von Natriumbicarbonat oder eines anderen der obengenannten alkalischen Mittel mittels der Druckpaste auf die Cellulosefaser aufgebracht und anschliessend die Verbindung der allgemeinen Formel (1) durch Dämpfen bei 101 bis 103 °C fixiert werden, oder aber nach einem zweiphasigen Verfahren zuerst mit neutraler oder schwach saurer Druckpaste auf die Faser gebracht werden, worauf sie dann entweder durch Hindurchführen des bedruckten Materials durch ein heisses elektrolythaltiges alkalisches Bad oder aber durch Überklotzen mit einer alkalischen elektrolythaltigen Flotte und anschliessendem Verweilenlassen oder einer Hitzebehandlung auf der Faser fixiert werden. Verwendet man ein elektrolythaltiges alkalisches Bad, durch das das mit der erfindungsgemässen Verbindung imprägnierte Material zur Fixierung hindurchgeführt wird, so wendet man eine Temperatur von 60 bis 105 °C für das Bad an, so dass eine anschliessende Behandlung durch Heissluft oder Dampf entfallen kann. Behandelt man das mit der erfindungsgemässen Verbindung imprägnierte Material mit einem starken wässrigen Alkali, wie z.B. Natrium- oder Kaliumhydroxyd oder Natrium- oder Kaliumsilikat oder Trinatriumphosphat, so genügt zur Fixierung der erfindungsgemässen Verbindungen ein längeres Verweilenlassen der feuchten alkalisch imprägnierten Ware, vorzugsweise der Drucke, bei Raumtemperatur.

Die so erhaltenen faserveredelnden Materialien werden anschliessend in üblicher Weise nachbehandelt, gespült und getrocknet. Die auf diese Weise mit den erfindungsgemässen Verbindungen faserveredelten Materialien zeigen vorzügliche Nassechtheiten, die insbesondere bei der Anwendung von erfindungsgemässen Verbindungen, in denen der faserveredelnd wirkende organische Rest Q der Rest eines Farbstoffmoleküls ist, besonders vorteilhaft in Erscheinung treten; von den guten Nassechtheiten der faserveredelnden Substraten sind insbesondere beispielsweise die Sodakochechtheit, die Waschechtheit bei 60 und 95 °C, die Peroxydwaschechtheit, wie beispielsweise bei 95 °C, die alkalische und saure Schweissechtheit, die Wasserechtheit (schwer), die Chlorbleichechtheit und Chlorbadewasserechtheit zu nennen. Weiterhin besitzen die mit der Verbindung der allgemeinen Formel (1) faserveredelten Materialien eine gute Mercerisierechtheit, eine gute Reibechtheit und eine gute Abgasechtheit. Die Lichtechtheit in trockenem und feuchtem Zustand des Substrates, insbesondere die von mit erfindungsgemässen Farbstoffen veredelten Fasermaterialien, ist bemerkenswert gut. Bei Anwendung von erfindungsgemässen Farbstoffen zeigen die Drucke darüberhinaus eine hohe Konturenschärfe und eine sehr hohe Reinheit des Weissfonds. Die erfindungsgemässen Verbindungen der allgemeinen Formel (1) besitzen allgemein ein sehr gutes Aufziehvermögen und zeigen einen hohen Fixiergrad. Die erfindungsgemässen Verbindungen fixieren auf den unterschiedlichen Fasermaterialien eines Baumwolle/Zellwolle-Mischgewebes in gleicher Intensität; so zeigen sie auf diesen Fasermaterialien gleich Nuance und Farbstärke.

Die nachfolgenden Beispiele dienen zur Erläuterung der Erfindung. Die dort angegebenen Teile sind Gewichtsteile, die Angaben in Prozent beziehen sich auf Gewichtsprozent, sofern nichts anderes vermerkt. Volumenteile verhalten sich zu Gewichtsteilen wie Liter zu Kilogramm.

Beispiel 1

4-Aminobenzoesäure wird in üblicher Weise diazotiert, das Diazoniumsalz mittels Natriumsulfit in üblicher Weise in das 4-Carboxy-phenylhydrazin-hydrochlorid übergeführt. Dieses Hydrochlorid wird in konzentrierter Schwefelsäure als Lösungsmittel bei 80 °C mit der äquimolaren Menge an Maleinsäureanhydrid während 3 Stunden umgesetzt. Nach Abkühlen des Reaktionsansatzes gibt man ihn auf Eiswasser; die gebildete 3-Hydroxy-6-pyridazonyl(1)-benzol-4'-carbonsäure scheidet sich dabei ab. Sie wird abfiltriert, mit Wasser gewaschen und getrocknet. Sie besitzt die Formel

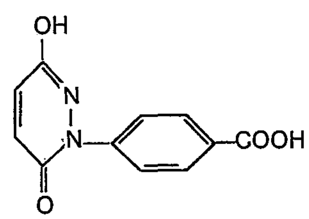

und zeigt einen Schmelzpunkt von 340 °C. Die Ausbeute beträgt, bezogen auf das eingesetzte Phenylhydrazin, 70% der Theorie.

Diese Carbonsäure-Verbindung wird in getrocknetem und pulverisiertem Zustand in Phosphoroxychlorid als Lösungsmittel suspendiert und mit der 3fach molaren Menge an Phosphorpentachlorid versetzt. Bei leichter Erwärmung erfolgt ab 40 °C eine starke Chlorwasserstoffentwicklung. Die Reaktion wird schliesslich unter weiterem Erwärmen durch drei- bis vierstündiges Erhitzen am Rückfluss bei einer Temperatur zwischen 100 und 105 °C zu Ende geführt. Anschliessend wird das Phosphoroxychlorid zuerst bei Normaldruck, sodann unter reduziertem Druck, vom Reaktionsprodukt, dem 3,5-Dichlor-6-pyridazonyl(1)-benzol-4'-carbonsäurechlorid, abdestilliert, das als pulvriger, poröser fester Körper zurückbleibt.

Aus Benzol, Toluol, Chlorbenzol und Essigsäureäthylester lässt es sich umkristallisieren. Es zeigt unter Zersetzung einen Schmelzpunkt von 190 bis 196 °C; die Elementaranalyse stimmt mit der Summenformel $C_{11}H_5Cl_3N_2O_2$, das NMR-Spektrum mit der Konstitution der Formel

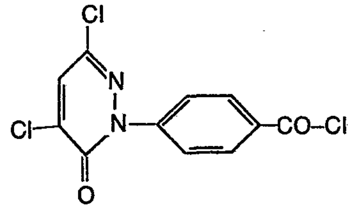

überein. Die Ausbeute ist quantitativ, bezogen auf die eingesetzte Carbonsäure-Verbindung.

Beispiel 2

β-Cyanoäthyl-hydrazin wird gemäss der Verfahrensweise der Deutschen Patentschrift 598 185 durch Umsetzung von Hydrazin mit Acrylnitril hergestellt, sodann analog der im obigen Beispiel 1 angegebenen Verfahrensweise mit Maleinsäureanhydrid in konzentrierter Schwefelsäure zum β-[3-Hydroxy-6-pyridazonyl(1)-]-propionitril umgesetzt. Da bei der Bildung des Pyridazonringes gleichzeitig Wasser frei wird, erfolgt unter den gegebenen Reaktionsbedingungen Verseifung der Nitrilgruppe zur entsprechenden Propionsäureverbindung. Diese wird gemäss den Angaben des Beispieles 1 aus Eiswasser isoliert, getrocknet und danach in Phosphoroxychlorid suspendiert. Mit der dreifach molaren Menge an Phosphorpentachlorid wird sie bei einer Reaktionszeit von 4 Stunden bei einer Reaktionstemperatur von bis zu 60 °C (höhere Temperaturen können zu Zersetzung führen) chloriert. Abschliessend wird unter reduziertem Druck das Phosphoroxychlorid abdestilliert. Zur Abtrennung von Verunreinigungen wird der ölige Rückstand in Methylenchlorid oder Aceton gelöst und von den unlöslichen Anteilen, die verworfen werden, abgetrennt. Das Säurechlorid lässt sich auch nicht im Vakuum unzersetzt destillieren. Es besitzt die Formel

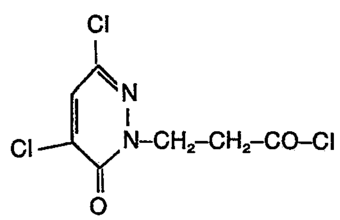

die den Werten der Elementaranalyse entspricht. Die Ausbeute beträgt 72% der Theorie, bezogen auf das eingesetzte β-Cyanoäthylhydrazin.

Zur Umsetzung des oben hergestellten Propionsäurechlorides mit einer aminogruppenhaltigen Verbindung zur Herstellung einer faserreaktiven Verbindung (wie beispielsweise eines Farbstoffes) oder eines Vorproduktes davon ist es nicht erforderlich, das Produkt erst zu reinigen. Die Umsetzung kann mit der von unlöslichen Anteilen befreiten Lösung des Dichlor-pyridazonylpropionsäurechlorids in Methylenchlorid vorgenommen werden.

Beispiel 3

Verfährt man in der in Beispiel 1 angegebenen Verfahrensweise, setzt jedoch anstelle der dort verwendeten 4-Aminobenzoesäure die äquivalente Menge an 4-Aminobenzolsulfonsäure ein, so erhält man die Verbindung der Formel

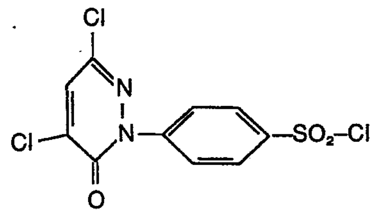

in einer Ausbeute von 80% der Theorie. Die Elementar- und NMR-Analyse entsprechen der oben angegebenen Strukturformel. Sein Schmelzpunkt ist nicht bestimmbar; es zersetzt sich oberhalb von 230 °C.

Beispiel 4

Verfährt man in der in Beispiel 1 angegebenen Verfahrensweise, setzt jedoch anstelle der dort verwendeten 4-Aminobenzoesäure die äquivalente Menge an 3-Aminobenzoesäure ein, so erhält man die Verbindung der Formel

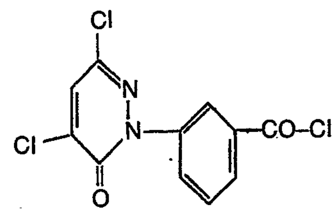

in einer Ausbeute von 90% der Theorie. Die Elementaranalyse entspricht der oben angegebenen Strukturformel. Die Verbindung besitzt einen Schmelzpunkt von 160–165 °C (unter Zersetzung).

Beispiel 5

Es wird eine neutrale wässrige Lösung von 31,9 Teilen 1-Amino-8-hydroxynaphthalin-3,6-disulfonsäure in 300 Teilen Wasser hergestellt, zu der 30 Teile kristallisiertes Natriumacetat gegeben werden. Bei einer Temperatur von 5 bis 10 °C werden unter gutem Rühren im Verlauf von 1 bis 2 Stunden 30,4 Teile 3,5-Dichlor-6-pyridazonyl(1)-benzol-4'-carbonsäurechlorid (Beispiel 1) in feinverteilter Form zugesetzt. Der Reaktionsansatz wird noch mehrere Stunden lang gerührt. Anschliessend wird die Lösung mit Natriumcarbonat auf einen pH-Wert von 6,5 gestellt, das Dinatriumsalz der Verbindung der Formel

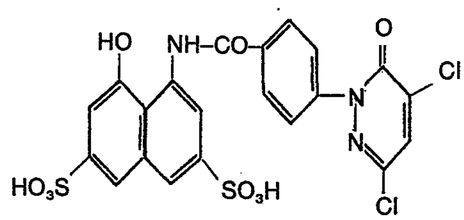

mit Natriumchlorid ausgesalzen, abgetrennt und getrocknet. Man erhält 73 Teile eines elektrolythaltigen grauen Pulvers mit einem Gehalt dieser Verbindung von etwa 73 Gew.-% entsprechend einer Ausbeute von 85% d.Th.

Die Abtrennung durch Aussalzen kann unterbleiben, wenn das Reaktionsgemisch gleich weiterverarbeitet wird, z.B. zu einem Azofarbstoff. Man ermittelt aus der Lösung sodann eine Ausbeute von 95% d.Th.

Beispiel 5a

Man verfährt wie in Beispiel 5 beschrieben, setzt jedoch anstelle der dort genannten 1-Amino-8-hydroxynaphthalin-3,6-disulfonsäure die gleiche Menge an 1-Amino-8-hydroxy-naphthalin-4,6-disulfonsäure ein. Man erhält in gleicher Ausbeute die Verbindung der Formel

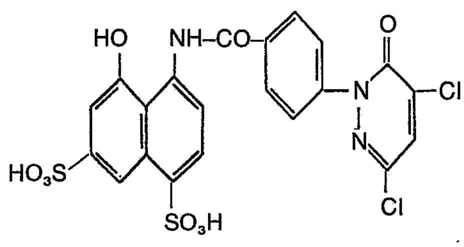

bzw. deren Natriumsalz.

Beispiel 5b

Verfährt man nach der in Beispiel 5 angegebenen Verfahrensweise, setzt jedoch anstelle der dort angegebenen Aminonaphthol-disulfonsäure äquivalente Mengen ähnlich gebauter Aminonaphtholsäuren ein, so beispielsweise die 2-Amino-8-hydroxynaphthalin-6-sulfonsäure, die 2-Methylamino-8-hydroxynaphthalin-6-sulfonsäure, die 3-Amino-8-hydroxynaphthalin-6-sulfonsäure oder deren N-Methyl-Verbindung, oder setzt entsprechende 1-(Aminophenyl)-pyrazolon-Verbindungen ein, so beispielsweise das 1-(3′-Amino-phenyl)-3-methyl-pyrazolon(5) oder das 1-(3′-Amino-5′-sulfo-6′-methyl-phenyl)-3-carboxy-pyrazolon(5), so erhält man in etwa gleich guter Ausbeute die entsprechenden erfindungsgemässen Verbindungen der allgemeinen Formel (9), die sich als Farbstoffvorprodukte für die Herstellung von faserreaktiven Azofarbstoffen verwenden lassen.

Beispiel 6

58,6 Teile der Verbindung von Beispiel 5 werden in Form des Dinatriumsalzes in 300 Teilen Wasser gelöst (anstelle dieser Lösung kann man auch die gemäss Beispiel 5 entstandene Syntheselösung, die vor dem Aussalzen erhalten wird, einsetzen). Zu ihr gibt man eine Diazoniumsalzlösung, die auf übliche Weise aus 17,3 Teilen 2-Amino-benzolsulfonsäure hergestellt wird. Man stellt mit Natriumcarbonat auf einen pH-Wert von 6 bis 7 ein und lässt die Kupplung etwa 2 Stunden lang durchführen, sobald die Prüfung auf freies Diazoniumsalz negativ ist, wird die Farbstofflösung geklärt, der gebildete Monoazofarbstoff mit Natriumchlorid oder Kaliumchlorid ausgesalzen. Er wird abgesaugt, mit Natriumchloridlösung gewaschen und getrocknet. Man erhält 95 Teile eines Farbstoffpulvers mit einem Gehalt von etwa 35% an Elektrolyt; der Farbstoff besitzt in Form der freien Säure die Konstitution

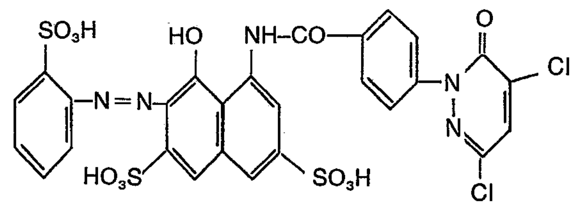

Er löst sich in Wasser mit roter Farbe und färbt nach den für faserreaktive Farbstoffe üblichen Färbe- und Druckverfahren, wie z.B. solche, die zuvor in der Beschreibung genannt sind, auf textilen Gebilden aus Polyamidfasern, Wolle und Seide, insbesondere aber auf Cellulosefasermaterialien, brillante rote Färbungen. Er zeichnet sich durch eine hohe Fixiergeschwindigkeit und Farbausbeute aus. Die mit ihm erhaltenen Färbungen und Drucke zeigen hervorragende Echtheiten, wie z.B. sehr gute Waschechtheiten bei 60 und 95 °C, gute Wasserechtheit (schwer), Sodakochechtheit, Peroxydwaschechtheit, saure und alkalische Schweissechtheit, Chlorbadewasserechtheit, saure Überfärbeechtheit, Mercerisierechtheit, Reibeechtheit, Abgasechtheit und eine gute Lichtechtheit. Die mit diesem Farbstoff hergestellten Drucke zeichnen sich durch ihre hohe Konturenschärfe und Reinheit des Weissfonds aus. Nicht fixierte Anteile des Farbstoffes lassen sich leicht auswaschen. Mischgewebe aus Baumwolle und Zellwolle zeigen auf den verschiedenen Fasermaterialien gleiche Nuancen und Farbstärke bei der Anwendung der üblichen Druck- und Färbeverfahren.

Beispiele 7 bis 22

Verfährt man gemäss der in Beispiel 6 angegebenen Verfahrensweise zur Herstellung des dort beschriebenen Monoazofarbstoffes, setzt jedoch anstelle der 2-Amino-benzol-sulfonsäure als Diazokomponente die in den nachfolgenden Beispielen tabellarisch angegebenen Diazokomponenten ein, so erhält man aus diesen Diazokomponenten und der Kupplungskomponente des Beispieles 5 in gleicher Reaktion und gleich guter Ausbeute anwendungstechnisch sehr gute Monoazofarbstoffe, die Fasermaterialien, insbesondere Cellulosefasermaterialien, in den angegebenen Farben echt färben:

| Bsp. | Diazokomponente | Farbton |
|---|---|---|
| 7 | 4-Amino-benzolsulfonsäure | rot |
| 8 | 2,5-Disulfo-anilin | rot |
| 9 | 2,4-Disulfo-anilin | rot |

(Fortsetzung)

| Bsp. | Diazokomponente | Farbton |
|---|---|---|
| 10 | 4-Acetylamino-2-sulfo-anilin | blaustichig rot |
| 11 | 5-Acetylamino-2-sulfo-anilin | blaustichig rot |
| 12 | 4-Acetylamino-2,6-disulfo-anilin | blaustichig rot |
| 13 | 2,5-Dimethoxy-4-sulfo-anilin | blaustichig rot |
| 14 | 2,4-Dimethoxy-5-sulfo-anilin | blaustichig rot |
| 15 | 2-Aminonaphthalin-4,8-disulfonsäure | blaustichig rot |
| 16 | 2-Aminonaphthalin-1-sulfonsäure | blaustichig rot |
| 17 | 2-Aminonaphthalin-1,5-disulfonsäure | blaustichig rot |
| 18 | 2-Aminonaphthalin-6,8-disulfonsäure | blaustichig rot |
| 19 | 1-Aminonaphthalin-4-sulfonsäure | blaustichig rot |
| 20 | 4-Amino-diphenylamin-2-sulfonsäure | blau |
| 21 | 4-Amino-diphenylamin-4'-methoxy-2-sulfonsäure | blau |
| 22 | 2-Methoxy-4-sulfo-anilin | blaustichig rot |

Beispiele 23 bis 49

Man verfährt in gleicher Weise wie in Beispiel 6 beschrieben und synthetisiert einen Monoazofarbstoff gemäss der allgemeinen Formel (1) durch Diazotierung der nachfolgend angegebenen aromatischen Amine und Kupplung mit den erfindungsgemässen Kupplungskomponenten, die den faserreaktiven Anker enthalten. Man erhält in gleicher Weise hochwertige neue Monoazofarbstoffe, die insbesondere Cellulosefasermaterialien sehr echt mit den angegebenen Nuancen färben. In den angegebenen Kupplungskomponenten ist zur Vereinfachung der Schreibweise der faserreaktive Anker mit dem Formelrest $R^1$ gekennzeichnet. Diese faserreaktive Gruppe entspricht dem Acylrest der Verbindung des Beispieles 1, so dass $R^1$ den folgenden Formelrest darstellt:

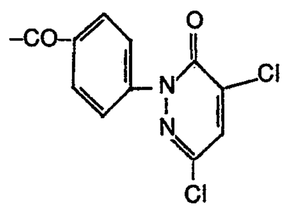

Kupplungskomponenten mit dem Formelrest $R^2$ besitzen die isomere faserreaktive Gruppe $R^2$ der Formel

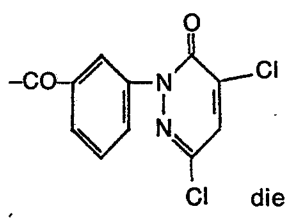

die dem Acylrest des Beispieles 4 entspricht.

| Bsp. | Diazokomponente | Kupplungskomponente | Farbton |
|---|---|---|---|
| 23 | 2,5-Disulfoanilin | 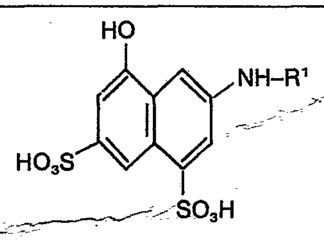 | rot |
| 24 | 4-Acetylamino-2,6-disulfo-anilin | dito | rot |
| 25 | 2-Amino-benzolsulfonsäure | dito | rot |
| 26 | 2-Aminonaphthalin-1,5-disulfonsäure | dito | blaust. rot |
| 27 | 2-Aminonaphthalin-4,8-disulfonsäure | dito | blaust. rot |
| 28 | 2-Aminonaphthalin-3,6,8-trisulfonsäure | dito | blaust. rot |
| 29 | 4-Amino-diphenylamin-4'-methoxy-2-sulfonsäure | dito | blau |

(Fortsetzung)

| Bsp. | Diazokomponente | Kupplungskomponente | Farbton |
|---|---|---|---|
| 30 | 2,5-Disulfo-anilin | 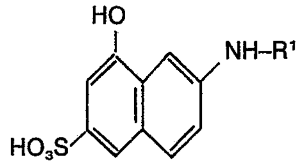 | orange |
| 31 | 2-Aminonaphthalin-1,5-disulfonsäure | dito | rotst.-orange |
| 32 | 2-Aminonaphthalin-3,6,8-trisulfonsäure | dito | braun |
| 33 | 2-Aminonaphthalin-3,6,8-trisulfonsäure | 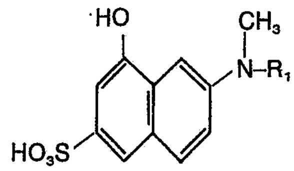 | braun |
| 34 | 2,5-Disulfo-anilin | dito | orange |
| 35 | 2,5-Disulfo-anilin | 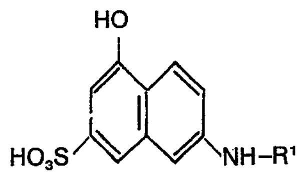 | gelbst.-orange |
| 36 | 2-Aminonaphthalin-3,6,8-trisulfonsäure | 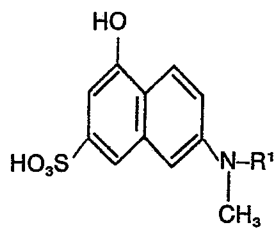 | scharlach |
| 37 | 2-Amino-benzolsulfonsäure | 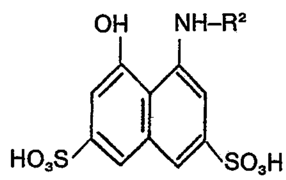 | rot |
| 38 | 2,5-Disulfo-anilin | dito | rot |
| 39 | 2-Aminonaphthalin-1,5-disulfonsäure | dito | blaust. rot |
| 40 | 2-Aminonaphthalin-4,8-disulfonsäure | dito | blaust. rot |
| 41 | 5-Acetylamino-2-sulfo-anilin | dito | rot |
| 42 | 4-Amino-diphenylamin-2′-methoxy-2-sulfonsäure | dito | blau |
| 43 | 4-Amino-benzolsulfonsäure | 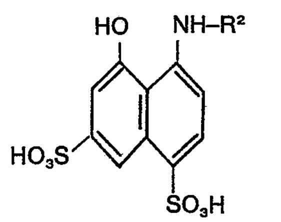 | rot |
| 44 | 2,5-Disulfo-anilin | dito | rot |
| 45 | 2-Aminonaphthalin-1-sulfonsäure | dito | blaust. rot |
| 46 | 2-Aminonaphthalin-1,5-disulfonsäure | dito | rot |
| 47 | 2-Aminonaphthalin-4,8-disulfonsäure | dito | blaust. rot |
| 48 | 2-Aminonaphthalin-3,6,8-trisulfonsäure | dito | blaust. rot |
| 49 | 4-Amino-diphenylamin-2′-methoxy-2-sulfonsäure | dito | blau |

Beispiel 50

Man löst 31,9 Teile 1-Amino-8-hydroxynaphthalin-3,6-disulfonsäure in 200 Teilen Wasser und gibt darauf 40 Teile kristallisiertes Natriumacetat zu. In diese Lösung tropft man innerhalb 1 Stunde bei einer Temperatur von unterhalb 10 °C und bei einem pH-Wert zwischen 3 und 6, der durch Zugabe eines Alkalis, wie einer wässrigen Lösung von Natriumcarbonat oder Natriumhydroxyd, eingestellt wird, eine Lösung von 25,6 Teilen β-(3,5-Dichlor-6-pyridazon-1-yl)-propionsäurechlorid in 30 Vol.-Teilen Aceton und rührt danach unter Konstanthaltung der Temperatur und des pH-Wertes 1 Stunde nach. Die so hergestellte acylierte Aminonaphtholsulfonsäure wird bei einem pH-Wert von 6 durch Zusatz von Natriumchlorid oder Kaliumchlorid ausgesalzen, abgesaugt und mit einer Natriumchloridlösung gewaschen und getrocknet. Man erhält das Alkalimetallsalz der Verbindung entsprechend der Konstitution

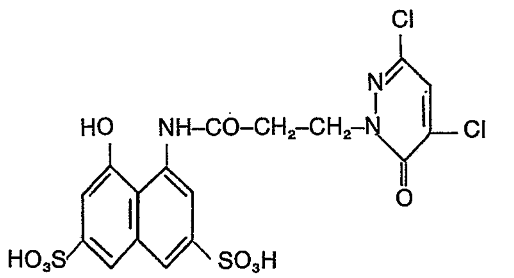

Beispiel 51

In gleicher Weise lassen sich faserreaktive Verbindungen herstellen, wenn man von entsprechenden Kupplungskomponenten, beispielsweise der Naphthol-, Pyrazolon-, Aminopyrazol- oder Acetoacetylarylid-Reihe ausgeht, die eine aromatisch gebundene primäre oder sekundäre Aminogruppe enthalten, und diese Verbindungen mit der faserreaktiven Verbindung des Beispieles 2 in analoger Weise zu der Methode des Beispieles 50 umsetzt. So lassen sich beispielsweise die β-(3,5-Dichlor-6-pyridazon-1-yl)-propionsäureamid-Verbindungen der 1-Amino-8-hydroxynaphthalin-4,6-disulfonsäure, der 2- oder 3-Amino-8-hydroxynaphthalin-6-sulfonsäure, der 2- oder 3-Methylamino-8-hydroxynaphthalin-6-sulfonsäure, des 1-(3′-Amino-5′-sulfo-6′-methyl-phenyl)-3-carboxy-5-pyrazolons oder des 1-(3′-Aminophenyl)-3-methyl-5-pyrazolons herstellen.

Beispiel 52

53,8 Teile der in Beispiel 50 beschriebenen Acylaminonaphthol-disulfonsäure werden zu einer wässrigen Diazoniumsalz-Suspension gegeben, die in üblicher Weise aus 17,3 Teilen 2-Amino-benzolsulfonsäure hergestellt wurde. Man stellt diesen Ansatz auf einen pH-Wert von 6 bis 7 mit Natriumcarbonat ein, rührt anschliessend einige Stunden nach und fällt die so synthetisierte Monoazoverbindung mittels Kaliumchlorid aus der Lösung aus. Sie wird abgenutscht, mit einer wässrigen Natriumchloridlösung gewaschen und getrocknet. Man erhält 90 Teile eines elektrolythaltigen roten Farbstoffpulvers, das 55% des Natriumsalzes der Verbindung der Formel

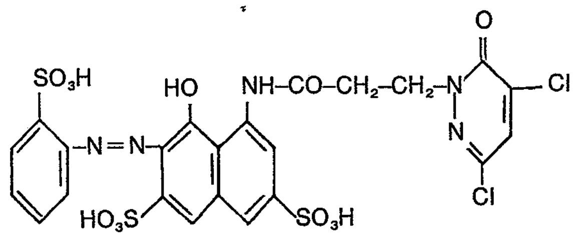

enthält. Mit ihr können Fasermaterialien aus natürlichen oder synthetischen Polyamiden oder Cellulosefasermaterialien nach den für faserreaktive Farbstoffe üblichen Färbe- und Druckverfahren gefärbt werden. Die erhaltenen Färbungen und Drucke zeigen ein brillantes Rot; die Echtheitseigenschaften sind sehr gut, insbesondere hiervon sind die Waschechtheit bei 60 und 95 °C, die Wasserechtheit (schwer), die Sodakochechtheit, die Peroxydwaschechtheit, die saure und alkalische Schweissechtheit, die Chlorbadewasserechtheit, die saure Überfärbeechtheit, die Mercerisierechtheit, die Reibeechtheit, die Abgasechtheit und die Lichtechtheit zu erwähnen.

Beispiele 53 bis 57

Verfährt man in der in Beispiel 52 angegebenen Weise zur Herstellung von Monoazoverbindungen, setzt jedoch anstelle der dort verwendeten Diazokomponente ein anderes aromatisches Amin ein, beispielsweise eines der nachfolgend genannten Amine, so erhält man ebenfalls wertvolle neue Monoazoverbindungen mit sehr guten Farbstoffeigenschaften, die Fasermaterialien in den angegebenen Nuancen echt färben:

| Bsp. | Diazokomponente | Farbton |
|---|---|---|
| 53 | 4-Amino-benzolsulfonsäure | rot |
| 54 | 2-Amino-benzolcarbonsäure | rot |
| 55 | 2,5-Disulfo-anilin | rot |
| 56 | 2,5-Dimethoxy-4-sulfo-anilin | blaust. rot |
| 57 | 2,4-Dimethoxy-5-sulfo-anilin | blaust. rot |

Beispiele 58 bis 67

Verfährt man in üblicher Weise zur Herstellung von Monoazoverbindungen, beispielsweise nach der in Beispiel 52 angegebenen Methode, und setzt die nachfolgend angegebenen Diazokomponenten und Kupplungskomponenten ein, so erhält man die entsprechenden Monoazofarbstoffe, die sehr gute faserreaktive Eigenschaften haben und Fastermaterialien in gleich guter Weise, wie für den Farbstoff des Beispieles 52 angegeben, echt färben.

Der in den nachfolgend beschriebenen Kupplungskomponenten befindliche Rest $R^3$ bedeutet den faserreaktiven Anker der Formel

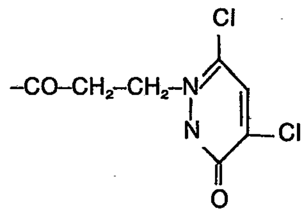

| Bsp. | Diazokomponente | Kupplungskomponente | Farbton auf Baumwolle |
|---|---|---|---|
| 58 | 2,5-Diäthoxy-4-sulfo-anilin | 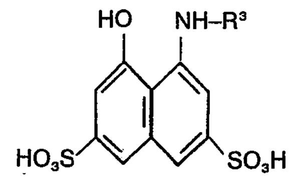 | blaust. rot |
| 59 | 2-Aminonaphthalin-1-sulfonsäure | dito | blaust. rot |
| 60 | 2-Aminonaphthalin-1,5-disulfonsäure | dito | blaust. rot |
| 61 | 2-Aminonaphthalin-4,8-disulfonsäure | dito | blaust. rot |
| 62 | 4-Amino-diphenylamin-4′-methoxy-2-sulfonsäure | dito | blau |
| 63 | 4-Acetylamino-2-sulfo-anilin | dito | blaust. rot |
| 64 | 2,5-Disulfo-anilin | 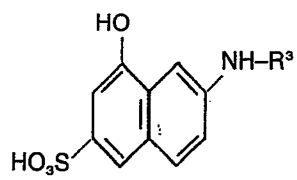 | orange |
| 65 | 2,5-Disulfo-anilin | 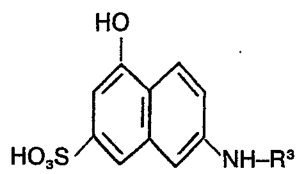 | orange |
| 66 | 2,5-Disulfo-anilin | 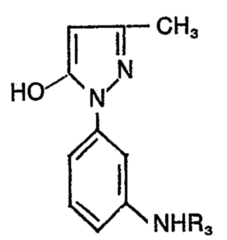 | grünst. gelb |
| 67 | 2,5-Disulfo-anilin | 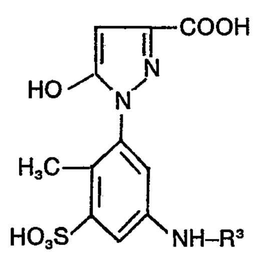 | gelb |

Beispiel 68

18,9 Teile 2-Aminophenol-5-sulfonsäure werden in 100 Teilen Wasser gelöst, die Lösung durch Aussenkühlung auf 5 bis 10 °C abgekühlt; zu ihr gibt man 14 Vol.-Teile einer 40%igen wässrigen Natriumnitritlösung, sodann 30 Vol.-Teile 30%ige wässrige Salzsäure. Man rührt eine Stunde nach und entfernt überschüssige salpetrige Säure mit Amidosulfonsäure. In die Lösung des so hergestellten Diazoniumsalzes gibt man anschliessend als Kupplungskomponente die wässrige Lösung von 58,6 Teilen des Natriumsalzes der 1-[4'-(3'',5''-Dichlor-6''-pyridazon-1''-yl-)-benzoylamino]-8-hydroxynaphthalin-3,6-disulfonsäure (s. Beispiel 5) in 300 Teilen Wasser. Unter Rühren stellt man einen pH-Wert von 6 bis 8 durch Zusatz von Natriumcarbonat ein und hält ihn während der Kupplung bei. Man lässt 6 bis 8 Stunden rühren, bis kein Diazoniumsalz mehr nachweisbar ist. Zu der so hergestellten braunvioletten Lösung der Monoazoverbindung gibt man 25 Teile kristallisiertes Kupfer(II)-sulfat und rührt bei einem pH-Wert von 5 bis 5,5 eine Stunde lang nach. Der hergestellte Kupferkomplexmonoazofarbstoff wird mittels Kaliumchlorid ausgefällt, abgesaugt, mehrmals mit konzentrierter wässriger Natriumchloridlösung gewaschen und sodann bei 100 °C getrocknet.

Es werden 90 Teile eines schwarz-violetten Farbstoffpulvers erhalten, das 56%ig an dem Natriumsalz der Verbindung der Formel

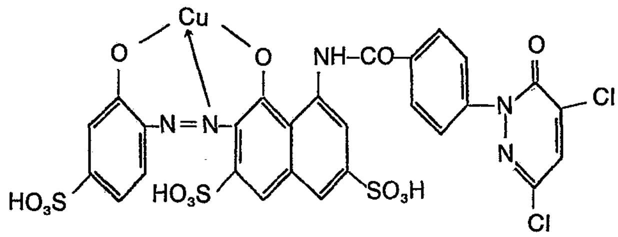

ist. Der Kupferkomplexmonoazofarbstoff liefert nach den für Reaktivfarbstoffe üblichen Färbe- und Druckverfahren auf natürlichen und synthetischen Polyamidfasermaterialien, insbesondere aber auf Cellulosefasermaterialien, Färbungen und Druck von violetter Nuance mit sehr guten Echtheitseigenschaften. Insbesondere sind die guten Wasch- und Lichtechtheiten hervorzuheben.

Beispiele 69 bis 76

Die nachfolgende Tabelle zeigt erfindungsgemässe Metallkomplexmonoazofarbstoffe, die in üblicher Weise zur Herstellung von Monoazofarbstoffen und deren Metallkomplexverbindungen hergestellt werden, so gemäss Methode A durch direkte Metallisierung, gemäss Methode B durch entalkylierende Kupferung und gemäss Methode C durch oxydative Kupferung der zuerst hergestellten Azoverbindungen. Die erfindungsgemässen Metallkomplexfarbstoffe zeigen ebenso wie der Farbstoff des Beispieles 68 gute Echtheitseigenschaften sowie sehr gute anwendungstechnische Eigenschaften.

| Bsp. | Diazokomponente | Kupplungskomponente | Metall | Methode | Farbton auf Baumwolle |
|---|---|---|---|---|---|
| 69 | 2-Methoxy-5-sulfo-anilin | 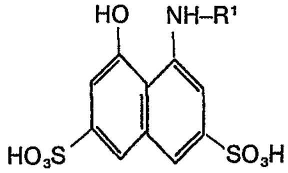 | Cu | B | violett |
| 70 | 2-Amino-naphthalin-4,8-disulfonsäure | dito | Cu | C | marineblau |
| 71 | 2-Hydroxy-6-nitro-4-sulfoaminonaphthalin | dito | Co | A | schwarz |
| 71a | dito | dito | Cr | A | schwarz |
| 72 | 6-Nitro-2-amino-naphthalin-4,8-disulfonsäure | dito | Cu | C | grünst. blau |
| 73 | dito | 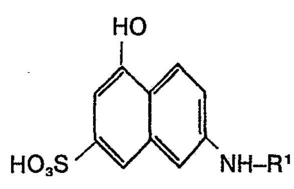 | Cu | C | marineblau |

(Fortsetzung)

| Bsp. | Diazokomponente | Kupplungskomponente | Metall | Methode | Farbton auf Baumwolle |
|---|---|---|---|---|---|
| 74 | 4-Nitro-2-aminophenol-6-sulfonsäure | 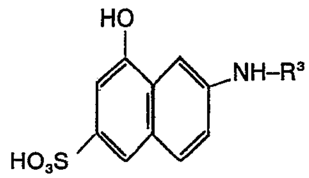 | Cu | A | dunkelbraun |
| 75 | 2-Aminonaphthalin-6,8-disulfonsäure | 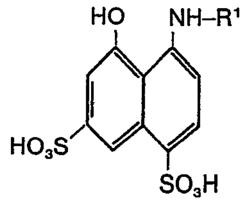 | Cu | C | marineblau |
| 76 | 2-Aminophenol-5-sulfonsäure | 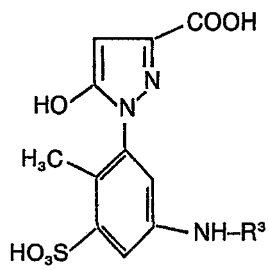 | Cu | A | braun |

Beispiel 77

18,8 Teile 1,3-Diaminobenzol-4-sulfonsäure werden unter Zusatz von Natriumcarbonat in 125 Teilen Wasser neutral gelöst; 40 Teile kristallisiertes Natriumacetat wird hinzugegeben. Unter gutem Rühren setzt man innerhalb von 2 Stunden bei einer Temperatur von 5 bis 10 °C 30,4 Teile 3,5-Dichlor-6-pyridazonyl(1)-benzol-4′-carbonsäurechlorid zu und hält während der Acylierungsreaktion den pH-Wert auf 4 bis 6 mittels Natriumcarbonat. Zur Vervollständigung der ausgefallenen Acylaminoverbindung gibt man noch 40 Teile Natriumchlorid hinzu; das abgeschiedene Produkt wird abgesaugt, mit einer wässrigen Natriumchloridlösung gewaschen und getrocknet.

Es wird das Natriumsalz der Verbindung der Formel

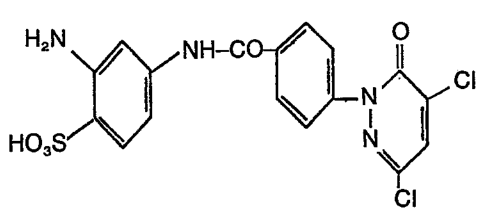

erhalten. Ihre Analysenwerte entsprechen den theoretisch erwarteten. Diese Aminoverbindung lässt sich als Diazokomponente zur Herstellung von faserreaktiven Azofarbstoffen einsetzen.

Beispiel 78

In ähnlicher Weise wie in Beispiel 77 beschrieben, lässt sich die 4-[4′-(3″,5″-Dichlor-6″-pyridazon-1″-yl)-benzoylamino]-aminobenzol-2-sulfonsäure durch Umsetzung entsprechender Mengen von 1,4-Diaminobenzol-2-sulfonsäure und 3,5-Dichlor-6-pyridazon-1-yl-benzol-4′-carbonsäurechlorid (Beispiel 1) herstellen und als Diazokomponente für die Synthese von faserreaktiven Azofarbstoffen verwenden.

Beispiel 78a

In analoger Weise, wie in Beispiel 77 beschrieben, lassen sich andere Aminobenzole und Aminonaphthaline herstellen, die diese faserreaktive Gruppe oder eine andere erfindungsgemässe faserreaktive Gruppe in Form deren Acylaminogruppe gebunden enthalten. Aromatische Amine, die in faserreaktive Diazokomponenten durch Umsetzung mit einem entsprechenden erfindungsgemässen faserreaktiven Säurechlorid übergeführt werden können, sind beispielsweise die 1,4-Diaminobenzol-2,6-disulfonsäure, die 3-Aminobenzylamin-4-sulfonsäure, die 1,6-Diamino-naphthalin-4-sulfonsäure oder die 1,6-Diamino-2-hydroxynaphthalin-4-sulfonsäure oder die 2,6-Diamino-1-hydroxynaphthalin-4,8-disulfonsäure, wobei die letztgenannten Diaminonaphthaline bevorzugt in Form von solchen Vorprodukten eingesetzt werden, in denen die zweite Aminogruppe zur Vermeidung der Diazotierung durch eine

Schutzgruppe, wie eine Acetylgruppe, geschützt ist oder eine Nitrogruppe darstellt, die erst später nach Durchführung der Diazotierung hydrolysiert bzw. reduziert werden.

Beispiel 79

44,1 Teile der Acylamino-aminobenzolsulfonsäure-Verbindung von Beispiel 77 werden in 200 Teilen Wasser gelöst; es lässt sich auch die gemäss Beispiel 77 erhaltene Suspension und Lösung des Acylierungsproduktes in die nachfolgende Diazotierungsreaktion einsetzen. Die Lösung dieser Acylamino-aminobenzolsulfonsäure wird mit einer Mineralsäure kongosauer gestellt und auf übliche Weise mittels einer Natriumnitritlösung diazotiert. Sodann gibt man 36,2 Teile 1-Acetylamino-8-hydroxynaphthalin-3,6-disulfonsäure als Kupplungskomponente hinzu, stellt mit Natriumcarbonat auf einen pH-Wert von 6 bis 7 ein, rührt mehrere Stunden, bis die Kupplung beendet ist (d.h. bis die Prüfung auf Diazoniumsalz negativ verläuft), erwärmt die erhaltene Suspension bei einem pH-Wert von 5 bis 6 auf 80 °C und saugt das Produkt über eine Nutsche ab. Das heisse Filtrat wird mit Kaliumchlorid versetzt, der fein ausgefallene Farbstoff abgesaugt, mit Natriumchloridlösung gewaschen, beide Rückstände vereinigt und bei 80 bis 100 °C unter reduziertem Druck getrocknet. Es werden 132 Teile eines salzhaltigen rotbraunen Farbstoffpulvers erhalten, das 47%ig an dem Alkalimetallsalz der Monoazoverbindungen der Formel

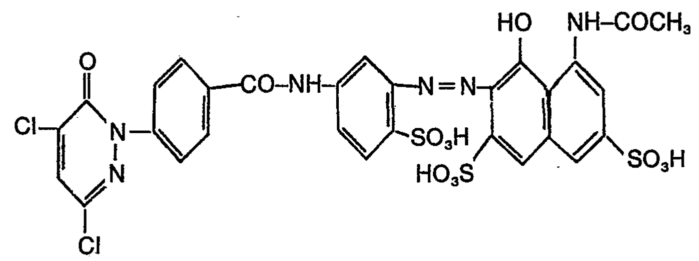

ist. Nach den für Reaktivfarbstoffe üblichen, insbesondere den in der Beschreibung angegebenen Verfahrensweisen zum Färben und Bedrucken von Fasermaterialien aus natürlichen und synthetischen Polyamiden, insbesondere von Cellulosefasermaterialien, werden rote Färbungen und Drucke erhalten, die sehr gute Echtheitseigenschaften, wie beispielsweise die in Beispiel 6 beschriebenen, besitzen. Der Farbstoff besitzt ebenso eine sehr gute Farbstärke.

Beispiele 80 bis 129

Verfährt man wie in Beispiel 79 beschrieben, setzt jedoch anstelle der dort erwähnten Diazokomponente und der dort erwähnten Kupplungskomponente die in den nachfolgenden Beispielen genannten Diazo- und Kupplungskomponenten ein, so erhält man erfindungsgemässe Monoazofarbstoffe mit sehr guten färberischen Eigenschaften, die Fasermaterialien, insbesondere Cellulosefasermaterialien, nach den für Reaktivfarbstoffen üblichen Applikations- und Fixierverfahren in den angegebenen Farbtönen echt färben:

| Bsp. | Diazokomponente | Kupplungskomponente | Farbton der Färbung |
|---|---|---|---|
| 80 | aus Bsp. 77 | 1-Benzoylamino-8-naphthol-3,6-disulfonsäure | blaust.-rot |
| 81 | dito | 1-(p-Methylphenylsulfonylamino)-8-naphthol-3,6-disulfonsäure | blaust.-rot |
| 82 | dito | 1-Acetylamino-8-naphthol-4,6-disulfonsäure | rot |
| 83 | dito | 2-Acetylamino-8-naphthol-3,6-disulfonsäure | scharlach |
| 84 | dito | 3-Acetylamino-8-naphthol-4,6-disulfonsäure | orange |
| 85 | dito | 2-(β-Carboxypropionylamino)-8-naphthol-6-sulfonsäure | orange |
| 86 | dito | 2-(β-Sulfopropionylamino)-8-naphthol-6-sulfonsäure | orange |
| 87 | dito | 2-Naphthol-3,6-disulfonsäure | rot |
| 88 | dito | 2-Naphthol-6,8-disulfonsäure | braun |
| 89 | dito | 4-Sulfo-1-naphthol | rotst. orange |
| 90 | dito | 3-Sulfo-1-naphthol | gelbst. orange |
| 91 | dito | 3,8-Disulfo-1-naphthol | gelbst. orange |
| 92 | dito | 3,6-Disulfo-1-naphthol | rotst. orange |

(Fortsetzung)

| Bsp. | Diazokomponente | Kupplungskomponente | Farbton der Färbung |
|---|---|---|---|
| 93 | aus Bsp. 77 | 3,6,8-Trisulfo-1-naphthol | rotst. orange |
| 94 | dito | 4,8-Disulfo-1-naphthol | rot |
| 95 | dito | N,N-Di-(β-sulfatoäthyl)-anilin | orange |
| 96 | dito | 1-(4'-Sulfophenyl)-3-methyl-5-pyrazolon | gelb |
| 97 | dito | 1-(2',5'-Disulfophenyl)-3-methyl-5-pyrazolon | gelb |
| 98 | dito | 1-(4'-Sulfophenyl)-3-carboxy-5-pyrazolon | gelb |
| 99 | dito | 1-Sulfophenyl-3-methyl-5-aminopyrazol | gelb |
| 100 | dito | N-Methyl-4-methyl-2-hydroxy-pyridon(6)-3-sulfonsäure | gelb |
| 101 | dito | 4-Methyl-2,6-dihydroxy-pyridin-3-sulfonsäure | gelb |
| 102 | dito | 2-Aminonaphthalin-3,6-disulfonsäure | orange |
| 103 | 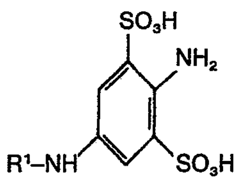 | 1-Acetylamino-8-naphthol-3,6-disulfonsäure | blaust. rot |
| 104 | dito | 1-Benzoylamino-8-naphthol-4,6-disulfonsäure | blaust. rot |
| 105 | dito | 2-Acetylamino-8-naphthol-6-sulfonsäure | rotst. orange |
| 106 | dito | 3-Acetylamino-8-naphthol-6-sulfonsäure | orange |
| 106a | dito | 1-Acetylamino-8-naphthol-4-sulfonsäure | blaust. rot |
| 107 | dito | 2-Amino-8-hydroxynaphthalin-6-sulfonsäure (sauer gekuppelt) | rot |
| 108 | dito | 4-Methyl-3-cyano-1-2,6-dihydroxy-pyridin | gelb |
| 109 | dito | N-Äthyl-N-(3'-sulfobenzyl)-anilin | scharlach |
| 109a | dito | N-Äthyl-N-(β-sulfatoäthyl)-anilin | orange |
| 109b | dito | 3-Methyl-N,N-di-(β-sulfatoäthyl)-anilin | orange |
| 110 | aus Bsp. 78 | 1-Acetylamino-8-naphthol-3,6-disulfonsäure | blaust. rot |
| 110a | dito | 1-Benzoylamino-8-naphthol-3,6-disulfonsäure | rotst. violett |
| 110b | dito | 1-Phenylsulfonylamino-8-naphthol-3,6-disulfonsäure | rotst. violett |
| 111 | dito | 1-Acetylamino-8-naphthol-4,6-disulfonsäure | blaust. rot |
| 111a | dito | 1-Benzoylamino-8-naphthol-4,6-disulfonsäure | blaust. rot |
| 112 | dito | 2-N-Methyl-acetylamino-8-naphthol-6-sulfonsäure | rot |
| 113 | dito | 3-Acetylamino-8-naphthol-4,6-disulfonsäure | scharlach |
| 113a | dito | 2-(β-Sulfopropionylamino)-8-naphthol-6-sulfonsäure | rot |
| 114 | dito | 1-(2',5'-Disulfophenyl)-3-methyl-5-pyrazolon | rotst. gelb |
| 114a | dito | 1-(4'-Sulfophenyl)-3-carboxy-5-pyrazolon | rotst. gelb |
| 115 | dito | 2-Naphthol-3,6-disulfonsäure | rot |
| 116 | dito | 2-Aminonaphthalin-3,6-disulfonsäure | scharlach |
| 117 | dito | 3,8-Disulfonaphthol | orange |
| 117a | dito | 3,6-Disulfonaphthol | rot |
| 117b | dito | 3,6,8-Trisulfonaphthol | rot |
| 118 | dito | N-Methyl-4-methyl-2-hydroxy-pyridon(6)-3-sulfonsäure | gelb |
| 119 | 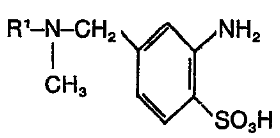 | 1-Acetylamino-8-naphthol-4,6-disulfonsäure | rot |

Beispiel 120

Einer eisgekühlten Mischung von 26,5 Teilen 6-Nitro-1-diazo-2-naphthol-4-sulfonsäure und 31,9 Teilen 1-Amino-8-naphthol-2,4-disulfonsäure in 300 Teilen Wasser werden 20 Teile Calciumhydroxyd zugegeben. Diese Mischung wird 10

Stunden lang zunächst bei einer Temperatur zwischen 5 und 10 °C, später unter Erwärmenlassen auf Raumtemperatur und Erwärmen bis zu 30 °C gerührt. Danach werden 44 Teile kristallisiertes Natriumsulfid hinzugefügt und die Reaktionsmischung eine Stunde bei 60–70 °C gerührt, danach auf 20 °C abgekühlt und mit 50%iger Schwefelsäure auf einen pH-Wert von 1,5 bis 2 gestellt. Es wird filtriert. Das Filtrat wird mit 40 Teilen kristallisiertem Natriumacetat versetzt, der pH-Wert mit konzentrierter Natronlauge auf einen Wert von 6 gestellt und die Lösung sodann auf eine Temperatur von 5 bis 10 °C gekühlt. Unter Beibehaltung dieser Temperatur werden unter gutem Rühren innerhalb einer Stunde 30,4 Teile pulverisiertes 3,5-Dichlor-6-pyridazon-1-yl-benzol-4'-carbonsäurechlorid zugegeben, das Ganze 3 Stunden lang erst bei 5 °C, dann weitere 6 Stunden bei 20 °C gerührt. Im Verlaufe dieser Umsetzung wird der pH-Wert bei 3,5 bis 6 gehalten.

Danach gibt man 22,5 Teile kristallisiertes Kupfer(II)-sulfat hinzu und stellt mit Natriumcarbonat einen pH-Wert von 5 bis 6 ein. Es wird eine Stunde bei 20 °C gerührt, sodann die blaue Lösung geklärt, das Filtrat eingedampft oder sprühgetrocknet. Es werden 98 Teile eines schwarzen Farbstoffpulvers erhalten, das 65%ig an dem Alkalimetallsalz der Verbindung der Formel

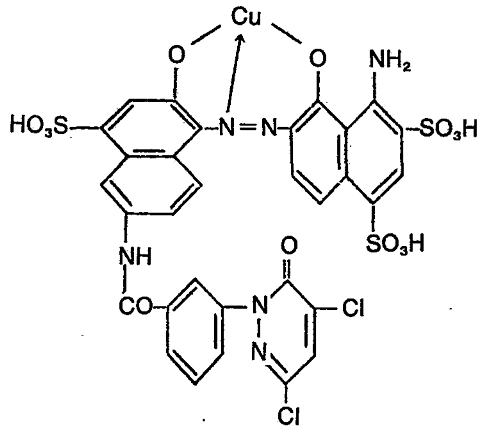

ist (der Farbstoff kann auch aus der wässrigen Syntheselösung durch Ausfällen mit Natrium- oder Kaliumchlorid erhalten werden). Mit diesem Farbstoff werden auf synthetischen oder natürlichen Polyamidfasermaterialien, insbesondere aber auf Cellulosefasermaterialien, nach den für Reaktivfarbstoffe üblichen Applikations- und Fixierverfahren farbstarke Färbungen und Drucke von grünstichigblauer Nuance erhalten, die sehr gute Echtheiten, wie die in Beispiel 6 beschriebenen, aufweisen.

Beispiele 121 bis 124

Verfährt man in ähnlicher Weise wie im Beispiel 120 beschrieben, setzt jedoch anstelle der dort genannten Diazokomponente 6-Nitro-1-diazoniumchlorid-2-naphthol-4-sulfonsäure die äquivalente Menge einer anderen Diazokomponente ein, so beispielsweise die der 6-Nitro-2-aminonaphthol-4,8-disulfonsäure oder der 6-Nitro-2-aminophenol-4-sulfonsäure, und verwendet entsprechende andere übliche Kupplungskomponenten, wie beispielsweise die 1-Acetyl-amino-8-naphthol-3,6-disulfonsäure, die 2-Acetylamino-8-naphthol-6-sulfonsäure, die 2-Hydroxynaphthalin-4,7-disulfonsäure oder das 1-(2',5'-Disulfophenyl)-3-methyl-5-pyrazolon, so erhält man beispielsweise die nachfolgend beschriebenen erfindungsgemässen faserreaktiven Farbstoffe, die Polyamidfasermaterialien, insbesondere aber Cellulosefasermaterialien, in sehr guten Allgemeinechtheiten und mit guter Farbstärke färben.

| Bsp. | Farbstoff | Farbton der Färbung |
|---|---|---|
| 121 | 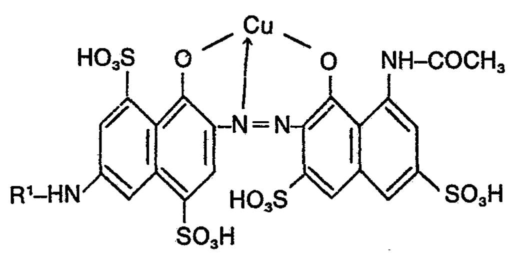 | marineblau |
| 122 | 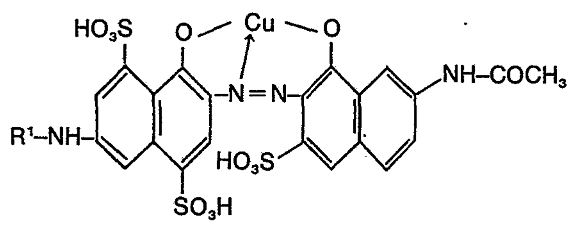 | marineblau |

(Fortsetzung)

| Bsp. | Farbstoff | Farbton der Färbung |
|---|---|---|
| 123 | 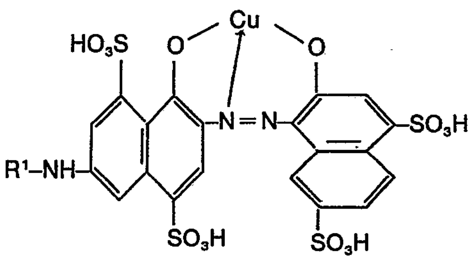 | marineblau |
| 124 | 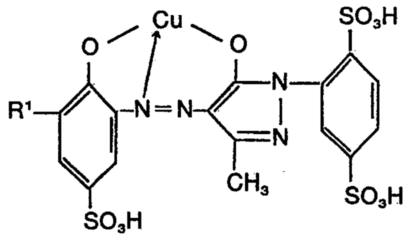 | gelbstichig braun |

Beispiel 125

23,9 Teile Anilin-2,4-disulfonsäure werden in 100 Teilen Wasser suspendiert; 30 Volumenteile einer 30%igen wässrigen Salzsäure und 100 Teile Eis werden hinzugegeben. Die Diazotierung erfolgt mittels 20 Volumenteilen einer wässrigen 5n-Natriumnitritlösung. Nach Diazotierung wird überschüssige salpetrige Säure mit etwas Amidosulfonsäure zerstört, sodann 15,1 Teile 3-Aminophenylharnstoff hinzugegeben und mehrere Stunden lang gerührt. Nach beendeter Kupplung stellt man den pH des Reaktionsgemisches mit Natronlauge auf den Wert von 6 ein, gibt 30 Teile kristallisiertes Natriumacetat hinzu und zusätzlich soviel Wasser, dass die gebildete Monoazoverbindung in Lösung geht. Man kühlt auf eine Temperatur von 5 bis 10 °C ab und gibt im Laufe einer Stunde 33 Teile 3,5-Dichlor-6-pyridazon-1-yl-benzol-4'-carbonsäurechlorid unter Rühren hinzu und rührt noch mehrere Stunden unter Beibehaltung eines pH-Wertes von 6 bis 3,5 nach. Das Reaktionsgemisch wird geklärt, der gebildete faserreaktive Farbstoff mittels Natriumchlorid aus dem Filtrat gefällt und isoliert. Es werden 106 Teile eines salzhaltigen Farbstoffpulvers erhalten, das etwa 45%ig an dem Alkalimetallsalz der Verbindung der Formel

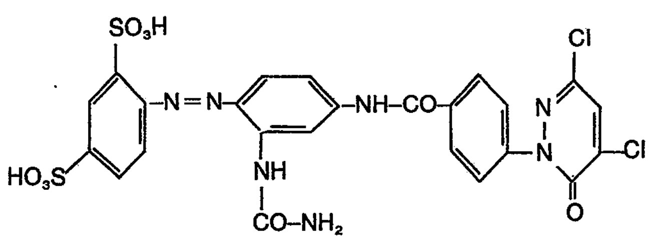

ist. Der Farbstoff liefert nach den für Reaktivfarbstoffe üblichen Applikations- und Fixierweisen auf natürlichen und synthetischen Polyamidfasern, insbesondere jedoch auf Cellulosefasern, goldgelbe Färbungen und Drucke mit sehr guten Echtheiten, wie beispielsweise den in Beispiel 6 beschriebenen. Die mit ihm erhaltenen Färbungen sind sehr farbstark.

Beispiele 126 bis 134

Verfährt man in gleicher oder ähnlicher Weise, wie im Beispiel 125 beschrieben, unter Verwendung anderer Diazo- und Kupplungskomponenten und setzt die gebildete Monoazoverbindung mit einem erfindungsgemässen faserreaktiven Säurechlorid in analoger Weise um, so erhält man ebenfalls erfindungsgemäss faserreaktive Monoazofarbstoffe, die insbesondere Cellulosefasermaterialien mit hoher Farbtiefe und sehr guten Echtheitseigenschaften färben. In den nachfolgenden Beispielen sind diese Monoazoverbindungen durch ihre Diazo- und Kupplungskomponenten und dem verwendeten faserreaktiven Säurechlorid der allgemeinen Formel (III) charakterisiert.

| Bsp. | Diazokomponente | Kupplungskomponente | faserreaktiver Säurerest mit obiger Definition | Farbton der Färbung |
|---|---|---|---|---|
| 126 | 2,5-Disulfo-anilin | 3-Acetylamino-6-methoxy-anilin | $-R^1$ | rotstich. gelb |
| 127 | 2-Aminonaphthalin-4,8-disulfonsäure | 3-Methylanilin | $-R^1$ | rotstich. gelb |
| 128 | 2-Aminonaphthalin-3,6,8-trisulfonsäure | 3-Methylanilin | $-R^1$ | rotstich. gelb |
| 129 | 2-Aminonaphthalin-1,5-disulfonsäure | 6- und 7-Sulfonaphthyl-amin | $-R^1$ | orange |
| 130 | 2-Aminonaphthalin-6,8-disulfonsäure | 3-Acetylamino-anilin | $-R^1$ | goldgelb |
| 131 | 2,4-Disulfo-anilin | 3-Acetylamino-6-methoxy-anilin | $-R^3$ | orange |
| 132 | 2-Aminonaphthalin-3,6,8-trisulfonsäure | 3-Methylanilin | $-R^3$ | orange |
| 133 | dito | dito | $-R^2$ | rotstich. gelb |
| 134 | 4-Acetylamino-2,6-disulfo-anilin | 3-Acetylamino-anilin | $-R^1$ | orange |

Beispiel 135

In üblicher Weise wird ein Monoazofarbstoff aus 25,3 Teilen Anilin-2,5-disulfonsäure als Diazokomponente und 30,3 Teilen 1-Amino-8-naphthol-3,6-disulfonsäure als Kupplungskomponente in wässrigem Medium hergestellt. 46,0 Teile 3-[4'-(3",5"-Dichlor-6"-pyridazon-1"-yl)-benzoylamino]-anilin-6-sulfonsäure werden auf übliche Weise diazotiert (siehe Beispiel 77); diese Diazoniumsalzlösung wird zur zuvor hergestellten Lösung der Monoazoverbindung zugegeben und zur Kupplung ein pH-Wert von 6 bis 7,5 mit Natriumcarbonat eingestellt. Bei diesem pH wird 15 Stunden lang gerührt, anschliessend wird der pH-Wert auf 5 bis 5,5 erhäht, das Reaktionsgemisch auf 80 °C zwecks Lösung des teilweise ausgefallenen Disazofarbstoffes erwärmt, diese Lösung heiss geklärt und das Filtrat mit Natriumchlorid oder Kaliumchlorid versetzt und auf 10 °C abgekühlt. Der ausgefallene Farbstoff wird abgenutscht und getrocknet.

(Der Farbstoff kann auch aus dem Filtrat durch Eindampfen oder Sprühtrocknen des Filtrats erhalten werden.)

Es werden 120 Teile eines schwarzen Farbstoffpulvers mit einem Gehalt von 70% an dem Alkalimetallsalz der Verbindung der Formel

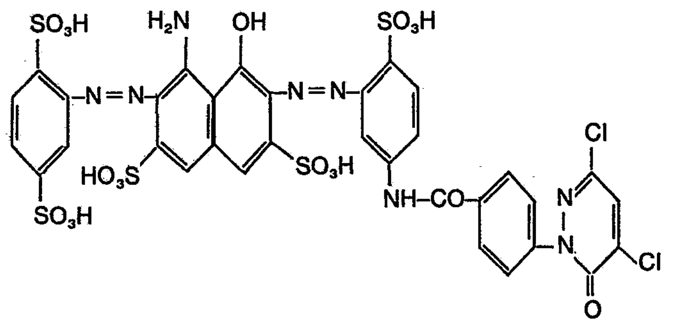

besitzt. Dieser Farbstoff färbt Polyamid- und Cellulosefasermaterialien nach für Reaktivfarbstoffe üblichen Applikations- und Fixierweisen in marineblauen bis schwarzen Farbtönen, die gute Echtheitseigenschaften aufweisen.

Beispiele 136 bis 172

Verfährt man analog zu der Verfahrensweise des Beispieles 135 zur Herstellung eines faserreaktiven Dis- oder Trisazofarbstoffes, verwendet jedoch zur Herstellung der Ausgangs-Monoazo- oder -disazoverbindung andere aromatische Amine als Diazokomponente Nr. 1 und die erwähnte oder eine andere bifunktionelle Kupplungskomponente und setzt diese Monoazoverbindung mit einem anderen faserreaktiven aromatischen Amin als Diazokomponente Nr. 2 um, so beispielsweise mit den in den nachfolgenden Beispielen beschriebenen Verbindungen, so erhält man ebenfalls erfindungsgemässe faserreaktive Disazofarbstoffe, die Fasermaterialien aus natürlichen oder synthetischen Polyamiden, insbesondere Cellulosefasermaterialien, mit sehr guten anwendungstechnischen Eigenschaften echt färben. Gleich gute Farbstoffe werden erhalten, wenn man anstelle der Diazokomponente 1 eine erfindungsgemässe Diazokomponente mit einem erfindungsgemässen faserreaktiven Pyridazonylrest einsetzt, so dass der erhaltene Azofarbstoff zwei faserreaktive erfindungsgemässe Anker besitzt. Die metallfreien Farbstoffe können auch durch übliche Metallisierungsverfahren in die entsprechenden Metallkomplexfarbstoffe übergeführt werden.

| Bsp. | Diazokomponente No. 1 | Kupplungskomponente | Diazokomponente No. 2 | Farbton der Färbung |
|---|---|---|---|---|
| 136 | 4-Amino-benzol-sulfonsäure | 1-Amino-8-naphthol-3,6-disulfonsäure | 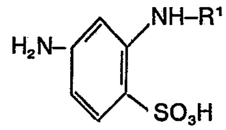 | schwarz |
| 137 | 2-Aminonaphthalin-1,5-disulfonsäure | dito | dito | grünst. schwarz |
| 138 | 2-Amino-4,8-disulfonaphthalin | dito | dito | grünst. schwarz |
| 139 | 2-Aminonaphthalin-6,8-disulfonsäure | dito | dito | grünst. schwarz |
| 140 | 2,4-Disulfo-anilin | dito | dito | schwarz |
| 141 | 4-Nitro-2-sulfo-anilin | dito | dito | grünst. schwarz |
| 142 | 2-Amino-benzol-sulfonsäure | dito | dito | schwarz |
| 143 | 2,4'-Disulfo-1-aminoazobenzol | dito | dito | grünst. schwarz |
| 144 | 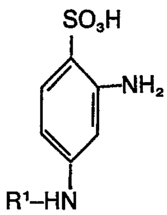 | dito | dito | schwarz |
| 145 | dito | dito | 2-Amino-benzol-sulfonsäure | schwarz |
| 146 | dito | 1-Amino-8-naphthol-4,6-disulfonsäure | 2-Aminonaphthalin-4,8-disulfonsäure | grünst. schwarz |
| 147 | 2,5-Disulfo-anilin | dito | 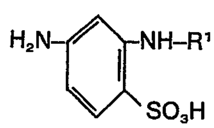 | marineblau bis schwarz |
| 148 | 2-Aminonaphthalin-1,5-disulfonsäure | dito | dito | schwarz |
| 149 | 2-Aminonaphthalin-4,8-disulfonsäure | 1-Amino-8-naphthol-4,6-disulfonsäure | dito | schwarz |
| 150 | 4-Sulfo-anilin | 1-Amino-8-naphthol-3,6-disulfonsäure | 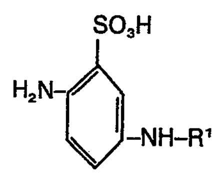 | grünst. schwarz |
| 151 | 2,5-Disulfo-anilin | dito | dito | grünst. schwarz |
| 152 | 2,4-Disulfo-anilin | dito | dito | grünst. schwarz |
| 153 | 2,4'-Disulfo-1-aminoazobenzol | dito | dito | dunkelgrün |
| 154 | 4'-Nitro-2,2'-disulfo-1-aminostilben | dito | dito | dunkelgrün |

(Fortsetzung)

| Bsp. | Diazokomponente No. 1 | Kupplungskomponente | Diazokomponente No. 2 | Farbton der Färbung |
|---|---|---|---|---|
| 155 | 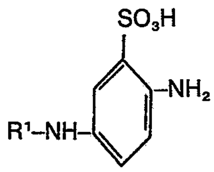 | 1-Amino-8-naphthol-3,6-disulfonsäure | 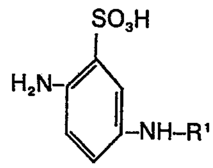 | grünst. schwarz |
| 156 | 2-Aminonaphthalin-1,5-disulfonsäure | dito | dito | grünst. schwarz |
| 157 | 2-Aminonaphthalin-3,6,8-trisulfonsäure | dito | dito | grünst. schwarz |
| 158 | 4-Amino-benzolsulfonsäure | dito | 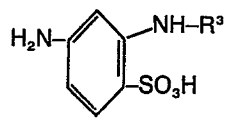 | dunkelblau bis schwarz |
| 159 | dito | dito | 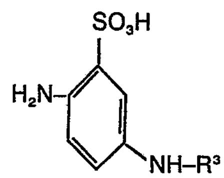 | schwarz |
| 160 | 4-Sulfo-naphthylamin | dito | dito | schwarz |
| 161 | 4'-Sulfo-4-amino-azobenzol | dito | dito | grünst. schwarz |
| 162 | 2,5-Disulfo-anilin | 1-Amino-8-naphthol-4,6-disulfonsäure | 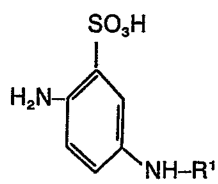 | schwarz |
| 163 | 2,4-Disulfo-anilin | dito | dito | schwarz |
| 164 | 2-Aminonaphthalin-1,5-disulfonsäure | dito | dito | grünst. schwarz |
| 165 | 2-Aminonaphthalin-3,6,8-trisulfonsäure | dito | dito | grünst. schwarz |
| 166 | 2-Aminonaphthalin-6,8-disulfonsäure | dito | dito | grünst. schwarz |

Kupferkomplexdisazofarbstoffe (durch direkte Kupferung):

| | | | | |
|---|---|---|---|---|
| 167 | 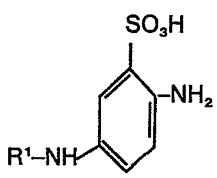 | 1-Amino-8-naphthol-3,6-disulfonsäure | 6-Nitro-2-aminophenol-4-sulfonsäure | grünst. blau |
| 168 | dito | dito | 2-Aminophenol-5-sulfonsäure | grünst. blau |
| 169 | 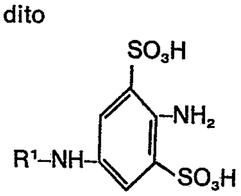 | dito | dito | grünst. blau |
| 170 | 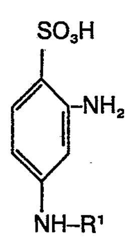 | dito | 6-Nitro-2-aminophenol-4-sulfonsäure | grünst. blau |

(Fortsetzung)

| Bsp. | Diazokomponente No. 1 | Kupplungskomponente | Diazokomponente No. 2 | Farbton der Färbung |
|---|---|---|---|---|
| Kupferkomplexdisazofarbstoffe (durch entalkylierende Kupferung): | | | | |
| 171 | 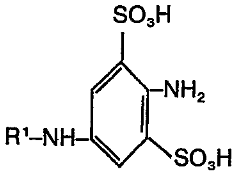 | 1-Amino-8-naphthol-3,6-disulfonsäure | 4-Nitro-2-methoxy-anilin-5-sulfonsäure | grünst. blau |
| 172 | 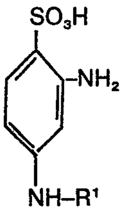 | dito | dito | grünst. blau |

Beispiel 173

17,2 Teile Benzidin-2,2'-disulfonsäure werden in üblicher Verfahrensweise in wässriger Phase tetrazotiert. Man gibt in diese Tetrazoniumsalzlösung eine wässrige Lösung von 59,0 Teilen 1-[4'-(3'',5''-Dichlor-6''-pyridazon-1''-yl)-benzoylamino]-8-naphthol-3,6-disulfonsäure als Natriumsalz (siehe Beispiel 5) und führt die Kupplung unter mehrstündigem Rühren bei einem pH-Wert von 6–7 durch. Anschliessend wird die erhaltene Disazoverbindung mit Natriumchlorid ausgesalzen, abgesaugt und getrocknet. Man erhält 87 Teile eines etwa 60%igen salzhaltigen Farbstoffpulvers mit dem Natriumsalz der Disazoverbindung der Formel

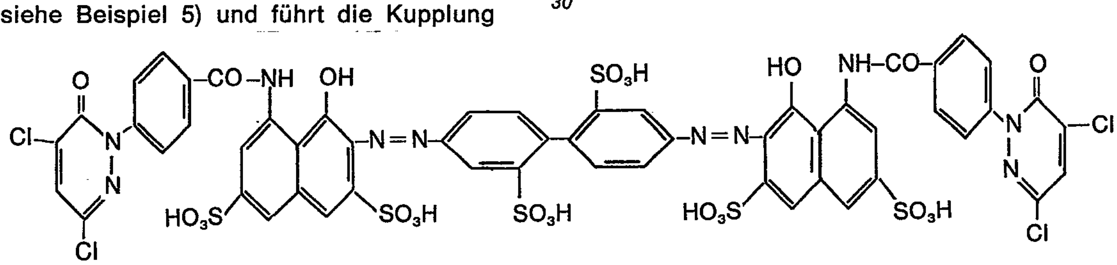

Mit diesem Farbstoff lassen sich nach für Reaktivfarbstoffe üblichen Färbe- und Druckverfahren auf Fasermaterialien aus natürlichen und synthetischen Polyamiden, insbesondere auf Cellulosefasermaterialien, farbstarke violette Färbungen und Drucke mit guten Echtheiten, wie beispielsweise den unter Beispiel 6 beschriebenen, herstellen.

Beispiele 174 bis 181

Man verfährt in üblicher und bekannter Verfahrensweise zur Herstellung von Disazofarbstoffen unter Verwendung von Tetrazokomponenten und Kupplungskomponenten, so beispielsweise wie in Beispiel 173 beschrieben, und setzt äquivalente Mengen der in den nachfolgenden Beispielen tabellarisch genannten Ausgangsverbindungen ein; man erhält auf diese Weise entsprechende erfindungsgemässe Disazofarbstoffe mit einer erfindungsgemässen faserreaktiven Gruppe, die ebenso gute anwendungstechnische Eigenschaften besitzen und beispielsweise Cellulosefasermaterialien mit guten Echtheiten färben:

| Bsp. | Tetrazokomponente | 1. Kupplungskomponente | 2. Kupplungskomponente | Farbton der Färbung |
|---|---|---|---|---|
| 174 | 1,4-Phenylendiamin | 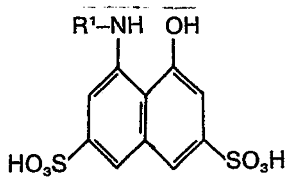 | 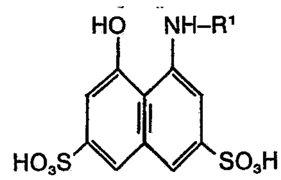 | violett |
| 175 | 3,3'-Diamino-diphenyl-sulfon | dito | dito | violett |

(Fortsetzung)

| Bsp. | Tetrazokomponente | 1. Kupplungskomponente | 2. Kupplungskomponente | Farbton der Färbung |
|---|---|---|---|---|
| 176 | 1,3-Phenylendiamin-4-sulfonsäure | 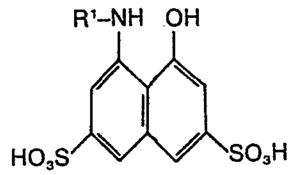 | 1-Acetylamino-8-naphthol-3,6-disulfonsäure | violett |
| 177 | 1,4-Phenylendiamin-3-sulfonsäure | dito | dito | violett |
| 178 | 4,4′-Diamino-diphenylamin-2-sulfonsäure | 1,3-Phenylen-diamin | 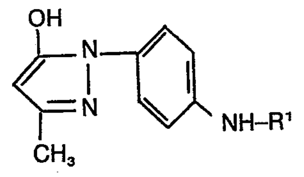 | dunkelbraun |
| 179 | 4,4′-Diamino-diphenyl-3,3′-disulfonsäure | 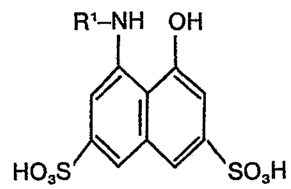 | 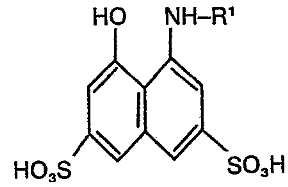 | blau |
| 180 | 3,3′-Dimethoxy-4,4′-diamino-diphenyl-2,2′-disulfonsäure | dito | dito | blau |
| 181 | 3,3′-Dimethyl-4,4′-diamino-diphenyl-2,2′-disulfonsäure | dito | dito | blau |

Beispiel 182

46,0 Teile 3-[4′-(3″,5″-Dichlor-6″-pyridazon-1″-yl)-benzolsulfonylamino]-anilin-6-sulfonsäure, die man analog den Angaben des Beispieles 77 durch Umsetzung von 18,8 Teilen 1,3-Diamino-benzol-4-sulfonsäure und 30,4 Teilen 3,5-Dichlor-6-pyridazonyl(1)-benzol-4′-sulfonsäurechlorid herstellen kann, werden in üblicher Weise diazotiert, beispielsweise gemäss den Angaben des Beispieles 79, und in üblicher Weise mit 22,3 Teilen eines Gemisches aus 1-Naphthylamin-6-sulfonsäure und 1-Naphthylamin-7-sulfonsäure bei einem pH-Wert von 0,5 bis 2 gekuppelt. Die so hergestellte Aminoazoverbindung wird danach in üblicher Weise in der Kälte in stark saurem Medium mittels 14 Volumenteilen einer wässrigen 40%igen Natriumnitritlösung diazotiert; nach der Nitritzugabe rührt man noch etwa 4 Stunden lang nach, zerstört überschüssige salpetrige Säure mit Amidosulfonsäure, gibt anschliessend als Kupplungskomponente eine neutrale Lösung von 30,1 Teilen N-Phenyl-1-naphthylamin-8-sulfonsäure in 200 Teilen Wasser und 6 Volumenteilen konzentrierter Natronlauge hinzu und verrührt das Kupplungsgemisch mehrere Stunden bei Einhaltung eines pH-Wertes von 2 bis 4. Nach beendeter Kupplung wird die Lösung mit Natriumcarbonat auf einen pH-Wert von 5 bis 6 eingestellt, der gebildete Disazofarbstoff mit Natriumchlorid abgeschieden, abgesaugt, mit einer wässrigen Natriumchloridlösung gewaschen und getrocknet. Es werden 107 Teile eines elektrolythaltigen blauschwarzen Farbstoffpulvers erhalten, das das Natriumsalz des Farbstoffes der Formel

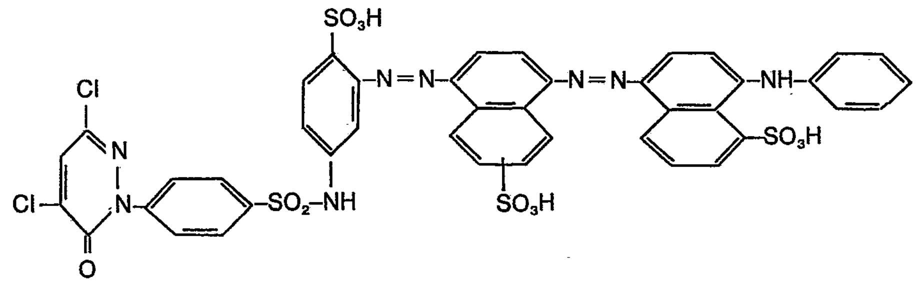

mit 64% enthält. Mit diesem Farbstoff lassen sich auf Polyamid- und Cellulosefasern nach den für faserreaktive Farbstoffe üblichen Applikations- und Fixierverfahren farbstarke marineblaue Färbungen und Drucke mit sehr guten Echtheitseigenschaften erzeugen.

Beispiele 183 bis 188

Verfährt man in üblicher Weise zur Herstellung von Disazofarbstoffen des Typs A→B→C, beispielsweise im Sinne der Angaben des Beispieles 182, so erhält man ebenfalls wertvolle erfindungsgemässe Disazofarbstoffe mit einer erfindungsgemässen faserreaktiven Gruppe, die insbesondere Cellulosefasermaterialien in den angegebenen Farbtönen echt färben:

| Bsp. | Diazokomponente A | Mittelkomponente B (Diazo- + Kupplungskomp.) | Kupplungskomponente C | Farbton der Färbung |
|---|---|---|---|---|
| 183 | 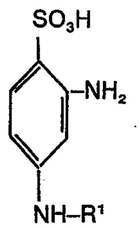 | 1-Aminonaphthalin-7-sulfonsäure | N-Äthyl-N-(3'-sulfobenzyl)-anilin | violett |
| 184 | dito | dito | N,N-Di-(β-sulfatoäthyl)-anilin | violett |
| 185 | dito | dito | N-Äthyl-N-(β-sulfatoäthyl)-3-chlor-anilin | violett |
| 186 | 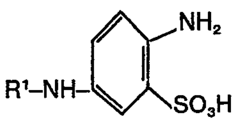 | 1-Naphthylamin-6- + 7-sulfonsäure | 1-Phenylaminonaphthalin-8-sulfonsäure | marineblau |
| 187 | dito | 1-Naphthylamin-6- + -7-sulfonsäure | N-Äthyl-N-(3'-sulfobenzyl)-anilin | violett |
| 188 | 2,5-Disulfo-anilin | dito | 2-Methoxy-5-methyl-anilin | braun |
| | wobei diese Disazoverbindung mit äquivalenten Mengen 3,5-Dichlor-6-pyradazon-1-yl-benzol-4'-carbonsäurechlorid umgesetzt wird | | | |

Beispiele 189 bis 193 (Kupferkomplexfarbstoffe)

In den nachfolgenden Beispielen werden erfindungsgemässe Kupferkomplexdisazofarbstoffe beschrieben, die in metallfreier Form dem Typ der Beispiele 182 bis 188 entsprechen. Diese metallfreien Disazofarbstoffe werden in entsprechender Weise hergestellt und anschliessend nach üblicher Methode mit Kupfersalzen unter Entalkylierung in die entsprechenden Kupferkomplexdisazofarbstoffe übergeführt:

| Bsp. | Diazokomponente A | Mittelkomponente B (Diazo- + Kupplungskomp.) | Kupplungskomponente C | Farbton der Färbung |
|---|---|---|---|---|
| 189 | 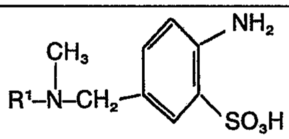 | 2-Methoxy-5-methyl-anilin | 3,6-Disulfo-1-naphthol | rotstichig blau |
| 190 | 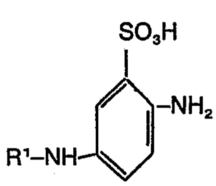 | 2-Methoxy-1-naphthylamin-6-sulfonsäure | 4,8-Disulfo-1-naphthol | dunkelgrün |
| 191 | 2,5-Disulfo-anilin | 2-Methoxy-5-methyl-anilin | 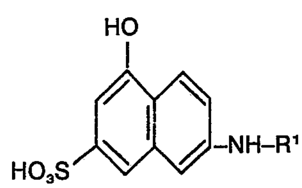 | marineblau |
| 192 | 2-Amino-benzolsulfonsäure | dito | 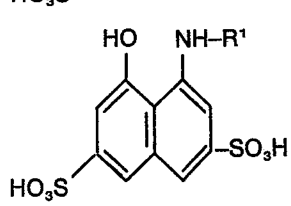 | marineblau |

(Fortsetzung)

| Bsp. | Diazokomponente A | Mittelkomponente B (Diazo- + Kupplungskomp.) | Kupplungskomponente C | Farbton der Färbung |
|---|---|---|---|---|
| 193 | 2,5-Dimethoxy-anilin-4-sulfonsäure | 2-Amino-8-naphthol-6-sulfonsäure | 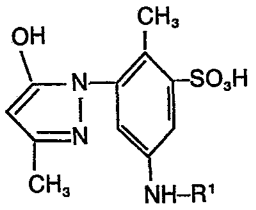 | schwarz |

Beispiel 194

38,1 Teile 1-Amino-4-brom-anthrachinon-2-sulfonsäure, 62,6 Teile Natriumhydrogencarbonat, 4,7 Teile Natriumcarbonat und 19,6 Teile 4-Amino-N-methyl-acetanilid werden in 200 Teilen Wasser unter Rühren suspendiert. Nach einer halben Stunde setzt man der Suspension 0,95 Teile Kupfer(I)-chlorid sowie etwa 200 bis 220 Teile Wasser zu. Man erhitzt das Reaktionsgemisch unter Luftausschluss und bei gutem Rühren auf eine Temperatur von 70 bis 75 °C und hält diese Temperatur unter Rühren 3 Stunden lang. Die gebildete blaue Anthrachinonverbindung wird aufgearbeitet, indem man das Reaktionsgemisch innerhalb etwa 60 bis 90 Minuten mit etwa 80 Volumenteilen konzentrierter wässriger Salzsäure auf einen pH-Wert von 1 stellt und es danach noch eine Stunde bei 70 °C rührt. Die ausgefallene Anthrachinonverbindung wird abfiltriert, mit einem Gemisch aus 1600 Teilen Wasser und 160 Volumenteilen konzentrierter wässriger Salzsäure in zwei Portionen gewaschen. Der feuchte Filterkuchen wird in 400 Teilen Wasser suspendiert, die wässrige Suspension mit einer wässrigen Ammoniaklösung auf einen pH von 7 bis 8 gestellt und danach sprühgetrocknet. Es werden 42 Teile einer etwa 78%igen, elektrolythaltigen Verbindung der Formel

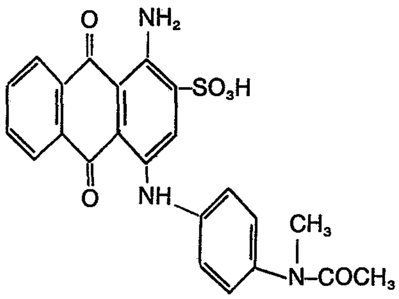

erhalten. Die erhaltene Verbindung wird anschliessend im Verlaufe von etwa 90 Minuten bei einer Temperatur von maximal 30 °C in ein Gemisch aus 20 Teilen Schwefelsäure-Monohydrat und 128 Teilen 20%igem Oleum eingetragen. Man verrührt das Gemisch 3 Stunden bei 25 bis 30 °C und gibt es dann langsam auf ein Gemisch aus 600 Teilen Eis und 100 Teilen Wasser; anschliessend wird 30 Minuten lang nachgerührt, die Suspension abgesaugt und der erhaltene feuchte Nutschkuchen in 225 Teilen Wasser und 40 Teilen 31%iger wässriger Salzsäure suspendiert, zum Sieden gebracht und 4 Stunden lang unter Rückfluss gerührt. Anschliessend lässt man auf Raumtemperatur abkühlen, neutralisiert mit etwa 70 Teilen 33%iger Natronlauge auf einen pH-Wert von 6,5 bis 7, bringt mit etwa 60 Teilen Wasser die teilweise ausgefallene Verbindung wiederum in Lösung, kühlt diese Lösung sodann auf eine Temperatur von 5 bis 10 °C ab, versetzt sie mit 30 Teilen kristallisiertem Natriumacetat und gibt ihr im Verlaufe von einer Stunde 21,3 Teile 3,5-Dichlor-6-pyridazonyl(1)-benzol-4'-carbonsäurechlorid hinzu und rührt das Ganze 15 Stunden lang.

Anschliessend wird der pH-Wert der Reaktionsmischung auf 5,5 gestellt und zur Vervollständigung der Ausfällung des gebildeten faserreaktiven Farbstoffes Natriumchlorid zugesetzt. Er wird abgesaugt und getrocknet. Man erhält 43 Teile eines elektrolythaltigen 65%igen schwarzen Farbstoffpulvers mit dem Farbstoff der Formel

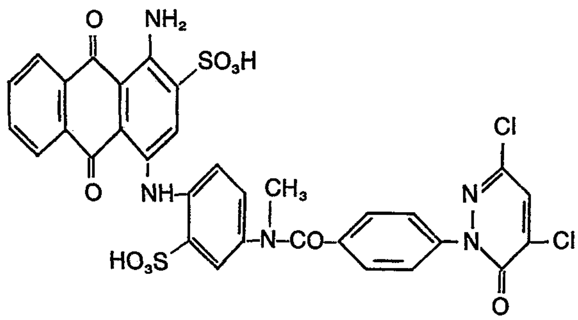

Dieser Farbstoff gibt auf natürlichen und synthetischen Polyamidfasern, insbesondere auf Cellulosefasermaterialien nach den für faserreaktive Farbstoffe üblichen Applikations- und Fixierverfahren farbstarke Färbungen und Drucke von brillanter blauer Nuance. Der Farbstoff zeichnet sich besonders durch sein gutes Ziehverhalten, seine hohe Fixiergeschwindigkeit und die damit erreichte Farbausbeute aus. Die mit ihm erhältlichen Färbungen und Drucke zeigen sehr gute Fabrikations- und Gebrauchsechtheiten, wie beispielsweise die in Beispiel 6 beschriebenen. Die mit ihm erhaltenen Drucke zeigen eine hohe Konturenschärfe und Reinheit des Weissfonds. Nuance und Farbstärke von Drucken und Färbungen sind auf Mischgeweben von Baumwolle und Zellwolle bei

den einzelnen Faserarten gleich. Nicht fixierte Anteile des Farbstoffes lassen sich leicht auswaschen.

Beispiele 195 bis 203

Verfährt man in analoger Weise wie im Beispiel 194 beschrieben, um konstitutionell ähnliche erfindungsgemässe Anthrachinonfarbstoffe mit der erfindungsgemässen faserreaktiven Gruppe herzustellen, und geht hierbei ebenso von der 1-Amino-4-brom-anthrachinon-2-sulfonsäure aus und setzt diese mit einer entsprechenden Diaminoverbindung um, wobei die zweite Aminogruppe durch eine Schutzgruppe geschützt ist, die anschliessend wieder abgespalten werden kann und versetzt diese Anthrachinonverbindung nach Abspaltung der Schutzgruppe mit einem erfindungsgemässen Säurechlorid, so erhält man beispielsweise mit den entsprechenden Diaminen der nachfolgenden Beispiele die der nachstehenden allgemeinen Anthrachinonverbindung entsprechenden erfindungsgemässen faserreaktiven Farbstoffe, worin der faserreaktive Anker $R^1$ und $R^3$ wie zu Anfang in der vorliegenden Beschreibung definiert ist. Diese erfindungsgemässen faserreaktiven Farbstoffe färben Fasermaterialien, insbesondere Cellulosefasermaterialien, in den angegebenen Farbtönen mit sehr guten Echtheitseigenschaften; sie sind im Farbstoff des Beispieles 194 vergleichbar.

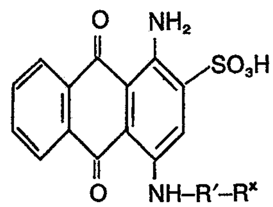

| Bsp. | R′ | $-R^x$ | Farbton der Färbung |
|---|---|---|---|
| 195 | 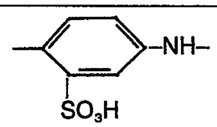 | $-R^1$ | blau |
| 196 | 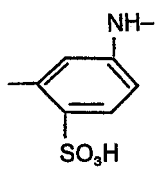 | $-R^1$ | blau |
| 197 | 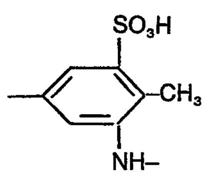 | $-R^1$ | blau |
| 198 | 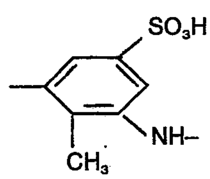 | $-R^1$ | blau |
| 199 | 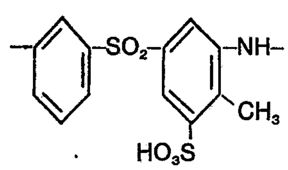 | $-R^1$ | blau |
| 200 | 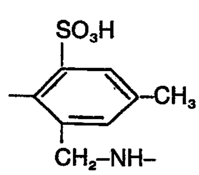 | $-R^1$ | blau |

(Fortsetzung)

| Bsp. | R′ | $-R^x$ | Farbton der Färbung |
|---|---|---|---|
| 201 | 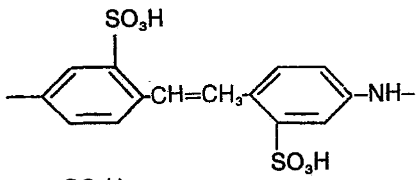 | $-R^1$ | grün |
| 202 | 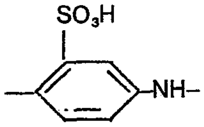 | $-R^3$ | blau |
| 203 | 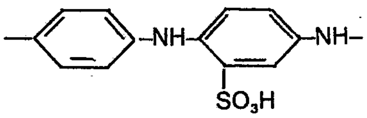 | $-R^3$ | oliv |

Beispiel 204

63,7 Teile der Formazan-Kupferkomplex-Verbindung der Formel

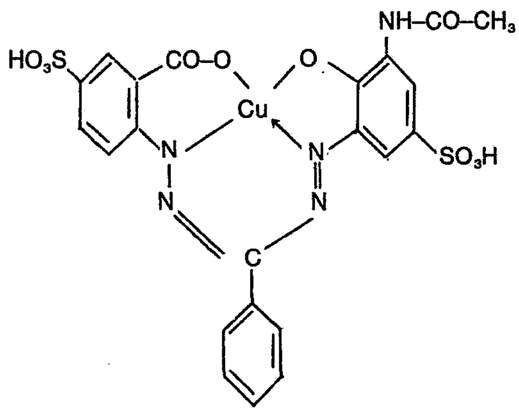

werden 2 Stunden lang mit 1n-Natronlauge zur Verseifung der Acetylaminogruppe gekocht. Anschliessend wird mit Eisessig ein pH-Wert von 6 eingestellt und die Aminogruppe bei einer Temperatur von 0 bis 5 °C mit der erfindungsgemässen faserreaktiven Verbindung 3,5-Dichlor-6-pyridazon-1-yl-benzol-4′-carbonsäurechlorid acyliert, indem 33 Teile dieser Verbindung der aminogruppenhaltigen Formazan-Verbindung innerhalb von einer Stunde zugegeben werden. Durch Zusatz von kristallisiertem Natriumacetat hält man während dieser Reaktion den pH-Wert zwischen 4 und 6. Man rührt bei einer Temperatur unterhalb von 10 °C 3 Stunden lang nach, klärt das Reaktionsgemisch, fällt aus dem Filtrat den so hergestellten faserreaktiven Farbstoff mit Natriumchlorid aus, wäscht ihn mit natriumchloridhaltiger wässriger Lösung und trocknet ihn. Man erhält 131 Teile eines blauen Farbstoffes der Konstitution

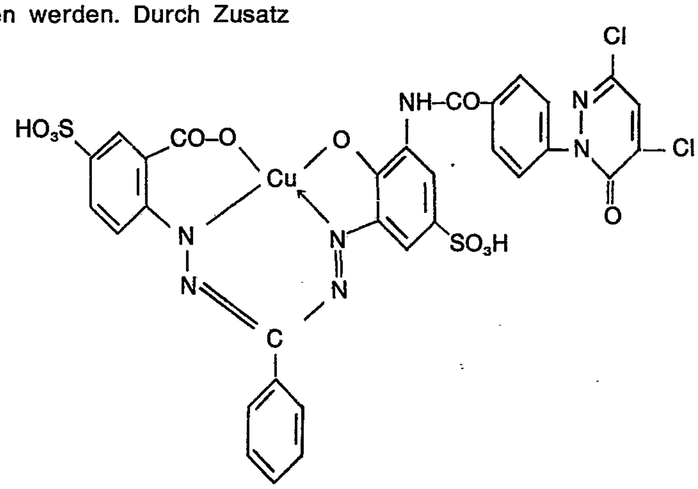

in Form seines Natriumsalzes. Mit ihm lassen sich in üblicher Weise nach Applikations- und Fixiermethoden für faserreaktive Farbstoffe Polyamidfasermaterialien, insbesondere aber Cellulosefasermaterialien mit intensiv blauen Farbtönen in guten Nass- und Lichtechtheiten färben.

Beispiele 205–207

In ähnlicher Weise, wie im Beispiel 204 beschrieben, lassen sich von entsprechenden Ausgangsverbindungen des Formazans, die eine primäre oder sekundäre Aminogruppe tragen, erfindungsgemässe faserreaktive Formazan-Kupferkomplex-Farbstoffe herstellen, in welchen die Aminogruppe mit dem faserreaktiven Säurechlorid der allgemeinen Formel (3), beispielsweise des Säurechlorids des Beispieles 1, dessen Acylrest hier mit $R^1$ bezeichnet ist, acyliert wurde. Diese Farbstoffe liefern ebenfalls auf Fasermaterialien, insbesondere Cellulosefasermaterialien, farbstarke Färbungen und Drucke mit guten Nass- und Lichtechtheiten in den nachfolgend angegebenen Farbtönen:

| Bsp. | Farbstoff der Formel (1) | Farbton |
|---|---|---|
| 205 | 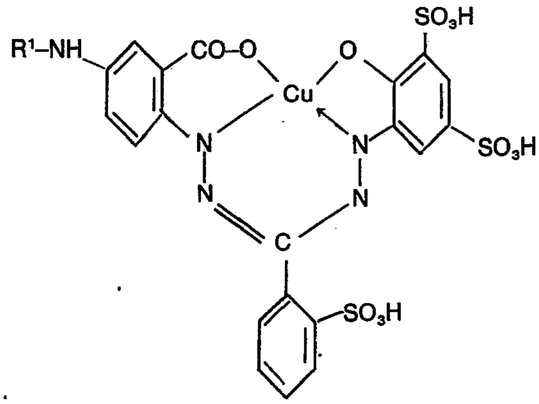 | blau |
| 206 | 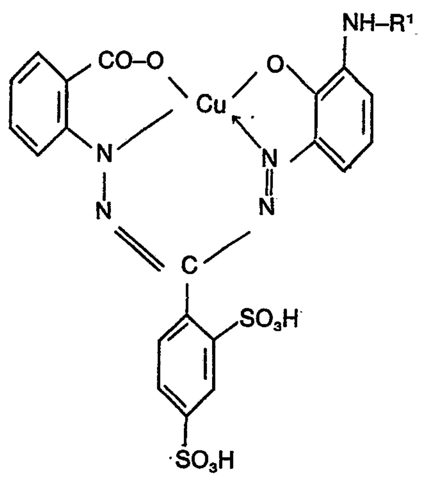 | blau |
| 207 | 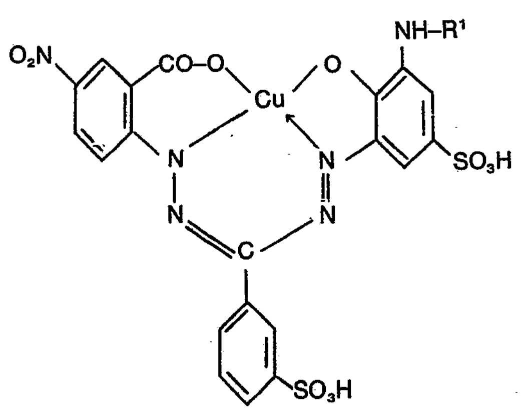 | grünstichig-blau |

Beispiel 208

96 Teile Kupferphthalocyanin-tetrasulfochlorid werden in 1200 Teilen eines Gemisches aus Eis und Wasser suspendiert und mit 44 Teilen 1,3-Diaminobenzol in 500 Teilen Wasser versetzt. Man rührt das Reaktionsgemisch 24 Stunden lang bei 20 °C, nachdem man einen pH-Wert von 8–9 eingestellt hat; diesen pH hält man mit Natriumcarbonat während der Reaktionszeit. Anschliessend wird das Reaktionsgemisch mit Salzsäure auf einen pH von 5 gestellt und mit Natriumchlorid ausgefällt und abgesaugt. Der feuchte Nutschkuchen wird in 1200 Teile eines Gemisches aus Wasser und Eis gelöst und auf einen pH von 6,5–7 gebracht; 40 Teile kristallisiertes Natriumacetat werden hinzugegeben, und im Verlaufe einer Stunde werden sodann bei einer Temperatur von 0–10 °C und einem pH-Wert von 4–6, der mit Natriumacetat eingestellt wird, 122 Teile 3,5-Dichlor-6-pyridazonyl(1)-benzol-4′-carbonsäurechlorid zugesetzt. Anschliessend wird noch 3–4 Stunden gerührt, das Reaktionsgemisch geklärt, das Filtrat mit Natriumchlorid versetzt und der abgeschiedene Farbstoff abgesaugt und bei 50–60 °C bei reduziertem Druck getrocknet. Man erhält ein elektrolythaltiges Pulver mit dem Alkalimetallsalz eines Farbstoffes, der im Mittel 2,5 Sulfogruppen und im Mittel 1,5 Sulfanilidgruppen mit der faserreaktiven Gruppe enthält. Er entspricht, in Form der freien Säure geschrieben, der nachstehenden Formel

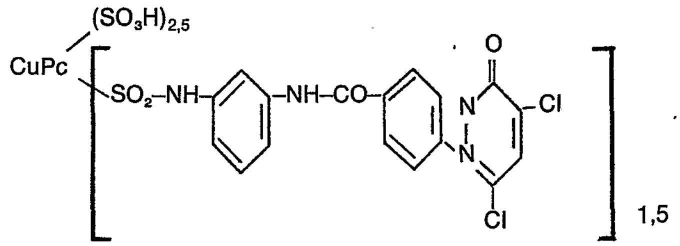

Mit diesem Farbstoff erhält man auf Polyamid- und Cellulosefasermaterialien nach den für Reaktivfarbstoffe üblichen Applikations- und Fixierverfahren klare blaue Drucke und Färbungen von guten Nass- und Lichtechtheiten sowie einer guten Reibechtheit.

Beispiele 209–211

Geht man in ähnlicher Weise, wie aus der Literatur bekannt oder wie in dem obigen Beispiel beschrieben, zur Synthese von Metallkomplex-Phthalocyanin-Farbstoffen von entsprechenden metallhaltigen, wie kupfer- kobalt- oder nickelhaltigen, Phthalocyaninsulfochloriden oder sulfogruppenhaltigen Phthalocyaninsulfochloriden aus und setzt diese mit einem aromatischen primären oder sekundären Diamin, gegebenenfalls auch zusätzlich mit einem aliphatischen primären oder sekundären Amin, um und lässt sodann auf die zweite Aminogruppe des aromatischen Amins ein erfindungsgemässes faserreaktives Säurechlorid der allgemeinen Formel (3) einwirken, so beispielsweise das Säurechlorid des Beispieles 1, so erhält man gleichfalls erfindungsgemässe faserreaktive Phthalocyaninfarbstoffe der allgemeinen Formel (1), so beispielsweise bei Verwendung von Phenylendiaminen oder deren Methyl, Methoxy und/oder Sulfo substituierten Derivate oder bei Verwendung von Diaminodiphenyl-Verbindungen, die durch Sulfo, Methoxy, Methyl oder Chlor substituiert sein können, beispielsweise die nachfolgend beschriebenen:

| Bsp. | erfindungsgemässer Farbstoff | Farbton der Färbung |
|---|---|---|
| 209 | 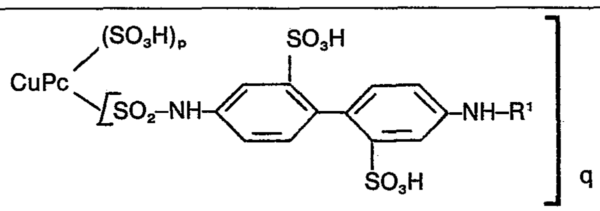 | türkisblau |
| 210 | 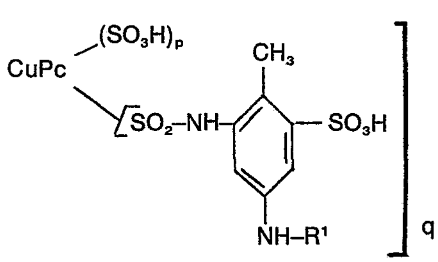 | türkisblau |

(Fortsetzung)

| Bsp. | erfindungsgemässer Farbstoff | Farbton der Färbung |
|---|---|---|
| 211 | 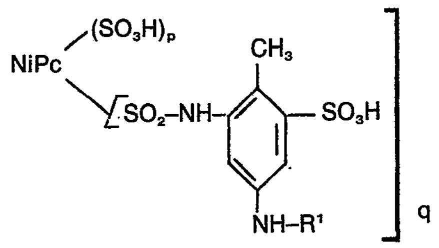 | blaust.-grün |

In den Formeln der Beispiele 209–211 bedeutet CuPc den Kupferphthalocyaninrest, NiPc den Nikkelphthalocyaninrest, p eine Zahl von 1–3 und q eine Zahl von 1–3, wobei p und q gleich oder verschieden voneinander sein können, jedoch die Summe von (p + q) maximal 4 ist.

Die Farbstoffe der Beispiele 209–211 ergeben bei Anwendung nach üblichen Applikations- und Fixierverfahren für faserreaktive Farbstoffe sehr gute nass- und lichtechte Färbungen und Drucke mit den angegebenen Farbtönen.

Beispiel 212

In ähnlicher Weise lassen sich auch Phthalocyaninfarbstoffe herstellen, wenn man als aromatisches Diamin beispielsweise die 1,6-Diamino-naphthalin-4-sulfonsäure, die 2,6-Diamino-naphthalin-4,8-disulfonsäure oder die symmetrischen Diaminodiphenylamin-, Di-(aminophenyl)-äthylen-, Di-(aminophenyl)-sulfon-, Di-(aminophenyl)-methan- oder -äthan- oder Di-amino-azobenzol-Verbindungen einsetzt, wobei die aromatischen Gruppen dieser genannten Verbindungen noch durch Sulfo, Methyl, Methoxy und/oder Chlor substituiert sein können.

Beispiel 213

72,5 Teile der Xanthenverbindung der Formel

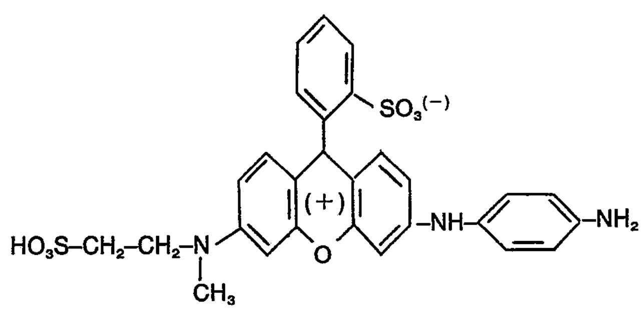

werden in 1000 Teilen Wasser bei 70 °C neutral gelöst. Die Lösung wird auf 0–10 °C abgekühlt, 30 Teile kristallines Natriumacetat werden zugesetzt, und anschliessend gibt man 33 Teile 3,5-Dichlor-6-pyridazonyl-1-benzol-4′-carbonsäurechlorid unter Rühren und Einhaltung eines pH-Wertes von 4–6 hinzu und rührt das Reaktionsgemisch noch mehrere Stunden bei diesem pH, bis kein diazotierbares Amin mehr nachweisbar ist. Man klärt das Reaktionsgemisch und fällt den gebildeten Farbstoff mit Natriumchlorid aus; er wird abgesaugt und bei 60–80 °C getrocknet. Man erhält ein elektrolythaltiges Pulver mit dem Farbstoff der Formel

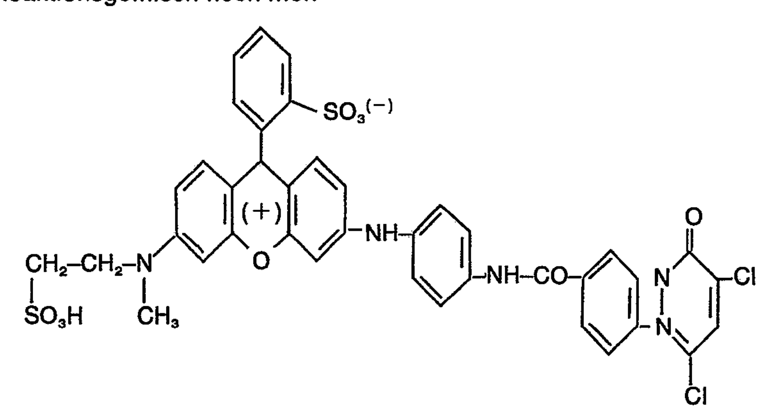

Dieser Farbstoff liefert auf Polyamidfasermaterialien nach den für faserreaktive Farbstoffe üblichen Anwendungsverfahren farbstarke Drucke und Färbungen von klarer violetter Nuance und guten Nassechtheiten.

Beispiel 214

Setzt man in ähnlicher Weise, wie im Beispiel 213 beschrieben, eine Xanthenverbindung ähnlicher Struktur, die eine primäre oder sekundäre Aminogruppe gebunden enthält, oder einen Triphenylmethanfarbstoff mit einer primären oder sekundären neuen Aminogruppe mit einem erfindungsgemässen Säurechlorid der allgemeinen Formel (3) um, so beispielsweise mit einem der Säurechloride der Beispiele 1–3, so erhält man ebenfalls erfindungsgemässe faserreaktive Farbstoffe, die insbesondere Cellulosefasermaterialien echt und farbstark in grünstichig-blauen bis rotstichig-blauen bis violetten Farbtönen färben.

**Patentansprüche**

1. Wasserlösliche Verbindungen der allgemeinen Formel (1)

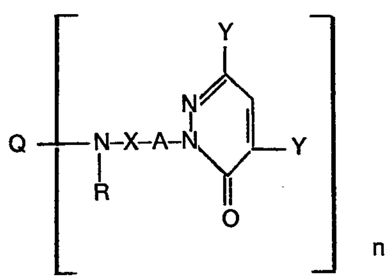

(1)

in welcher

Q der Rest einer organischen Verbindung ist, die faserveredelnde Eigenschaften besitzt, wobei Q eine oder mehrere wasserlöslichmachende Gruppen enthält,

A einen Alkylenrest von 1 bis 6 C-Atomen oder einen Alkylenrest von 2 bis 8 C-Atomen, der durch einen Phenylenrest unterbrochen ist, oder einen Phenylenrest oder einen Phenylen-alkylen-Rest mit 1 bis 4 C-Atomen im Alkylenrest oder einen Alkylen-phenylen-Rest mit 1 bis 4 C-Atomen im Alkylenrest bedeutet,

R für ein Wasserstoffatom oder eine Alkylgruppe mit 1 bis 4 C-Atomen, die durch einen Arylrest oder durch eine niedere Alkoxy-, eine Hydroxy-, Phenoxy- oder niedere Alkanoyloxy-Gruppe substituiert sein kann, oder für einen Cycloalkylrest oder für einen Arylrest steht,

X die Carbonyl- oder Sulfonylgruppe ist,

Y für ein Halogenatom steht und

n eine ganze Zahl von 1 bis 3 darstellt.

2. Verbindungen nach Anspruch 1, in welchen Q für den Rest eines wasserlöslichen organischen Farbstoffes steht.

3. Verfahren zur Herstellung der Verbindungen der allgemeinen Formel (1), dadurch gekennzeichnet, dass man eine organische Verbindung der allgemeinen Formel (2)

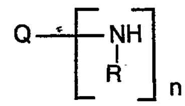

(2)

in welcher Q, R und n die in Anspruch 1 genannten Bedeutungen haben, mit einer Verbindung der allgemeinen Formel (3)

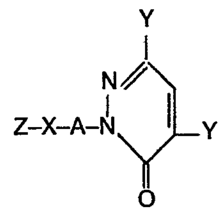

(3)

in welcher A, X und Y die obengenannten Bedeutungen haben und Z für ein Halogenatom steht, wobei Y und Z gleich oder verschieden voneinander sein können, umsetzt, oder indem man organische Vorprodukte für die Synthese des Molekülrestes der Formel Q in üblicher und hierfür geeigneter Weise miteinander umsetzt, wobei diese Vorprodukte so ausgewählt sind, dass mindestens eines davon eine oder mehrere wasserlöslich-machende Gruppen und mindestens eine Gruppe der Formel (4)

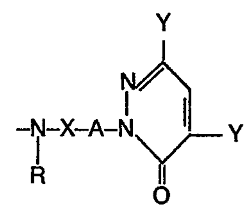

(4)

mit A, R, X und Y der in Anspruch 1 genannten Bedeutung enthält und dass man dabei wiederum diese Vorprodukte so auswählt, dass eine Verbindung der Formel (1) erhalten wird, in welcher n für eine Zahl von 1 bis 3 steht.

4. Verwendung der Verbindungen der allgemeinen Formel (1) gemäss Anspruch 1 zur Faserveredlung von Materialien aus natürlichen, regenerierten oder synthetischen stickstoffhaltigen Fasern oder aus natürlichen, regenerierten oder synthetischen hydroxygruppenhaltigen Fasern oder von Leder.

5. Verwendung der Verbindungen gemäss Anspruch 2 als Farbstoffe zum Färben und Bedrukken von Materialien aus natürlichen, regenerierten oder synthetischen stickstoffhaltigen Fasern oder aus natürlichen, regenerierten oder synthetischen hydroxygruppenhaltigen Fasern oder von Leder.

6. Verbindungen der allgemeinen Formel (3)

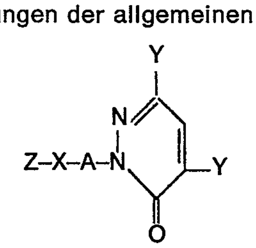

(3)

in welcher

A einen Alkylenrest von 1 bis 6 C-Atomen oder einen Alkylenrest von 2 bis 8 C-Atomen, der durch einen Phenylenrest unterbrochen ist, oder einen Phenylenrest oder einen Phenylen-alkylen-Rest mit 1 bis 4 C-Atomen im Alkylenrest oder einen Alkylen-phenylen-Rest mit 1 bis 4 C-Atomen im Alkylenrest bedeutet,

X die Carbonyl- oder Sulfonylgruppe ist, und

Y und Z gleich oder verschieden voneinander sind und jedes für ein Halogenatom steht.

7. Verfahren zur Herstellung der Verbindungen der allgemeinen Formel (3) gemäss Anspruch 6, dadurch gekennzeichnet, dass man eine Hydrazinverbindung der allgemeinen Formel (5)

$$HO{-}X{-}A{-}NH{-}NH_2 \qquad (5)$$

in welcher A und X die in Anspruch 6 genannten Bedeutungen haben oder die Gruppierung HO–X– für die Cyangruppe steht, mit Maleinsäureanhydrid zu einer Verbindung der Formel (6)

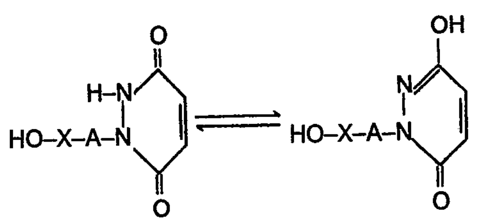

(6)

in welcher A und X die in Anspruch 6 genannten Bedeutungen haben oder die Gruppierung HO–X– für die Cyangruppe steht, umsetzt, die gegebenenfalls vorhandene Cyangruppe zur Carboxygruppe hydrolysiert und die erhaltene Verbindung der Formel (6) mit einem Halogenierungsmittel in die Verbindungen der Formel (3) überführt.

8. Verwendung der Verbindungen der allgemeinen Formel (3) gemäss Anspruch 6 als faserreaktive Anker in Verbindungen mit faserveredelnden Eigenschaften.

**Claims**

1. Water-soluble compounds of the general formula (1)

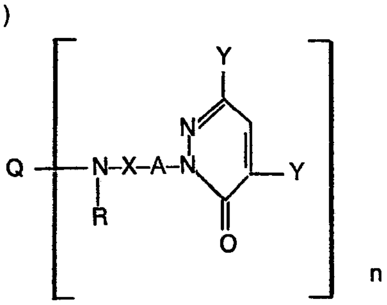

(1)

wherein

Q is the residue of an organic compound having fiberfinishing properties, Q containing one or several water-solubilizing groups,

A is an alkylene radical of 1 to 6 carbon atoms, or an alkylene radical of 2 to 8 carbon atoms which is interrupted by a phenylene radical, or A is a phenylene radical or a phenylene-alkylene radical having 1 to 4 carbon atoms in the alkylene moiety, or an alkylene-phenylene radical having 1 to 4 carbon atoms in the alkylene moiety,

R is a hydrogen atom or an alkyl group of 1 to 4 carbon atoms which may be substituted by an aryl radical or by a lower alkoxy, hydroxy, phenoxy or lower alkanoyloxy group, or R is a cycloalkyl radical or an aryl radical,

X is the carbonyl or sulfonyl group,

Y is a halogen atom and

n is an integer of from 1 to 3.

2. Compounds according to claim 1, wherein Q is the residue of a water-soluble organic dyestuff.

3. Process for the manufacture of the compounds of the general formula (1), characterized in that an organic compound of the general formula (2)

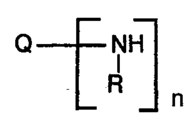

(2)

wherein

Q, R and n are as defined in claim 1, is reacted with a compound of the general formula (3)

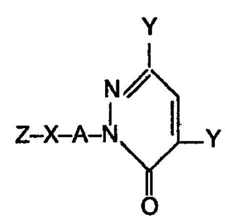

(3)

wherein A, X and Y are as defined above and Z is a halogen atom, Y and Z being identical to or different from one another, or in that organic primary products for the synthesis of the molecule residue of the formula Q are reacted with one another in usual manner appropriate for this purpose, these primary products being chosen such that at least one thereof contains one or several water-solubilizing groups and at least one group of the formula (4)

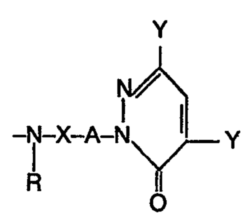

(4)

wherein

A, R, X and Y are as defined in claim 1, and that these primary products are chosen such that a compound of the formula (1) is obtained, wherein n is an integer of from 1 to 3.

4. Use of the compounds of the general formula (1) of claim 1 for fiber-finishing procedures of materials made from natural, regenerated or synthetic nitrogen-containing fibers or made from natural, regenerated or synthetic hydroxy groups-containing fibers or from leather.

5. Use of the compounds of claim 2 as dyestuffs for dyeing and printing materials made from natural, regenerated or synthetic nitrogen-containing fibers or from natural, regenerated or synthetic hydroxy groups-containing fibers or from leather.

6. Compounds of the general formula (3)

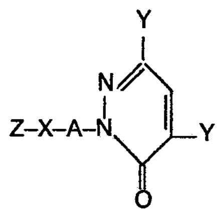 (3)

wherein

A is an alkylene radical of 1 to 6 carbon atoms, or an alkylene radical of 2 to 8 carbon atoms which is interrupted by a phenylene radical, or A is a phenylene radical or a phenylene-alkylene radical having 1 to 4 carbon atoms in the alkylene moiety, or an alkylene-phenylene radical having 1 to 4 carbon atoms in the alkylene moiety,

X is the carbonyl or sulfonyl group and

Y and Z are identical to or different from one another and each is a halogen atom.

7. Process for the manufacture of the compounds of the general formula (3) of claim 6, characterized in that a hydrazine compound of the general formula (5)

$$HO–X–A–NH–NH_2$$

wherein

A and X are defined as in claim 6, or the grouping HO–X– denotes the cyano group, is reacted with maleic anhydride to provide a compound of the formula (6)

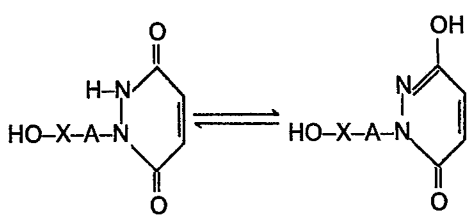 (6)

wherein

A and X are defined as in claim 6, or the grouping HO–X– denotes the cyano group, the optionally present cyano group is hydrolyzed to provide the carboxy group, and the compound of the formula (6) obtained is converted to the compounds of the formula (3) by means of a halogenation agent.

8. Use of the compounds of the general formula (3) of claim 6 as fiber-reactive anchor in compounds having fiber-finishing properties.

## Revendications

1. Composés hydrosolubles répondant à la formule générale (1):

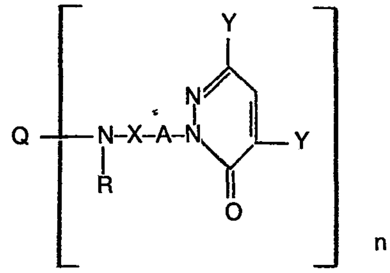 (1)

dans laquelle

Q représente le radical d'un composé organique qui a des propriétés ennoblissantes pour certaines matières fibreuses, ce radical Q contenant un ou plusieurs groupes hydrosolubilisants,

A représente un radical alkylène contenant de 1 à 6 atomes de carbone, un radical alkylène qui contient de 2 à 8 atomes de carbone et qui est interrompu par un radical phénylène, un radical phénylène, un radical phénylène-alkylène contenant de 1 à 4 atomes de carbone dans sa partie alkylène, ou un radical alkylène-phénylène contenant de 1 à 4 atomes de carbone dans sa partie alkylène,

R représente un atome d'hydrogène, un radical alkyle qui contient de 1 à 4 atomes de carbone et qui peut porter un radical aryle ou un radical alcoxy inférieur, hydroxy, phénoxy ou alcanoyloxy inférieur, ou représente un radical cycloalkyle ou un radical aryle,

X représente un radical carbonyle ou sulfonyle,

Y représente un atome d'halogène et

n représente un nombre entier de 1 à 3.

2. Composés selon la revendication 1, dans lesquels Q représente le radical d'un colorant organique soluble dans l'eau.

3. Procédé de préparation des composés de formule générale (1), procédé caractérisé en ce qu'on fait réagir un composé organique répondant à la formule générale (2):

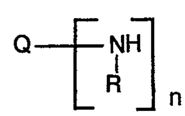 (2)

dans laquelle Q, R et n ont les significations données à la revendication 1, avec un composé répondant à la formule générale (3):

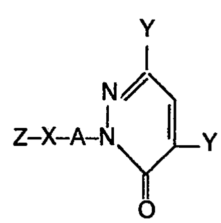 (3)

dans laquelle A, X et Y ont les significations indiquées ci-dessus et Z représente un atome d'halogène, Y et Z pouvant être identiques ou différents l'un de l'autre, ou on fait réagir entre eux des précurseurs organiques pour la synthèse du radical de formule Q, d'une manière usuelle et appropriée à cette fin, ces précurseurs étant choisis de

telle façon qu'au moins l'un d'entre eux contienne un ou plusieurs groupes hydrosolubilisants et au moins un radical répondant à la formule (4):

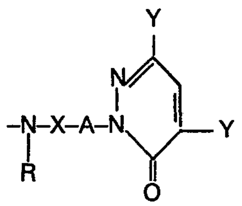

(4)

dans laquelle A, R, X et Y ont les significations données à la revendication 1, et ces précurseurs étant en outre choisis de telle façon qu'on obtienne un composé de formule (1) dans lequel n représente un nombre de 1 à 3.

4. Application des composés de formule générale (1) selon la revendication 1 pour l'ennoblissement de matières en fibres azotées naturelles, régénérées ou synthétiques, ou en fibres porteuses de groupes hydroxy naturelles, régénérées ou synthétiques, ou du cuir.

5. Application des composés selon la revendication 2, comme colorants pour la teinture et l'impression de matières en fibres azotées naturelles, régénérées ou synthétiques, ou en fibres porteuses de groupes hydroxy naturelles, régénérées ou synthétiques, ou du cuir.

6. Composés répondant à la formule générale (3):

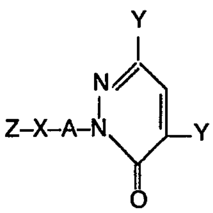

(3)

dans laquelle

A représente un radical alkylène contenant de 1 à 6 atomes de carbone, un radical alkylène qui contient de 2 à 8 atomes de carbone et qui est interrompu par un radical phénylène, un radical phénylène, un radical phénylène-alkylène dont la partie alkylène contient de 1 à 4 atomes de carbone, ou un radical alkylène-phénylène dont la partie alkylène contient de 1 à 4 atomes de carbone,

X représente un groupe carbonyle ou sulfonyle et

Y et Z, qui sont identiques ou différents, représentent chacun un atome d'halogène.

7. Procédé de préparation des composés de formule générale (3) selon la revendication 6, procédé caractérisé en ce qu'on fait réagir un composé hydrazinique répondant à la formule générale (5):

$$HO–X–A–NH–NH_2 \qquad (5)$$

dans laquelle A et X ont les significations données à la revendication 6 où le groupement HO–X– représente le radical cyano, avec l'anhydride maléique de manière à obtenir un composé répondant à la formule (6):

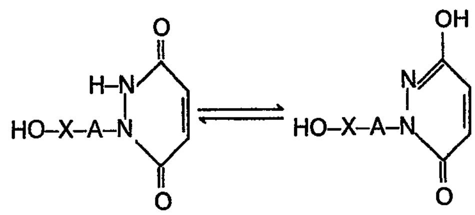

(6)

dans laquelle A et X ont les significations données à la revendication 6 où le groupement HO–X– représente le radical cyano, on hydrolyse l'éventuel radical cyano de manière à le convertir en un radical carboxy et on transforme le composé de formule (6) obtenu, au moyen d'un agent d'halogénation, en un composé de formule (3).

8. Application des composés de formule générale (3) selon la revendication 6 comme moyens d'ancrage, capables de réagir avec les fibres, dans des composés ayant des propriétés ennoblissantes pour des fibres.